# EUROPEAN PATENT APPLICATION

(11) **EP 3 822 288 A1**
(43) Date of publication of application: **19.05.2021**
(21) Application number: 19209795.4
(22) Date of filing: 18.11.2019
(51) Int. Cl.: C07K 16/28, A61P 35/00

(54) **ANTIBODIES TARGETING, AND OTHER MODULATORS OF, THE CD276 ANTIGEN, AND USES THEREOF**

(71) Applicant: Deutsches Krebsforschungszentrum, Stiftung des öffentlichen Rechts, 69120 Heidelberg (DE); Eberhard Karls Universität Tübingen, 72074 Tübingen (DE)
(72) Inventor: The designation of the inventor has not yet been filed
(74) Representative: Kuttenkeuler, David

(57) **Abstract**

The invention pertains to antibodies or other antigen binding proteins targeting the CD276 antigen, also known as B7-H3. The invention provides an improved set of antibodies which bind at new positions within the CD276 antigen and are of particular use as therapeutics in the treatment of CD276 positive cancer. Further the invention provides antibody conjugate and bispecific antibodies which were developed on basis of the novel anti-CD276 antibodies of the invention. Furthermore, the invention discloses the therapeutic use of the antibodies and other modulators in the treatment of CD276 positive cancer. Finally, the nucleic acid constructs encoding the molecules of the invention, recombinant cells expressing them, as well as particular uses and methods are provided.

## Description

### FIELD OF THE INVENTION

The invention pertains to antibodies or other antigen binding proteins targeting the CD276 antigen, also known as B7-H3. The invention provides an improved set of antibodies which bind at new positions within the CD276 antigen and are of particular use as therapeutics in the treatment of CD276 positive cancer. Further the invention provides antibody conjugate and bispecific antibodies which were developed on basis of the novel anti-CD276 antibodies of the invention. Furthermore, the invention discloses the therapeutic use of the antibodies and other modulators in the treatment of CD276 positive cancer. Finally, the nucleic acid constructs encoding the molecules of the invention, recombinant cells expressing them, as well as particular uses and methods are provided.

### DESCRIPTION

Humanized monoclonal antibodies (mAb) like Rituximab and Herceptin, which are employed for treatment of B cell-lymphoma and Her2/neu-positive breast cancer, respectively, have considerably improved therapeutic options for these disease entities. Central for the therapeutic activity of these mAbs is their ability to stimulate Fc-receptor (FcR) bearing effector cells, which results in lysis of target cells ("antibody dependent cellular cytotoxicity", ADCC). To increase efficacy, promising strategies comprise efforts to enforce the ability of a given antitumor mAb to stimulate FcR-mediated ADCC by modulating the amino acid sequence, e.g. using the SDIE modification1, or glycosylation pattern of the CH2 domain [1]. Another approach to improve the recruitment of NK- and possibly other effector cells- is the fusion of antibodies to cytokines such as IL2 or IL-15. Such immunocytokines have shown promising activity against neuroblastoma in children [2] and are currently evaluated in clinical trials with patients suffering from malignant melanoma. We have recently described a new class of immunocytokines containing a modified IL-15 moiety that confers target cell restricted activity (MIC proteins) [3].

The most promising approach for the optimization of antitumor antibodies appears to be the antibody-mediated stimulation of T cells with their - compared to NK cells - profoundly higher effector potential [4]. In fact, antibody based strategies that allow recruitment of T-cells against cancer cells have achieved remarkable successes: (i) monoclonal antibodies (mAbs) which block inhibitory "checkpoint" molecules on T-cells, such as CTLA4 or PD-1/PD-L1, induce long lasting remissions even in patients with high tumor burden. However, durable responses are achieved in a minor subset of patients only, and side effects caused by off-target activation of T-cells are considerable [5]. (ii) T-cells transfected with chimeric receptors (CAR T-cells) containing an antibody part directed to the B-cell associated CD19-molecule and CD3 associated signaling domains eradicate high numbers of malignant cells in patients suffering from B-cell derived malignancies [6,7] and (iii) Blinatumomab, a bispecific antibody (bsAb) with CD19xCD3-specificity in the so called bispecific T-cell engager (BiTE)-format, has received breakthrough designation in acute lymphatic leukaemia of B cell origin in 2014 [8-10].

However, in initial clinical studies the activity of CAR T cells as well as bispecific antibodies against solid tumors appears to be less remarkable [11-12], most likely because of a limited access of T cells to solid tumor sites. That sufficient influx of effector cells is a critical prerequisite for most immunotherapeutic strategies against solid tumors is suggested by reports demonstrating that even large numbers of tumor specific T-cells fail to exert sufficient antitumor activity unless of a proinflammatory environment has been generated at the tumor site [13,14]. Therefore, an optimal target antigen would be expressed not only on tumor cells but also on tumor vessels to allow for sufficient influx of immune cells across damaged vascular endothelium and subsequent tumor cell destruction.

CD276 belongs to the class of immunoglobulins. CD276, also called B7-H3 or B7RP-2, is a 77 kDa glycosylated class 1 transmembrane protein, involved in the immunological stimuli signal cascade. It is part of the family of B7 molecules and consists of an N-terminal signal peptide, an extracellular V- and C-lg-like domain, a transmembrane section and a 45-amino acid long C-terminus. The B7 family and their receptors, the CD28 family, are responsible for the binding of antigens to T-cells. This happens through positive co-stimulation and negative co-inhibition. B7-H3 was first described in 2001 by Chapoval, A.I., et al., (Nat Immunol, 2001. 2(3): p. 269-74) as molecule involved in the co-stimulation of the T-cell response and IFN-γ production. B7-H3 works in this context as inhibitor of T-cell stimulation, which at the same time stimulates the production of effector cytokines such as IL-4 and IFN-γ (Prasad, D.V., et al., J Immunol, 2004. 173(4): p. 2500-6). Additionally, it is involved in the stimulation of primary CD8 cytotoxic T-cells (CTLs), which are proven to reduce the tumor growth rate. Known are two isoforms: the human and the murine form, which differ in the number of V- and C-lg-like domains. The human protein (4 Ig B7H3) consists of 4 tandem Ig-like domains while the murine protein consists only of 2 domains (2 Ig B7H3). In contrast to other B7 family members, CD276 (B7-H3) RNA is present in almost all human tissues. However, the protein translation is tightly regulated via microRNAs, leading to an over-expression in many human cancers such as prostate cancer, non-small cell lung cancer, stomach cancer, neuroblastoma and ovarian cancer [25]. Studies in prostate cancer showed a correlation of CD276 expression with tumor dissemination, tumor relapse and overall survival. CD276 is one of the very few target antigens that exerts such dual expression on tumor and vascular cells and own immunohistochemical studies have confirmed that expression is rather specific for malignant as compared to normal tissue. In addition to its dual expression CD276 has been characterized as an immune checkpoint molecule with suppressive effects on T cell function. More information on CD276 can be derived from the UniProt database, version of November 2019 under accession number: Q5ZPR3. The amino acid sequences of the four human isoforms are herein provided as SEQ ID NO: 41- 44.

WO 2008/116219 discloses a monoclonal anti-CD276 antibody 8H9 which binds the antigen at the 4G domain and induces antibody dependent cell cytotoxicity (ADCC).

As summarized, there is the need for further therapeutic options which allow for an efficient targeting of tumor mass, which may be achieved by targeting such tumor associated antigens which are presented on the tumor vasculature. Hence, the present invention seeks to identify antibodies which can be used for the treatment of cancerous disorders.

### BRIEF DESCRIPTION OF THE INVENTION

Generally, and by way of brief description, the main aspects of the present invention can be described as follows:
In **a first aspect,** the invention pertains to an isolated antigen binding protein (ABP) which specifically binds to a B7-H3 protein, or a variant thereof, and wherein the isolated ABP is able to induce an antibody dependent cell-mediate cytotoxicity against a cell expressing the B7-H3.
In **a second aspect,** the invention pertains a bispecific antigen binding protein (ABP) which comprises a first antigen binding domain capable of binding to the B7-H3 antigen, or the variant thereof, and a second antigen binding domain capable of binding to the human cluster of differentiation 3 (CD3) antigen.
In **a third aspect,** the invention pertains to an isolated nucleic acid comprising a sequence encoding for an ABP, or for an antigen binding fragment or a monomer, such as a heavy or light chain, of an ABP, of any one of the first aspect, or encoding for a bispecific ABP according to the second aspect.
In **a fourth aspect,** the invention pertains to a nucleic acid construct (NAC) comprising a nucleic acid of the third aspect and one or more additional sequence features permitting the expression of the encoded ABP or bispecific ABP, or a component of said ABP or bispecific ABP (such as an antibody heavy chain or light chain) in a cell.
In **a fifth aspect,** the invention pertains to a recombinant host cell comprising a nucleic acid or a NAC according to the third or fourth aspect.
In **a sixth aspect,** the invention pertains to a pharmaceutical composition comprising: (i) an ABP or bispecific ABP of the first or second aspect, or (ii) a nucleic acid or NAC of the third or fourth aspect, or (iii) a recombinant host cell according to the fifth aspect, and a pharmaceutically acceptable carrier, stabiliser and/or excipient.
In **a seventh aspect,** the invention pertains to a component for use in medicine, wherein the component is selected from the list consisting of: (i) an ABP or bispecific ABP of the first or second aspect, or (ii) a nucleic acid or NAC of the third or fourth aspect, or (iii) a recombinant host cell according to the fifth aspect and (iv) a pharmaceutical composition according to the sixth aspect.
In **an eighth aspect,** the invention pertains to a method of enhancing a cell-mediated immune response to a human cell that expresses human B7-H3, comprising contacting said cell with (i) an ABP or bispecific ABP of the first or second aspect, or (ii) a nucleic acid or NAC of the third or fourth aspect, or (iii) a recombinant host cell according to the fifth aspect and (iv) a pharmaceutical composition according to the sixth aspect, in the presence of an immune cell, such as a T-cell or natural killer (NK) cell, thereby enhancing a cell-mediated immune response, preferably cytotoxicity, against said human cell.
In **a ninth aspect** the invention pertains to a method for the prevention and/or treatment of a proliferative disorder in a subject, comprising the administration of a therapeutically effective amount of a component recited in the seventh aspect to the subject; and wherein the proliferative disorder is characterized by an expression of B7-H3 in cells associated with the proliferative disorder.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The present invention in some aspects and embodiments provides polypeptides and proteins comprising an antigen binding domain of an anti-CD276 antibody as disclosed herein. The polypeptides and proteins advantageously specifically recognize and bind to CD276 (also known as B7-H3) with high affinity. The polypeptides and proteins advantageously specifically recognize and bind to soluble CD276 and also specifically recognize and bind to CD276 expressed on a cell surface. CD276 is expressed or overexpressed on a variety of human tumors, including pediatric solid tumors and adult carcinomas. Examples of cancers that express or overexpress CD276 include, but are not limited to, neuroblastoma, Ewing's sarcoma, rhabdomyosarcoma, and prostate, ovarian, colorectal, and lung cancers. CD276 is also expressed in tumor vasculature and is a tumor endothelial marker. Without being bound to a particular theory or mechanism, it is believed that by specifically recognizing and binding to CD276, the inventive polypeptides and proteins may, advantageously, target CD276-expressing cancer cells and/or tumor vasculature. In an embodiment of the invention, the inventive polypeptides and proteins may elicit an antigen-specific response against CD276. Accordingly, without being bound to a particular theory or mechanism, it is believed that by specifically recognizing and binding CD276, the inventive proteins and polypeptides may provide for one or more of the following: detecting CD276-expressing cancer cells and/or tumor vasculature, targeting and destroying CD276-expressing cancer cells and/or tumor vasculature, reducing or eliminating cancer cells and/or tumor vasculature, facilitating infiltration of immune cells and/or effector molecules to tumor site(s) and/or tumor vasculature, and enhancing/extending anti-cancer and/or anti-tumor vasculature responses.

In **a first aspect,** the invention pertains to an isolated antigen binding protein (ABP) which specifically binds to a B7-H3 protein, or a variant thereof, and wherein the isolated ABP is able to induce an antibody dependent cell-mediate cytotoxicity (ADCC) against a cell expressing the B7-H3.

### Antigen binding proteins targeting CD276

An "antigen binding protein" ("ABP") as used herein means a protein that specifically binds to a target antigen, such as to one or more epitope(s) displayed by or present on a target antigen. The antigen of the ABPs of the invention is CD276 or a orthologue (or paralogue) or other variant thereof; and the ABP can, optionally bind to one or more domains of said CD276 or variant (such as the epitope(s) can be displayed by or present on one or more extracellular domains of said CD276 or variant). Typically, an antigen binding protein is an antibody (or a fragment thereof), however other forms of antigen binding protein are also envisioned by the invention. For example, the ABP may be another (non-antibody) receptor protein derived from small and robust non-immunoglobulin "scaffolds", such as those equipped with binding functions for example by using methods of combinatorial protein design (Gebauer & Skerra, 2009; Curr Opin Chem Biol, 13:245). Particular examples of such non-antibody ABPs include: Affibody molecules based on the Z domain of Protein A (Nygren, 2008; FEBS J 275:2668); Affilins based on gamma-B crystalline and/or ubiquitin (Ebersbach et al, 2007; J Mo Biol, 372:172); Affimers based on cystatin (Johnson et al, 2012; Anal Chem 84:6553); Affitins based on Sac7d from Sulfolobus acidcaldarius (Krehenbrink et al, 2008; J Mol Biol 383:1058); Alphabodies based on a triple helix coiled coil (Desmet et al, 2014; Nature Comms 5:5237); Anticalins based on lipocalins (Skerra, 2008; FEBS J 275:2677); Avimers based on A domains of various membrane receptors (Silverman et al, 2005; Nat Biotechnol 23:1556); DARPins based on an ankyrin repeat motif (Strumpp et al, 2008; Drug Discov Today, 13:695); Fynomers based on an SH3 domain of Fyn (Grabulovski et al, 2007; J Biol Chem 282:3196); Kunitz domain peptides based on Kunitz domains of various protease inhibitors (Nixon et al, Curr opin Drug Discov Devel, 9:261) and Centyrins and Monobodies based on a 10th type III domain of fibronectin (Diem et al., 2014; Protein Eng Des Sel 27:419 doi: 10.1093/protein/gzu016; Koide & Koide, 2007; Methods Mol Biol 352:95). In the context of an ABP of the present invention that specifically binds human CD276, preferably isoform 1.

The term "epitope" includes any determinant capable of being bound by an antigen binding protein, such as an antibody. An epitope is a region of an antigen that is bound by an antigen binding protein that targets that antigen, and when the antigen is a protein, includes specific amino acids that bind the antigen binding protein (such as via an antigen binding domain of said protein). Epitope determinants can include chemically active surface groupings of molecules such as amino acids, sugar side chains, phosphoryl or sulfonyl groups, and can have specific three dimensional structural characteristics, and/or specific charge characteristics. Generally, antigen binding proteins specific for a particular target antigen will preferentially recognise an epitope on the target antigen in a complex mixture of proteins and/or macromolecules.

Preferred epitopes that may be targeted by the antibodies, or variants thereof, in accordance with the invention are those epitopes which comprise any one of the following amino acid positions in human CD276. The following positions are amino acids which are involved in the protein-protein interaction between the ABP of the invention and the human CD276 protein. The epitopes are the therefore regions having at least 8, and preferably not more than 20 consecutive amino acids of the human CD276 amino acid sequence provided herein, and comprise the following positions:

Epitopes in the IgC domain of human 276 in accordance with the invention are epitopes comprising Q179. Epitopes in the IgV domain of human CD276 any one, any two or all of: A115, G43 and/or F120. Further epitopes include R127 or G130.

An antigen binding protein is "specific" when it binds to one antigen (such as CD276; eg human CD276, orthologues and other variants thereof) more preferentially (eg, more strongly or more extensively) than it binds to a second antigen. The term "specifically binds" (or "binds specifically" and the like) used herein in the context of an ABP means that said ABP will preferentially bind to the desired antigen (eg CD276, in particular an IgC domain of CD276) than to bind to other proteins (or other molecules), such as preferentially binding to such CD276 compared to one or more of other Immunoglobulin (Ig) superfamily genes. Therefore, preferably, the binding affinity of the ABP to the one antigen (e.g. CD276) is at least 2-fold, 5-fold, at least 10-fold, at least 20-fold, at least 50-fold, at least 100-fold, at least 200-fold, at least 500-fold, at least 1000-fold, at least 2000-fold, at least 5000-fold, at least 10000-fold, at least 105-fold or even at least 106-fold, most preferably at least 2-fold, compared to its affinity to the other targets (e.g. unrelated proteins such as mouse or human Fc domain, or streptavidin).

IgV domain" (or "Ig-like V domain") and "IgC domain" (or "Ig-like C domain") as used herein, refer broadly to Ig superfamily member domains. These domains correspond to structural units that have distinct folding patterns called Ig-like folds. Ig-like folds are comprised of a sandwich of two sheets of antiparallel beta strands, with a conserved disulphide bond between the two sheets in most, but not all, domains. IgC domains of Ig, TCR, and MHC molecules share the same types of sequence patterns and are called the C1 set domains within the Ig superfamily. Other IgC domains fall within the IgC2 set domains. IgV domains also share sequence patterns and are called V set domains.

The term "orthologue" as used herein means a variant that descends from the same ancestral gene but which is present in another organism due to a speciation event. Orthologues of CD276 are typically expected to retain the same function as (or have a similar function to) human CD276.

The term "paralogue" as used herein means a variant in the same organism that descends from the same ancestral gene by a duplication event. A paralogue of CD276 is typically expected to be an immunoglobulin superfamily protein, in particular one having at least 70%, 80% 85% or 90% sequence identity to the amino acid sequence of the CD276 (if any such a paralogue exists in humans).

The term "variant" as used herein in the context of a protein means any natural or non-natural version of such protein which comprises one or more amino acid mutations compared to the reference protein, but which shares significant amino acid sequence identity with the reference protein, e.g. at least 70% or 75% amino acid sequence identity, preferably at least 80% amino acid sequence identity, more preferably at least 90% amino acid sequence identity and most preferably at least 95%, 96%, 97%, 98% or 99% amino acid sequence identity. Preferably, the variant of the protein possesses and/or maintains at least one function/activity that is the same, essentially the same or similar as the reference protein. Variants of CD276 may include orthologues to and natural variants of human CD276. Variants of CD276 may also correspond to human CD276 with one or more amino acid residues inserted into, or deleted from the amino acid sequence, such as those variants of CD276 naturally found within a population or those made by genetic manipulation, such as to specifically engineer amino acid changes into one or more domains (such as extracellular domains) of the variant. Variants of CD276 include fusion proteins of CD276 (for example, a human CD276 fused to a heterologous polypeptide chain, such as Fc immunoglobulin domains or tags), and/or CD276 conjugated to another chemical moiety such as an effector group or a labelling group. A variant of CD276 can, in certain embodiments, comprise a fragment of CD276, for example a polypeptide that consists of one or more IgC or IgV domains (or regions or (sub)domains thereof) of CD276 without one or other (or any other).

The term "identity" refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) are preferably addressed by a particular mathematical model or computer program (i.e., an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAM J. Applied Math. 48:1073.

In calculating percent identity, the sequences being compared are typically aligned in a way that gives the largest match between the sequences. One example of a computer program that can be used to determine percent identity is the GCG program package, which includes GAP (Devereux et al., 1984, Nucl. Acid Res. 12:387; Genetics Computer Group, University of Wisconsin, Madison, WI). The computer algorithm GAP is used to align the two polypeptides or polynucleotides for which the percent sequence identity is to be determined. The sequences are aligned for optimal matching of their respective amino acid or nucleotide (the "matched span", as determined by the algorithm). A gap opening penalty (which is calculated as 3x the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm.

A standard comparison matrix (see, Dayhoff et al., 1978, Atlas of Protein Sequence and Structure 5:345-352 for the PAM 250 comparison matrix; Henikoff et al., 1992, Proc. Natl. Acad. Sci. U.S.A. 89:10915-10919 for the BLOSUM 62 comparison matrix) may also be used by the algorithm.

Examples of parameters that can be employed in determining percent identity for polypeptides or nucleotide sequences using the GAP program are the following: (i) Algorithm: Needleman et al., 1970, J. Mol. Biol. 48:443-453; (ii) Comparison matrix: BLOSUM 62 from Henikoff et al., 1992, supra; (iii) Gap Penalty: 12 (but with no penalty for end gaps); (iv) Gap Length Penalty: 4; (v) Threshold of Similarity: 0.

A preferred method of determining similarity between a protein or nucleic acid and (or between) human CD276, a paralogue, orthologue or other variant thereof, is that provided by the Blast searches supported at Uniprot supra (e.g., http://www.uniprot.org/uniprot/ Q5ZPR3); in particular for amino acid identity, those using the following parameters: Program: blastp; Matrix: blosum62; Threshold: 10; Filtered: false; Gapped: true; Maximum number of hits reported: 250.

Certain alignment schemes for aligning two amino acid sequences may result in matching of only a short region of the two sequences, and this small aligned region may have very high sequence identity even though there is no significant relationship between the two full-length sequences. Accordingly, the selected alignment method (GAP program) can be adjusted if so desired to result in an alignment that spans at least about 10, 15, 20, 25, 30, 35, 40, 45, 50 or other number of contiguous amino acids of the target polypeptide or region thereof.

In particular embodiments of the invention, the CD276 is human CD276, preferably a protein comprising an amino acid sequence selected from the group consisting of: SEQ ID NO: 41, SEQ ID NO: 42, SEQ ID NO: 43 and SEQ ID NO: 44 (in particular, SEQ ID NO. 41), or a protein having no more than two, four, six, eight, or ten, for example no more than one, two or three, such as no more than one, amino acid substitutions, insertions or deletions compared to these sequences.

In the context of variants of CD276, the invention includes those embodiments where a variant of CD276 is a protein comprising an amino acid sequence having at least 80%, 85%, 90%, 92% 95% or 97% sequence identity (in particular, at least 92% or 95% sequence identity) to the sequence of SEQ ID NO: 41.

### ABPs of the invention comprising one or more complementarity determining regions

In particular embodiments, an ABP of the invention can preferentially comprise at least one complementarity determining region (CDR), such as one from an antibody (in particular from a human antibody), and in particular embodiments the ABP can comprise a CDR having an amino acid sequence with at least 80%, 85%, 90% or 95% sequence identity to (preferably, at least 90% sequence identity to), or having no more than three or two, preferably no more than one amino acid substitution(s), deletion(s) or insertion(s) compared to, a CDR sequence set forth in Table 1 herein.

The term "complementarity determining region" (or "CDR" or "hypervariable region"), as used herein, refers broadly to one or more of the hyper-variable or complementarily determining regions (CDRs) found in the variable regions of light or heavy chains of an antibody. See, for example: "IMGT", Lefranc et al, 20003, Dev Comp Immunol 27:55; Honegger & Plückthun, 2001, J Mol Biol 309:657, Abhinandan & Martin, 2008, Mol Immunol 45:3832, Kabat, et al. (1987): Sequences of Proteins of Immunological Interest National Institutes of Health, Bethesda, Md. These expressions include the hypervariable regions as defined by Kabat et al (1983) Sequences of Proteins of Immunological Interest, US Dept of Health and Human Services, or the hypervariable loops in 3-dimensional structures of antibodies (Chothia and Lesk, 1987; J Mol Biol 196:901). The CDRs in each chain are held in close proximity by framework regions and, with the CDRs from the other chain, contribute to the formation of the antigen-binding site. Within the CDRs there are select amino acids that have been described as the selectivity determining regions (SDRs) which represent the critical contact residues used by the CDR in the antibody-antigen interaction. (Kashmiri, 2005; Methods 36:25).

As described above, in particular embodiments of the invention, an ABP can comprise at least one complementarity determining region (CDR). In certain of such embodiments, an ABP of the invention comprises at least one complementarity determining region 3 (CDR3), such as one having an amino acid sequence with at least 80%, 85%, 90% or 95% (preferably at least 90%) sequence identity to, or having no more than three or two, preferably no more than one amino acid substitution(s), deletion(s) or insertion(s) compared to, a sequence selected from those heavy and light chain CDR3 sequences shown in Table 1 (eg, a sequence selected from the list consisting of SEQ ID Nos: 3, 7, 11, 15, 19, 23, 27, 31, 35, and 39.

An ABP of the invention may, alternatively or as well as a CDR3 sequence, comprise at least one CDR1, and/or at least one CDR2 (such as one from an antibody, in particular from a human antibody). Preferably, and ABP of the invention comprises at least one such CDR3, as well as at least one such CDR1 and at least one such CDR2, more preferably where each of such CDRs having an amino acid sequence with at least 80%, 85%, 90% or 95% (preferably at least 90%) sequence identity to, or having no more than three or two, preferably no more than one amino acid substitution(s), deletion(s) or insertion(s) compared to, a sequence selected from the corresponding (heavy and light chain) CDR1, CD2 and CD3 sequences shown in Table 1.

In particular embodiments, an ABP of the invention can be an antibody or an antigen binding fragment thereof.

As used herein, the term "antibody" may be understood in the broadest sense as any immunoglobulin (Ig) that enables binding to its epitope. An antibody as such is a species of an ABP. Full length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chain of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FRi, CDR1, FR2, CDR2, FR3, CDR3, FR4. The variable regions of the heavy and light chains contain a binding domain that interacts with an antigen. The constant regions of the antibodies may mediate the binding of the immunoglobulin to cells or factors, including various cells of the immune system (e.g., effector cells) and the first component (Ciq) of the classical complement system. Other forms of antibodies include heavy-chain antibodies, being those which consist only of two heavy chains and lack the two light chains usually found in antibodies. Heavy-chain antibodies include the hcIgG (IgG-like) antibodies of camelids such as dromedaries, camels, llamas and alpacas, and the IgNAR antibodies of cartilaginous fishes (for example sharks). And yet other forms of antibodies include single-domain antibodies (sdAb, called Nanobody by Ablynx, the developer) being an antibody fragment consisting of a single monomeric variable antibody domain. Single-domain antibodies are typically produced from heavy-chain antibodies, but may also be derived from conventional antibodies.

Antibodies (or those from which fragments thereof can be isolated) can include, for instance, chimeric, humanized, (fully) human, or hybrid antibodies with dual or multiple antigen or epitope specificities, antibody fragments and antibody sub-fragments, e.g., Fab, Fab' or F(ab')2 fragments, single chain antibodies (scFv) and the like (described below), including hybrid fragments of any immunoglobulin or any natural, synthetic or genetically engineered protein that acts like an antibody by binding to a specific antigen to form a complex.

Accordingly, in certain embodiments an ABP of the invention can comprise an antibody heavy chain, or an antigen binding fragment thereof, and/or an antibody light chain, or an antigen binding fragment thereof.

In further embodiments, an ABP of the invention can comprise an antibody heavy chain variable region, or an antigen binding fragment thereof, and/or an antibody light chain variable region, or an antigen binding fragment thereof, and in yet further embodiments, an ABP of the invention can comprise an antibody heavy chain variable region CDR1, CDR2, and CDR3, and/or an antibody light chain variable region CDR1, CDR2, and CDR3.

In particular embodiments of the invention, when the ABP comprises an antibody heavy chain sequence and/or an antibody light chain sequence, or an antigen binding fragment thereof; the antibody heavy chain sequence, or the fragment thereof, can comprise a CDR3 having at least 80%, 85%, 90%; or 95% (preferably at least 90%) sequence identity to, or having no more than three or two, preferably no more than one amino acid substitution(s), deletion(s) or insertion(s) compared to, a CDR3 sequence selected from those heavy chain CDR3 sequences shown in Table 1 (eg, a sequence selected from the list consisting of SEQ ID Nos: 3, 11, 19, 27, and 35), and/or wherein antibody light chain sequence, or the fragment thereof, can comprise a CDR3 having at least 80%, 85%, 90%; or 95% (preferably at least 90%) sequence identity to, or having no more than three or two, preferably no more than one amino acid substitution(s), deletion(s) or insertion(s) compared to, a CDR3 sequence selected from those light chain CDR3 sequences shown in Table 1 (eg, a sequence selected from the list consisting of SEQ ID Nos: 7, 15, 23, 31, and 39.

In further embodiments of the invention, when the ABP comprises an antibody heavy chain, or an antigen binding fragment thereof, the antibody heavy chain sequence, or the fragment thereof, can further comprise a CDR1 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 1, 9, 17, 25, and 33 (eg a heavy chain CDR1 sequence disclosed in Table 1); and/or a CDR2 having at 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 2, 10, 18, 26, and 34 (eg a CDR2 sequence disclosed in Table 1).

In yet further embodiments of the present invention, an ABP of the invention comprises an antibody light chain, or an antigen binding fragment thereof, wherein the antibody light chain sequence, or the fragment thereof, further comprises a CDR1 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 5, 13, 21, 29, and 37 (eg a light chain CDR1 sequence disclosed in Table 1); and/or a CDR2 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 6, 14, 22, 30, and 38 (eg a light chain CDR2 sequence disclosed in Table 1).

In other embodiments of the present invention, an ABP of the invention can comprise an antibody variable chain sequence having at least 80%, 85%, 90%; or 95% (preferably at least 90%) sequence identity to, or having no more than ten, nine, eight, seven, six, five, four, three, two or one, preferably no more than three, two or one amino acid substitution(s), deletion(s) or insertion(s) compared to, a sequence selected from SEQ ID NOs. 4, 8, 12, 16, 20, 24, 28, 32, 36, and 40 (eg, a VH or VL sequence disclosed in Table 1).

In particular embodiments of the invention, an ABP of the invention comprises an antigen binding fragment of an antibody, wherein the antigen binding fragment comprises CDR1, CDR2 and CDR3. In certain of such embodiments, the CDR1 is selected from those disclosed in Table 1, the CDR2 is selected from those disclosed in Table 1 and the CDR3 is selected from those disclosed in Table 1 (eg, the CDR1, CDR2 and CDR3 are selected from the CDR1, CDR2 and CDR3 sequences having the respective amino acid sequences of SEQ ID Nos. 1, 2, 3, or 5, 6, 7, or 9, 10, 11, or 13, 14, 15, or. 17, 18, 19, or 21, 22, 23, or 25, 26, 27, or. 29, 30, 31, or 33, 34, 35, or 37, 38, 39); in each case independently, optionally with no more than three or two, preferably no more than one, amino acid substitution(s), insertion(s) or deletion(s) compared to these sequences.

In further particular embodiments of the present invention, an ABP of the invention can comprise an antibody heavy chain variable region CDR1, CDR2, and CDR3, and/or an antibody light chain variable region CDR1, CDR2, and CDR3, wherein the CDR1 has an amino acid sequence of a heavy or light chain CDR1 shown in Table 1 (eg has an amino acid sequence selected from the list consisting of SEQ ID No 1, 5, 9, 13, 17, 21, 25, 29, 33, and 37), and wherein the CDR2 has an amino acid sequence of a heavy or light chain CDR2 shown in Table 1 (eg has an amino acid sequence selected from the list consisting of SEQ ID No 2, 6, 10, 14, 18, 22, 26, 30, 34, and 38), and wherein the CDR3 has an amino acid sequence of a heavy or light chain CDR3 shown in Table 1 (eg has an amino acid sequence selected from the list consisting of SEQ ID No 3, 7, 11, 15, 19, 23, 27, 31, 35, and 39); in each case independently, optionally with no more than three or two, preferably no more than one, amino acid substitution(s), insertion(s) or deletion(s) compared to these sequences.

In preferred of such embodiments, the ABP may be an antibody, or an antigen binding fragment thereof, composed of at least one, preferably two, antibody heavy chain sequences, and at least one, preferably two, antibody light chain sequences, wherein at least one, preferably both, of the antibody heavy chain sequences and at least one, preferably both, of the antibody light chain sequences comprise CDR1 to CDR3 sequences in a combination selected from any of the combinations of heavy chain CDRs shown in Table A and/or selected from any of the combinations of light chain CDRs shown in Table A; in each case independently, optionally with no more than three or two, preferably no more than one, amino acid substitution(s), insertion(s) or deletion(s) compared to these sequences. In particular preferred is such antibody shown in combination of the heavy and light chain sequences of the first row in table A below.

**Table A: preferred combinations of heavy chain CDRs and preferred combinations of light chain CDRs**

| **Heavy Chain CDR1 to CDR3 (SEQ ID NO)** | | | **Light Chain CDR1 to CDR3 (SEQ ID NO)** | | |
|---|---|---|---|---|---|
| 1 | 2 | 3 | 5 | 6 | 7 |
| 9 | 10 | 11 | 13 | 14 | 15 |
| 17 | 18 | 19 | 21 | 22 | 23 |
| 25 | 26 | 27 | 29 | 30 | 31 |
| 33 | 34 | 35 | 37 | 38 | 39 |

In a preferred embodiment, the invention pertains to an isolated antigen binding protein (ABP) which specifically binds to a CD276 protein, or a variant thereof, and wherein the isolated ABP is able to induce an antibody dependent cell-mediated cytotoxicity (ADCC) in a cell expressing the CD276 protein; and the ABP has at least one antigen binding domain which:
**(A)** comprises an antibody heavy chain CDR1 sequence shown in SEQ ID NO: 1, an antibody heavy chain CDR2 sequence shown in SEQ ID NO: 2, and an antibody heavy chain CDR3 sequence shown in SEQ ID NO: 3; and an antibody light chain CDR1 sequence shown in SEQ ID NO: 5, an antibody light chain CDR2 sequence shown in SEQ ID NO: 6, and an antibody light chain CDR3 sequence shown in SEQ ID NO: 7; in each case independently, optionally with no more than three or two, preferably no more than one, amino acid substitution(s), insertion(s) or deletion(s) compared to these sequences [such ABP is or is derived from the herein disclosed antibody with the internal designation 7C4]; or
**(B)** comprises an antibody heavy chain CDR1 sequence shown in SEQ ID NO: 9, an antibody heavy chain CDR2 sequence shown in SEQ ID NO: 10, and an antibody heavy chain CDR3 sequence shown in SEQ ID NO: 11; and an antibody light chain CDR1 sequence shown in SEQ ID NO: 13, an antibody light chain CDR2 sequence shown in SEQ ID NO: 14, and an antibody light chain CDR3 sequence shown in SEQ ID NO: 15; in each case independently, optionally with no more than three or two, preferably no more than one, amino acid substitution(s), insertion(s) or deletion(s) compared to these sequences[such ABP is or is derived from the herein disclosed antibody with the internal designation 11A7]; or
**(C)** comprises an antibody heavy chain CDR1 sequence shown in SEQ ID NO: 17, an antibody heavy chain CDR2 sequence shown in SEQ ID NO: 18, and an antibody heavy chain CDR3 sequence shown in SEQ ID NO: 19; and an antibody light chain CDR1 sequence shown in SEQ ID NO: 21, an antibody light chain CDR2 sequence shown in SEQ ID NO: 22, and an antibody light chain CDR3 sequence shown in SEQ ID NO: 23; in each case independently, optionally with no more than three or two, preferably no more than one, amino acid substitution(s), insertion(s) or deletion(s) compared to these sequences[such ABP is or is derived from the herein disclosed antibody with the internal designation 8D9]; or
**(D)** comprises an antibody heavy chain CDR1 sequence shown in SEQ ID NO: 25, an antibody heavy chain CDR2 sequence shown in SEQ ID NO: 26, and an antibody heavy chain CDR3 sequence shown in SEQ ID NO: 27; and an antibody light chain CDR1 sequence shown in SEQ ID NO: 29, an antibody light chain CDR2 sequence shown in SEQ ID NO: 30, and an antibody light chain CDR3 sequence shown in SEQ ID NO: 31; in each case independently, optionally with no more than three or two, preferably no more than one, amino acid substitution(s), insertion(s) or deletion(s) compared to these sequences[such ABP is or is derived from the herein disclosed antibody with the internal designation 10A7]; or
**(E)** comprises an antibody heavy chain CDR1 sequence shown in SEQ ID NO: 33, an antibody heavy chain CDR2 sequence shown in SEQ ID NO: 34, and an antibody heavy chain CDR3 sequence shown in SEQ ID NO: 35; and an antibody light chain CDR1 sequence shown in SEQ ID NO: 36, an antibody light chain CDR2 sequence shown in SEQ ID NO: 37, and an antibody light chain CDR3 sequence shown in SEQ ID NO: 38; in each case independently, optionally with no more than three or two, preferably no more than one, amino acid substitution(s), insertion(s) or deletion(s) compared to these sequences[such ABP is or is derived from the herein disclosed antibody with the internal designation 8H8].

In other preferred embodiments of the invention, the ABP may be an antibody, or an antigen binding fragment thereof, composed of at least one, preferably two, antibody heavy chain sequence, and at least one, preferably two, antibody light chain sequence, wherein the antibody heavy chain sequence and the antibody light chain sequence each comprises a variable region sequence in a combination of heavy and light chain variable domain shown in Table B in each case independently, optionally with no more than ten, nine, eight, seven, six, five, four, preferably no more than three, two or one, amino acid substitution(s), insertion(s) or deletion(s) compared to these sequences. In particular preferred is such antibody shown in combination of the heavy and light chain sequences of the first row in table B below.

**Table B: preferred combinations of heavy and light chain variably domains**

| **Heavy Chain Variable Domain (SEQ ID NO)** | **Light Chain Variable Domain (SEQ ID NO)** |
|---|---|
| 4 | 8 |
| 12 | 16 |
| 20 | 24 |
| 28 | 32 |
| 36 | 40 |

In preferred embodiments of all ABPs of the invention, the ABP is isolated and/or substantially pure.

The term "isolated" as used herein in the context of a protein, such as an ABP (an example of which could be an antibody), refers to a protein that is purified from proteins or polypeptides or other contaminants that would interfere with its therapeutic, diagnostic, prophylactic, research or other use. An isolated ABP according to the invention may be a recombinant, synthetic or modified (non-natural) ABP. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. Preferably an isolated ABP or nucleic acid or cells is/are substantially pure. In this context, a "recombinant" protein or nucleic acid is one made using recombinant techniques. Methods and techniques for the production of recombinant nucleic acids and proteins are well known in the art.

The term "isolated" as used herein in the context of a protein, such as an ABP (an example of which could be an antibody), refers to a protein that is purified from proteins or polypeptides or other contaminants that would interfere with its therapeutic, diagnostic, prophylactic, research or other use. An isolated ABP according to the invention may be a recombinant, synthetic or modified (non-natural) ABP. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. Preferably an isolated ABP or nucleic acid or cells is/are substantially pure. In this context, a "recombinant" protein or nucleic acid is one made using recombinant techniques. Methods and techniques for the production of recombinant nucleic acids and proteins are well known in the art.

In some embodiments, an ABP of the invention may bind to (e.g., via one or more epitope(s) displayed by one or more domain(s) of) CD276 or a paralogue, orthologue or other variant thereof (such as any CD276 or variant described herein) with a KD that is less than 20nM, such as less than about 10nM, 5nM or 2nM (in particular, less than about 1 nM). In a preferred embodiment, the ABP of the invention will bind (e.g. said epitope(s) of) said CD276 or variant with a KD that is less than 100 pM. In a more preferred embodiment, the ABP of the invention will bind said CD276 or variant with a KD that is about (+/-5) 10 pM. Binding of an ABP of the invention, such as an antibody of the invention, to a human cell line expressing said CD276 or variant may, in some embodiments, occur at an EC50 of less than about 10µg/mL, 5µg/mL, 2µg/mL, 1µg/mL, 0.5µg/mL or 0.2µg/mL, preferably with an EC50 of less than 2µg/mL. Binding of an ABP of the invention, such as an antibody of the invention, to a Cynomolgus cell line expressing an orthologue of said CD276 or variant may, in some embodiments, occur at an EC50 of less than about 10µg/mL, 5µg/mL, 2µg/mL, 1µg/mL, 0.5µg/mL, 0.2µg/mL, 0.1µg/ml, less than 50 ng/ml, or less than 25ng/ml, preferably with an EC50 of less than 10ng/mL, more preferably less than 5 ng/ml, most preferably of about 4 ng/ml.

In other embodiments, an ABP of the invention may: (i) bind to the CD276, or to the variant of CD276, with a KD that is less than 20nM, such as less than about 10nM, 5nM or 2nM (in particular, less than about 1 nM), is less than 100 pM, or is about 10 pM; and/or (ii) binds to a human cell line expressing the CD276 or the variant of CD276 with an EC50 of less than 10ng/mL.

The term "KD", as used herein, is intended to refer to the dissociation constant, which is obtained from the ratio of Kd to Ka (i. e., Kd/Ka) and is expressed as a molar concentration (M). KD values for antibodies can be determined using methods well established in the art such as plasmon resonance (BIAcore®), ELISA and KINEXA. A preferred method for determining the KD of an antibody is by using surface plasmon resonance, preferably using a biosensor system such as a BIAcore® system or by ELISA. "Ka" (or "K-assoc"), as used herein, refers broadly to the association rate of a particular antibody-antigen interaction, whereas the term "Kd" (or "K-diss"), as used herein, refers to the dissociation rate of a particular antibody-antigen interaction.

In one embodiment, an ABP of the invention is a polyclonal antibody (mixture), or the antigen binding fragment is a fragment of a polyclonal antibody (mixture).

In an alternative, and preferred, embodiment of all ABPs of the invention, the ABP is an antibody or an antigen binding fragment thereof, and the antibody is a monoclonal antibody, or wherein the antigen binding fragment is a fragment of a monoclonal antibody.

The term "monoclonal antibody" or "mAb" as used herein refers to an antibody obtained from a population of substantially identical antibodies based on their amino acid sequence. Monoclonal antibodies are typically highly specific. Furthermore, in contrast to conventional (polyclonal) antibody preparations which typically include different antibodies directed against different determinants (e.g. epitopes) of an antigen, each mAb is typically directed against a single determinant on the antigen. In addition to their specificity, mAbs are advantageous in that they can be synthesized by cell culture (hybridomas, recombinant cells or the like) uncontaminated by other immunoglobulins. The mAbs herein include for example chimeric, humanized or human antibodies or antibody fragments.

Monoclonal antibodies in accordance with the present invention may be prepared by methods well known to those skilled in the art. For example, mice, rats or rabbits may be immunized with an antigen of interest together with adjuvant. Splenocytes are harvested as a pool from the animals that are administered several immunisations at certain intervals with test bleeds performed to assess for serum antibody titers. Splenocytes are prepared that are either used immediately in fusion experiments or stored in liquid nitrogen for use in future fusions. Fusion experiments are then performed according to the procedure of Stewart & Fuller, J. Immunol. Methods 1989, 123:45-53. Supernatants from wells with growing hybrids are screened by eg enzyme-linked immunosorbent assay (ELISA) for mAb secretors. ELISA-positive cultures are cloned either by limiting dilutions or fluorescence-activated cell sorting, typically resulting in hybridomas established from single colonies. The ability of an antibody, including an antibody fragment or sub-fragment, to bind to a specific antigen can be determined by binding assays known in the art, for example, using the antigen of interest as the binding partner.

In a further preferred embodiment, an ABP of the invention is an antibody or an antigen binding fragment thereof, wherein the antibody is a human antibody a humanised antibody or a chimeric-human antibody, or wherein the antigen binding fragment is a fragment of a human antibody a humanised antibody or a chimeric-human antibody.

Human antibodies can also be derived by in vitro methods. Suitable examples include but are not limited to phage display (CAT, Morphosys, Dyax, Biosite/Medarex, Xoma, Yumab, Symphogen, Alexion, Affimed) and the like. In phage display, a polynucleotide encoding a single Fab or Fv antibody fragment is expressed on the surface of a phage particle (see e.g., Hoogenboom et al., J. Mol. Biol., 227: 381 (1991); Marks et al., J Mol Biol 222:581 (1991); U.S. Patent No. 5,885,793). Phage are "screened" to identify those antibody fragments having affinity for target. Thus, certain such processes mimic immune selection through the display of antibody fragment repertoires on the surface of filamentous bacteriophage, and subsequent selection of phage by their binding to target. In certain such procedures, high affinity functional neutralizing antibody fragments are isolated. A complete repertoire of human antibody genes may thus be created by cloning naturally rearranged human V genes from peripheral blood lymphocytes (see, e.g., Mullinax et al., Proc Natl Acad Sci (USA), 87: 8095-8099 (1990)) or by generating fully synthetic or semi-synthetic phage display libraries with human antibody sequences (see Knappik et al 2000; J Mol Biol 296:57; de Kruif et al, 1995; J Mol Biol 248):97).

The antibodies described herein may alternatively be prepared through the utilization of the XenoMouse® technology. Such mice are capable of producing human immunoglobulin molecules and antibodies and are deficient in the production of murine immunoglobulin molecules and antibodies. In particular, a preferred embodiment of transgenic production of mice and antibodies is disclosed in U.S. Patent Application Serial No. 08/759,620, filed December 3,1996 and International Patent Application Nos. WO 98/24893, published June 11, 1998 and WO 00/76310, published December 21, 2000. See also Mendez et al., Nature Genetics, 15:146-156 (1997). Through the use of such technology, fully human monoclonal antibodies to a variety of antigens have been produced. Essentially, XenoMouse® lines of mice are immunized with an antigen of interest. e.g. CD276, lymphatic cells (such as B-cells) are recovered from the hyper-immunized mice, and the recovered lymphocytes are fused with a myeloid-type cell line to prepare immortal hybridoma cell lines. These hybridoma cell lines are screened and selected to identify hybridoma cell lines that produce antibodies specific to the antigen of interest. Other "humanised" mice are also commercially available: eg, Medarex - HuMab mouse, Kymab - Kymouse, Regeneron - Velocimmune mouse, Kirin - TC mouse, Trianni - Trianni mouse, OmniAb - OmniMouse, Harbour Antibodies - H2L2 mouse, Merus - MeMo mouse. Also are available are "humanised" other species: rats: OmniAb - OmniRat, OMT - UniRat. Chicken: OmniAb - OmniChicken.

The term "humanised antibody" according to the present invention refers to immunoglobulin chains or fragments thereof (such as Fab, Fab', F(ab')2, Fv, or other antigen-binding sub-sequences of antibodies), which contain minimal sequence (but typically, still at least a portion) derived from non-human immunoglobulin. For the most part, humanised antibodies are human immunoglobulins (the recipient antibody) in which CDR residues of the recipient antibody are replaced by CDR residues from a non-human species immunoglobulin (the donor antibody) such as a mouse, rat or rabbit having the desired specificity, affinity and capacity. As such, at least a portion of the framework sequence of said antibody or fragment thereof may be a human consensus framework sequence. In some instances, Fv framework residues of the human immunoglobulin need to be replaced by the corresponding non-human residues to increase specificity or affinity. Furthermore, humanised antibodies can comprise residues which are found neither in the recipient antibody nor in the imported CDR or framework sequences. These modifications are made to further refine and maximise antibody performance. In general, the humanised antibody will comprise substantially all of at least one, and typically at least two, variable domains, in which all or substantially all of the CDR regions correspond to those of a non-human immunoglobulin and all or substantially all of the framework regions are those of a human immunoglobulin consensus sequence. The humanised antibody optimally also will comprise at least a portion of an immunoglobulin constant region, typically that of a human immunoglobulin, which (eg human) immunoglobulin constant region may be modified (eg by mutations or glycoengineering) to optimise one or more properties of such region and/or to improve the function of the (eg therapeutic) antibody, such as to increase or reduce Fc effector functions or to increase serum half-life. Exemplary such Fc modification (for example, Fc engineering or Fc enhancement) are described elsewhere herein.

The term "chimeric antibody" according to the present invention refers to an antibody whose light and/or heavy chain genes have been constructed, typically by genetic engineering, from immunoglobulin variable and constant regions which are identical to, or homologous to, corresponding sequences of different species, such as mouse and human. Alternatively, variable region genes derive from a particular antibody class or subclass while the remainder of the chain derives from another antibody class or subclass of the same or a different species. It covers also fragments of such antibodies. For example, a typical therapeutic chimeric antibody is a hybrid protein composed of the variable or antigen-binding domain from a mouse antibody and the constant or effector domain from a human antibody, although other mammalian species may be used.

In particular of such embodiments, an ABP of the invention comprises an antigen binding domain of an antibody wherein the antigen binding domain is of a human antibody. Preferably, ABP comprises an antigen binding domain of an antibody or an antigen binding fragment thereof, which is a human antigen binding domain; (ii) the antibody is a monoclonal antibody, or wherein the antigen binding fragment is a fragment of a monoclonal antibody; and (iii) the antibody is a human antibody or a humanised antibody, or wherein the antigen binding fragment is a fragment of a human antibody, a humanised antibody or a chimeric-human antibody.

Light chains of human antibodies generally are classified as kappa and lambda light chains, and each of these contains one variable region and one constant domain. Heavy chains are typically classified as mu, delta, gamma, alpha, or epsilon chains, and these define the antibody's isotype as IgM, IgD, IgG, IgA, and IgE, respectively. Human IgG has several subtypes, including, but not limited to, lgG1, lgG2, lgG3, and lgG4. Human IgM subtypes include IgM, and lgM2. Human IgA subtypes include lgA1 and lgA2. In humans, the IgA and IgD isotypes contain four heavy chains and four light chains; the IgG and IgE isotypes contain two heavy chains and two light chains; and the IgM isotype contains ten or twelve heavy chains and ten or twelve light chains. Antibodies according to the invention may be IgG, IgE, IgD, IgA, or IgM immunoglobulins.

In some embodiments, the ABP of the invention is an IgG antibody or fragment thereof. In some embodiments, the ABP of the invention is an IgE antibody or fragment thereof. In some embodiments, the ABP of the invention is an IgD antibody or fragment thereof. In some embodiments, the ABP of the invention is an IgA antibody or fragment thereof. In some embodiments, the ABP of the invention is an IgM antibody or fragment thereof. Preferably the ABP of the invention is, comprises or is derived from an IgG immunoglobulin or fragment thereof; such as a human, human-derived IgG immunoglobulin, or a rabbit- or rat-derived IgG, and/or an IgG2 immunoglobulin, or fragment thereof. When the ABP of the invention is, comprises or is derived from a rat-derived IgG, then preferably, the ABP is, comprises or is derived from, a rat IgG2a or IgG2b immunoglobulin. When the ABP of the invention is, comprises or is derived from a human-derived IgG, then more preferably, the ABP of the invention is, comprises or is derived from a human IgG1, IgG2 or IgG4, most preferably, the ABP of the invention is, comprises or is derived from a human IgG1 or IgG2

Accordingly, in particular embodiments of the invention, an ABP is an antibody wherein the antibody is an IgG, IgE, IgD, IgA, or IgM immunoglobulin; preferably an IgG immunoglobulin.

An ABP of the invention, where comprising at least a portion of an immunoglobulin constant region (typically that of a human immunoglobulin) may have such (eg human) immunoglobulin constant region modified - for example eg by glycoengineering or mutations - to optimise one or more properties of such region and/or to improve the function of the (eg therapeutic) antibody, such as to increase or reduce Fc effector functions or to increase serum half-life.

ABPs of the invention, in particular those useful in the present methods include antibodies that induce antibody-dependent cytotoxicity (ADCC) of CD276-expressing cells. The ADCC of an anti-CD276 antibody can be improved by using antibodies that have low levels of or lack fucose. Antibodies lacking fucose have been correlated with enhanced ADCC (antibody-dependent cellular cytotoxicity) activity, especially at low doses of antibody (Shields et ah, 2002, J. Biol. Chem. 277:26733-26740; Shinkawa et ah, 2003, J. Biol. Chem. 278:3466).

Methods of preparing fucose-less antibodies or antibodies with reduced fucose levels include growth in rat myeloma YB2/0 cells (ATCC CRL 1662). YB 2/0 cells express low levels of FUT8 mRNA, which encodes an enzyme (.alpha. 1,6- fucosyltransferase) necessary for fucosylation of polypeptides.

Alternatively, during the expression of such antibodies, an inhibitor against an enzyme relating to the modification of a sugar chain may be used, including: tunicamycin which selectively inhibits formation of GlcNAc-P-P-Dol which is the first step of the formation of a core oligosaccharide which is a precursor of an N- glycoside-linked sugar chain, castanospermin and W-methyl-1-deoxynojirimycin which are inhibitors of glycosidase I, kifunensine which is an inhibitor of mannosidase I, bromocondulitol which is an inhibitor of glycosidase II, 1 - deoxynojirimycin and 1,4-dioxy-1,4-imino-D-mannitol which are inhibitors of mannosidase I, swainsonine which is an inhibitor of mannosidase II, swainsonine which is an inhibitor of mannosidase II and the like. Examples of an inhibitor specific for a glycosyltransf erase include deoxy derivatives of substrates against N-acetylglucosamine transferase V (GnTV) and the like. Also it is known that 1-deoxynojirimycin inhibits synthesis of a complex type sugar chain and increases the ration of high mannose type and hybrid type sugar chains (Glycobiology series 2 - Destiny of Sugar Chain in Cell, edited by Katsutaka Nagai, Senichiro Hakomori and Akira Kobata, 1993).

Based on these data, several cell lines have been genetically engineered to produce antibodies containing no or low levels of fucose (Mori et al, 2004; Yamane-Ohnuki et al., 2004) to engineer the glycosylation patterns of IgG in order to select therapeutic monoclonal antibodies exhibiting particular profiles of Fc-gamma-R engagement that could be used in various pathologies.

Umana et al. and Davis et al. showed that an lgG1 antibody engineered to contain increasing amounts of bisected complex oligosaccharides (bisecting A/-acetylglucosamine, GlcNAC) allows triggering a strong ADCC as compared to its parental counterpart (Umana et al., 1999; Davies et al., 2001). Second, a lack of fucose on human lgG1 N-linked oligosaccharides has been shown to improve FCGRIII binding and ADCC.

GLYCART BIOTECHNOLOGY AG (Zurich, CH) has expressed N-acetyl-glucosaminyltransferase III (GnTIII) which catalyses the addition of the bisecting GlcNac residue to the N-linked oligosaccharide, in a Chinese hamster ovary (CHO) cell line, and showed a greater ADCC of lgG1 antibody produced (WO 99/54342; WO 03/01 1878; WO 2005/044859).

WO20070166306 is related to the modification of an antibody anti-CD19 containing 60% N-acetylglucosamine bisecting oligosaccharides and 10% non-fucosylated N-acetylglucosamine bisecting oligosaccharides produced in a mammalian human 293T embryonal kidney cells transfected with (i) the cDNA for the anti-CD19 antibody and (ii) the cDNA for the GnTIII enzyme.

Recombinant human lgG1 produced in YB2/0 cells (Shinkawa et al., 2003; Siberil et al., 2006) or in CHO-Lec13 (Shields et al., 2002) which exhibited a low-fucose content or were deficient in fucose as compared to the same lgG1 produced in wild-type CHO cells, showed an enhanced ability to trigger cellular cytotoxicity. By contrast, a correlation between galactose and ADCC was not observed and the content of bisecting GlcNAC only marginally affected ADCC (Shinkawa et al., 2003).

By removing or supplanting fucose from the Fc portion of the antibody, KYOWA HAKKO KOGYO (Tokyo, Japan) has enhanced Fc binding and improved ADCC, and thus the efficacy of the MAb (US 6,946,292). This improved Fc-gamma-RIIIA-dependent effector functions of low-fucosylated IgG has been shown to be independent from Fc-gamma-RI II allelic form (Niwa et al., 2005). Moreover, it has been recently shown that the antigenic density required to induce an efficient ADCC is lower when the IgG has a low content in fucose as compared to a highly fucosylated IgG (Niwa et al., 2005)

The Laboratoire Frangais du Fractionnement et des Biotechnologies (LFB) (France) showed that the ratio Fuc/Gal in MAb oligosaccharide should be equal or lower than 0.6 to get antibodies with a high ADCC (FR 2 861 080).

Cardarelli et al., 2019 produce an anti-CD19 antibody in Ms-704PF CHO cells deficient in the FUT8 gene which encodes alphal ,6-fucosyltransferase, Non-fucosylation of the antibody in this paper requires the engineering of an enzymes-deficient cell line. This paper does not consider amino acid mutations.

Herbst et al. generated a humanized lgG1 MAb MEDI-551 expressed in a fucosyltransferase-deficient producer CHO cell line This paper does not consider amino acid mutations (Herbst et al., 2010). [0049] S. Siberil et al used the rat myeloma YB2/0 cell line to produce a MAb anti RhD with a low fucose content. Whereas the MAb produced in a wild type CHO exhibited a high fucose content (81 %), the same MAb produced in YB2/0 cell exhibiited a lower fucose content (32%). This paper does consider amino acid mutations (Siberil et al., 2006).

Accordingly, An ABP of the invention may be prepared and/or may have one or more of the characteristics of such glycoengineering (eg afucosylated) approaches/antibodies described above.

Alternative methods for increasing ADDC activity for an ABP of the invention include mutations in an Fc portion of such ABP, particularly mutations which increase antibody affinity for an Fc-gamma-R receptor.

Accordingly, any of the ABPs of the invention described above can be produced with different isotypes or mutant isotypes to control the extent of binding to different Fc-gamma receptors. Antibodies lacking an Fc region (e.g., Fab fragments) lack binding to different Fc-gamma receptors. Selection of isotype also affects binding to different Fc-gamma receptors. The respective affinities of various human IgG isotypes for the three different Fc-gamma receptors, Fc-gamma-RI, Fc- gamma-RII, and Fc- gamma-RIII, have been determined. (See Ravetch & Kinet, Annu. Rev. Immunol. 9, 457 (1991)). Fc- gamma-RI is a high affinity receptor that binds to IgGs in monomeric form, and the latter two are low affinity receptors that bind IgGs only in multimeric form. In general, both IgGi and IgG3 have significant binding activity to all three receptors, IgG4 to Fc-gamma-RI, and IgG2 to only one type of Fc-gamma-RII called IIaLR (see Parren et al., J. Immunol. 148, 695 (1992). Therefore, human isotype IgG1 is usually selected for stronger binding to Fc-gamma receptors is desired, and IgG2 is usually selected for weaker binding.

A correlation between increased Fc-gamma-R binding with mutated Fc has been demonstrated using targeted cytoxicity cell- based assays (Shields et ah, 2001, J. Biol. Chem. 276:6591-6604; Presta et ah, 2002, Biochem Soc. Trans. 30:487-490). Methods for increasing ADCC activity through specific Fc region mutations include the Fc variants comprising at least one amino acid substitution at a position selected from the group consisting of: 234, 235, 239, 240, 241, 243, 244, 245, 247, 262, 263, 264, 265, 266, 267, 269, 296, 297, 298, 299, 313, 325, 327, 328, 329, 330 and 332, wherein the numbering of the residues in the Fc region is that of the EU index as in Kabat (Kabat et ah, Sequences of Proteins of Immunological Interest (National Institute of Health, Bethesda, Md. 1987).

In certain specific embodiments, said Fc variants comprise at least one substitution selected from the group consisting of L234D, L234E, L234N, L234Q, L234T, L234H, L234Y, L234I, L234V, L234F, L235D, L235S, L235N, L235Q, L235T, L235H, L235Y, L235I, L235V, L235F, S239D, S239E, S239N, S239Q, S239F, S239T, S239H, S239Y, V240I, V240A, V240T, V240M, F241W, F241L, F241Y, F241E, F241R, F243W, F243L, F243Y, F243R, F243Q, P244H, P245A, P247V, P247G, V262I, V262A, V262T, V262E, V263I, V263A, V263T, V263M, V264L, V264I, V264W, V264T, V264R, V264F, V264M, V264Y, V264E, D265G, D265N, D265Q, D265Y, D265F, D265V, D265I, D265L, D265H, D265T, V266I, V266A, V266T, V266M, S267Q, S267L, E269H, E269Y, E269F, E269R, Y296E, Y296Q, Y296D, Y296N, Y296S, Y296T, Y296L, Y296I, Y296H, N297S, N297D, N297E, A298H, T299I, T299L, T299A, T299S, T299V, T299H, T299F, T299E, W313F, N325Q, N325L, N325I, N325D, N325E, N325A, N325T, N325V, N325H, A327N, A327L, L328M, L328D, L328E, L328N, L328Q, L328F, L328I, L328V, L328T, L328H, L328A, P329F, A330L, A330Y, A330V, A330I, A330F, A330R, A330H, I332D, I332E, I332N, I332Q, I332T, I332H, I332Y and I332A, wherein the numbering of the residues in the Fc region is that of the EU index as in Kabat.

Fc variants can also be selected from the group consisting of V264L, V264I, F241W, F241L, F243W, F243L, F241L/F243L/V262I/V264I, F241W/F243W, F241W/F243W/V262A/V264A, F241L/V262I, F243L/V264I, F243L/V262I/V264W, F241Y/F243Y/V262T/V264T, F241E/F243R/V262E/V264R, F241E/F243Q/V262T/V264E, F241R/F243Q/V262T/V264R, F241E/F243Y/V262T/V264R, L328M, L328E, L328F, I332E, L3238M/I332E, P244H, P245A, P247V, W313F, P244H/P245A/P247V, P247G, V264I/I332E, F241E/F243R/V262E/V264R/I332E, F241E/F243Q/V262T/264E/I332E, F241R/F243Q/V262T/V264R/I332E, F241E/F243Y/V262T/V264R/I332E, S298A/I332E, S239E/I332E, S239Q/I332E, S239E, D265G, D265N, S239E/D265G, S239E/D265N, S239E/D265Q, Y296E, Y296Q, T299I, A327N, S267Q/A327S, S267L/A327S, A327L, P329F, A330L, A330Y, I332D, N297S, N297D, N297S/I332E, N297D/I332E, N297E/I332E, D265Y/N297D/I332E, D265Y/N297D/T299L/I332E, D265F/N297E/I332E, L328I/I332E, L328Q/I332E, I332N, I332Q, V264T, V264F, V240I, V263I, V266I, T299A, T299S, T299V, N325Q, N325L, N325I, S239D, S239N, S239F, S239D/I332D, S239D/I332E, S239D/I332N, S239D/I332Q, S239E/I332D, S239E/I332N, S239E/I332Q, S239N/I332D, S239N/I332E, S239N/I332N, S239N/I332Q, S239Q/I332D, S239Q/I332N, S239Q/I332Q, Y296D, Y296N, F241Y/F243Y/V262T/V264T/N297D/I332E, A330Y/I332E, V264I/A330Y/I332E, A330L/I332E, V264I/A330L/I332E, L234D, L234E, L234N, L234Q, L234T, L234H, L234Y, L234I, L234V, L234F, L235D, L235S, L235N, L235Q, L235T, L235H, L235Y, L235I, L235V, L235F, S239T, S239H, S239Y, V240A, V240T, V240M, V263A, V263T, V263M, V264M, V264Y, V266A, V266T, V266M, E269H, E269Y, E269F, E269R, Y296S, Y296T, Y296L, Y296I, A298H, T299H, A330V, A330I, A330F, A330R, A330H, N325D, N325E, N325A, N325T, N325V, N325H, L328D/I332E, L328E/I332E, L328N/I332E, L328Q/I332E, L328V/I332E, L328T/I332E, L328H/I332E, L328I/I332E, L328A, I332T, I332H, I332Y, I332A, S239E/V264I/I332E, S239Q/V264I/I332E, S239E/V264I/A330Y/I332E, S239E/V264I/S298A/A330Y/I332E, S239D/N297D/I332E, S239E/N297D/I332E, S239D/D265V/N297D/I332E, S239D/D265I/N297D/I332E, S239D/D265L/N297D/I332E, S239D/D265F/N297D/I332E, S239D/D265Y/N297D/I332E, S239D/D265H/N297D/I332E, S239D/D265T/N297D/I332E, V264E/N297D/I332E, Y296D/N297D/I332E, Y296E/N297D/I332E, Y296N/N297D/I332E, Y296Q/N297D/I332E, Y296H/N297D/I332E, Y296T/N297D/I332E, N297D/T299V/I332E, N297D/T299I/I332E, N297D/T299L/I332E, N297D/T299F/I332E, N297D/T299H/I332E, N297D/T299E/I332E, N297D/A330Y/I332E, N297D/S298A/A330Y/I332E, S239D/A330Y/I332E, S239N/A330Y/I332E, S239D/A330L/I332E, S239N/A330L/I332E, V264I/S298A/I332E, S239D/S298A/I332E, S239N/S298A/I332E, S239D/V264I/I332E, S239D/V264I/S298A/I332E, AND S239D/204I/A330L/I332E, wherein the numbering of the residues in the Fc region is that of the EU index as in Kabat. See also WO2004029207, incorporated by reference herein.

In particular embodiments, mutations on, adjacent, or close to sites in the hinge link region (e.g., replacing residues 234, 235, 236 and/or 237 with another residue) can be made, in all of the isotypes, to reduce affinity for Fc-gamma receptors, particularly Fc-gamma-RI receptor (see, eg US6624821). Optionally, positions 234, 236 and/or 237 are substituted with alanine and position 235 with glutamate. (See, eg US5624821.) Position 236 is missing in the human IgG2 isotype. Exemplary segments of amino acids for positions 234, 235 and 237 for human IgG2 are Ala Ala Gly, Val Ala Ala, Ala Ala Ala, Val Glu Ala, and Ala Glu Ala. A preferred combination of mutants is L234A, L235E and G237A, or is L234A, L235A, and G237A for human isotype IgGi. A particular preferred ABP of the invention is an antibody aving human isotype IgG and one of these three mutations of the Fc region. Other substitutions that decrease binding to Fc-gamma receptors are an E233P mutation (particularly in mouse IgGi) and D265A (particularly in mouse IgG2a). Other examples of mutations and combinations of mutations reducing Fc and/or C1q binding are E318A/K320A/R322A (particularly in mouse IgGi), L235A/E318A/K320A/K322A (particularly in mouse IgG2a). Similarly, residue 241 (Ser) in human IgG4 can be replaced, eg with proline to disrupt Fc binding.

Additional mutations can be made to a constant region to modulate effector activity. For example, mutations can be made to the IgG1 or IgG2a constant region at A330S, P331S, or both. For IgG4, mutations can be made at E233P, F234V and L235A, with G236 deleted, or any combination thereof. IgG4 can also have one or both of the following mutations S228P and L235E. The use of disrupted constant region sequences to modulate effector function is further described, eg in WO2006118,959 and WO2006036291.

Additional mutations can be made to the constant region of human IgG to modulate effector activity (see, e.g., WO200603291). These include the following substitutions: (i) A327G, A330S, P331S; (ii) E233P, L234V, L235A, G236 deleted; (iii) E233P, L234V, L235A; (iv) E233P, L234V, L235A, G236 deleted, A327G, A330S, P331S; and (v) E233P, L234V, L235A, A327G, A330S, P331S to human IgGi; or in particular, (vi) L234A, L235E, G237A, A330S and P331S (eg, to human IgGi), wherein the numbering of the residues in the Fc region is that of the EU index as in Kabat. See also WO2004029207, incorporated by reference herein.

The affinity of an antibody for the FcR can be altered by mutating certain residues of the heavy chain constant region. For example, disruption of the glycosylation site of human IgG1 can reduce FcR binding, and thus effector function, of the antibody (see, eg WO2006036291). The tripeptide sequences NXS and NXT, where X is any amino acid other than proline, are the enzymatic recognition sites for glycosylation of the N residue. Disruption of any of the tripeptide amino acids, particularly in the CH2 region of IgG, will prevent glycosylation at that site. For example, mutation of N297 of human IgG1 prevents glycosylation and reduces FcR binding to the antibody.

A preferred enhancement of Fc receptor binding can be achieved by introducing the Fc domain mutants of human IgG1 referred to herein generally as "SDIE", which denote the mutations S239D/I332E.

Although activation of ADCC and CDC is often desirable for therapeutic antibodies, there are circumstances in which an ABP of the invention unable to activate effector functions is preferential (eg, an ABP of the invention that is an agnostic modulator). For these purposes IgG4 has commonly been used but this has fallen out of favour in recent years due the unique ability of this sub-class to undergo Fab-arm exchange, where heavy chains can be swapped between IgG4 in vivo. Accordingly, Fc engineering approaches can also be used to determine the key interaction sites for the Fc domain with Fc-gamma receptors and C1q and then mutate these positions, such as in an Fc of an ABP of the invention, to reduce or abolish binding. Through alanine scanning Duncan and Winter (1998; Nature 332:738) first isolated the binding site of C1q to a region covering the hinge and upper CH2 of the Fc domain. Researchers at Genmab identified mutants K322A, L234A and L235A, which in combination are sufficient to almost completely abolish Fc-gamma-R and C1q binding (Hezareh et al, 2001; J Virol 75:12161). In a similar manner MedImmune later identified a set of three mutations, L234F/L235E/P331S (dubbed TM), which have a very similar effect (Oganesyan et al, 2008; Acta Crystallographica 64:700). An alternative approach is modification of the glycosylation on asparagine 297 of the Fc domain, which is known to be required for optimal FcR interaction. A loss of binding to FcRs has been observed in N297 point mutations (Tao et al, 1989; J Immunol 143:2595), enzymatically degylcosylated Fc domains (Mimura et al, 2001; J Biol Chem 276:45539), recombinantly expressed antibodies in the presence of a glycosylation inhibitor (Walker et al, 1989; Biochem J 259:347) and the expression of Fc domains in bacteria (Mazor et al 2007; Nat Biotechnol 25:563). Accordingly, the invention also includes embodiments of the ABPs in which such technologies or mutations have been used to reduce effector functions.

IgG naturally persists for a prolonged period in (eg human) serum due to FcRn-mediated recycling, giving it a typical half-life of approximately 21 days. Despite this there have been a number of efforts to engineer the pH dependant interaction of the Fc domain with FcRn to increase affinity at pH 6.0 while retaining minimal binding at pH 7.4. Researchers at PDL BioPharma identified the mutations T250Q/M428L, which resulted in an approximate 2-fold increase in IgG half-life in rhesus monkeys (Hinto et al, 2004; J Biol Chem 279:6213), and researchers at MedImmune have identified mutations M252Y/S254T/T256E (dubbed YTE), which resulted in an approximate 4-fold increase in IgG half-life in cynomolgus monkeys (Dall'Acqua, et al 2006; J Biol Chem 281:23514). ABPs of the invention may aslo be PEGylated. PEGylation, ie chemical coupling with the synthetic polymer poly-ethylene glycol (PEG), has emerged as an accepted technology for the development of biologics that exercise prolonged action, with around 10 clinically approved protein and peptide drugs to date (Jevsevar et al., 2010; Biotechnol J 5:113). ABPs of the invention may also be subjected to PASylation, a biological alternative to PEGylation for extending the plasma half-life of pharmaceutically active proteins (Schlapschy et al, 2013; Protein Eng Des Sel 26:489; XL-protein GmbH, Germany). Accordingly, the invention also includes embodiments of the ABPs in which such technologies or mutations have been used to prolong serum half-life, especially in human serum.

Antibody fragments include "Fab fragments", which are composed of one constant and one variable domain of each of the heavy and the light chains, held together by the adjacent constant region of the light chain and the first constant domain (CH1) of the heavy chain. These may be formed by protease digestion, e.g. with papain, from conventional antibodies, but similar Fab fragments may also be produced by genetic engineering. Fab fragments include Fab', Fab and "Fab-SH" (which are Fab fragments containing at least one free sulfhydryl group).

Fab' fragments differ from Fab fragments in that they contain additional residues at the carboxy terminus of the first constant domain of the heavy chain including one or more cysteines from the antibody hinge region. Fab' fragments include "Fab'-SH" (which are Fab' fragments containing at least one free sulfhydryl group).

Further, antibody fragments include F(ab')2 fragments, which contain two light chains and two heavy chains containing a portion of the constant region between the CH1 and CH2 domains ("hinge region"), such that an interchain disulphide bond is formed between the two heavy chains. A F(ab')2 fragment thus is composed of two Fab' fragments that are held together by a disulphide bond between the two heavy chains. F(ab')2 fragments may be prepared from conventional antibodies by proteolytic cleavage with an enzyme that cleaves below the hinge region, e.g. with pepsin, or by genetic engineering.

An "Fv region" comprises the variable regions from both the heavy and light chains, but lacks the constant regions. "Single-chain antibodies" or "scFv" are Fv molecules in which the heavy and light chain variable regions have been connected by a flexible linker to form a single polypeptide chain, which forms an antigen binding region.

An "Fc region" comprises two heavy chain fragments comprising the CH2 and CH3 domains of an antibody. The two heavy chain fragments are held together by two or more disulphide bonds and by hydrophobic interactions of the CH3 domains.

Accordingly, in some embodiments, the ABP of the invention is an antibody fragment selected from the list consisting of: Fab', Fab, Fab'-SH, Fab-SH, Fv, scFv and F(ab')2.

In those embodiments of ABPs that are fragments of immunoglobulins, such as an antibody fragment, preferred are those fragments capable of binding to (eg an epitope displayed by) the extracellular domain(s) of CD276, or a paralogue, orthologue or other variant thereof, such as any epitope or other binding characteristic as described herein: and more preferably said fragment is a modulator (such as an inhibitor or antagonist) of the expression, function, activity and/or stability of CD276 or a paralogue, orthologue or other variant of CD276.

In a preferred embodiment, an ABP of the invention is an antibody wherein at least a portion of the framework sequence of said antibody or fragment thereof is a human consensus framework sequence, for example, comprises a human germline-encoded framework sequence.

In some embodiments, an ABP of the invention is modified or engineered to increase antibody-dependent cellular cytotoxicity (ADCC). As will now be understood by the person of ordinary skill, such ABPs of the invention will have particular utility in the therapy of diseases or disorders associated with cellular resistance against immune cells like CTLs (such as an CD276-positive cancer); as the ADCC mechanism (a cell-mediated immune defence whereby an effector cell of the immune system actively lyses a target cell, whose membrane-surface antigens have been bound by specific antibodies) would be enhanced in respect of the cells having resistance against immune cells like CTLs, hence leading to an increase in attachment by and/or lysis of such cells by effector cells of the immune system.

As used herein, "therapy" is synonymous with treating a disease, disorder or condition, which includes reducing symptoms of the disease, disorder or condition, inhibiting progression of the disease, disorder or condition, causing regression of the disease, disorder or condition and/or curing the disease, disorder or condition.

Various techniques to modify or engineer an ABP of the invention to increase ADCC are known (Satoh et al, 2006; Expert Opin Biol Ther 6:1161; WO2009/135181), and hence such embodiments include those wherein an ABP of the invention may be afucosylated (GlycArt Biotechnology) e.g., in which antibodies are produced in CHO cells in which the endogenous FUT8 gene has been knocked out; or the ABP may be a "Sugar-Engineered Antibody" (Seattle Genetics), e.g. in which fucose analogues are added to antibody-expressing CHO cells, resulting in a significant reduction in fucosylation. Other afucosylation approaches that may be applied to an ABP of the invention are described elsewhere herein.

Other techniques to modify or engineer an ABP of the invention to increase ADCC include mutations in a Fc portion of the ABP, (such as described in more detail elsewhere herein), in particular where one or more of residues 234, 235, 236 and/or 237, and/or residues 330, 331 of human Fc are so mutated; wherein such numbering of the residues in the Fc region is that of the EU index as in Kabat (Kabat et ah, Sequences of Proteins of Immunological Interest (National Institute of Health, Bethesda, Md. 1987).

Accordingly, in certain embodiments, the ABP of the invention is modified or engineered to increase antibody-dependent cell-mediated cytotoxicity (ADCC), preferably wherein said ABP is afucosylated and/or an Fc of said ABP is mutated (eg where an Fc is mutated using one or more of the following residue changes: L234A, L235E, G237A, A330S and/or P331S). In alternative embodiments, the ABP of the invention is modified or engineered to reduce antibody-dependent cell-mediated cytotoxicity (ADCC).

In other certain embodiments, the ABP of the invention is modified to prolong serum half-life, especially in human serum. For example, an ABP of the invention may be PEGylated and/or PASylated, or has an Fc region with a T250Q/M428L or M252Y/S254T/T256E modification.

In certain embodiments, the ABP of the invention binds to (a) one or more epitopes displayed by an extracellular domain of CD276, or the variant of CD27611; or which binds to (b) two or more epitopes displayed by an extracellular domain of CD276, or the variant of CD27611. Preferably, one or more of said epitopes is displayed between amino acid residues 23 and 241 (ECD of human CD276 protein) of SEQ ID NO: 371, such as between amino acid residues 23 and 136 (Ig-like V-type domain of human CD276 protein) of SEQ ID NO: 371.

An ABP of the present invention may be mono-specific (i.e, it possesses antigen binding domain(s) that bind to only one antigen) or may be multi-specific (i.e, it possesses two or more different antigen binding domain(s) that bind to different antigens). For example, a "bi-specific", "dual-specific" or "bifunctional" ABP or antibody is a hybrid ABP or antibody, respectively, having two different antigen binding sites. Bi-specific antigen binding proteins and antibodies are a species of multi-specific antigen binding protein antibody and can be produced by a variety of methods including, but not limited to, fusion of hybridomas or linking of Fab' fragments (see, e.g., Songsivilai and Lachmann, 1990; Kostelny et al., 1992). The two binding sites of a bi-specific antigen binding protein or antibody will bind to two different epitopes, which can reside on the same or different protein targets.

In certain of such embodiments, the ABP may be a bi-specific, tri-specific, or tetra-specific antibody, in particular a bi-specific antibody is selected from: a bispecific T-cell engager (BiTE) antibody, a dual-affinity retargeting molecule (DART), a CrossMAb antibody, a DutaMab™ antibody, a DuoBody antibody; a Triomab, a TandAb, a bispecific NanoBody, Tandem scFv, a diabody, a single chain diabody, a HSA body, a (scFv)2 HSA Antibody, an scFv-IgG antibody, a Dock and Lock bispecific antibody, a DVD-IgG antibody, a TBTI DVD-IgG, an IgG-fynomer, a Tetravalent bispecific tandem IgG antibody, a dual-targeting domain antibody, a chemically linked bispecific (Fab')2 molecule, a crosslinked mAb, a Dual-action Fab IgG (DAF-IgG), an orthoFab-IgG, a bispecific CovX-Body, a bispecific hexavalent trimerbody, and an ART-Ig.

Accordingly, in certain embodiments, the ABP of the invention is a multi-specific antibody comprising at least two antigen binding domains, wherein each antigen binding domain specifically binds to a different antigen epitope.

Accordingly, in some embodiments, the ABP of the invention binds (e.g. via one or more first antigen binding domain(s)) to an extracellular domain(s) of the CD276, paralogue, orthologue or other variant when expressed on the surface of a mammalian cell, and in addition comprises one or more additional antigen binding domain(s) that bind(s) to antigen(s) other than said CD276 or variant. Such other antigen may, in certain embodiments of the inventive ABP, be another immunoglobulin superfamily gene; and/or such other antigen may be an antigen present on a mammalian T-cell. Antigens present on a mammalian T-cell, that may be bound by such an additional antigen binding domain, include CD3, CD40, OX-40, ICOS and 4-1BB. Such other antigen may, in certain embodiments of the inventive ABP, also be albumin, e.g., human albumin. It may also be another component of blood or blood serum the binding of which by the ABP will confer an extended serum half-life upon the ABP, e.g., a half-life similar to that when bound to albumin.

Preferred variants of the ABP of the invention pertain to bispecifics, which comprise the antigen binding regions of the antibodies of the invention and a second antigen binding region directed at CD3, for example an scFv construct known as UCHT1. One preferred construct of the invention comprises two antigen binding domains of the herein designated 7C4 antibody and two anti-CD3 binding domains, for example the aforementioned UCHT1 scFv construct.

In other embodiments, an ABP of the invention can comprise two or more antigen binding regions, preferably comprising two, three or four antigen binding regions.

In yet other embodiments, an ABP of the invention can comprise a chimeric antigen receptor (CAR), and preferably comprises an extracellular antigen binding region, a membrane anchor such as a transmembrane domain, and an intracellular region, for example, an intracellular signalling region.

In preferred embodiments, an ABP of the invention can comprise at least one antibody constant domain, in particular wherein at least one antibody constant domain is a CH1, CH2, or CH3 domain, or a combination thereof.

In further of such embodiments, an ABP of the invention having antibody constant domain comprises a mutated Fc region, for example for increasing interaction of the Fc region with a Fc receptor (Fc receptor on an immune effector cell (eg Saxena & Wu, 2016; Front Immunol 7:580). Examples and embodiments thereof are described elsewhere herein.

In other embodiments, an ABP of the invention may comprises an effector group and/or a labelling group.

The term "effector group" means any group, in particular one coupled to another molecule such as an antigen binding protein, that acts as a cytotoxic agent. Examples for suitable effector groups are radioisotopes or radionuclides. Other suitable effector groups include toxins, therapeutic groups, or chemotherapeutic groups. Examples of suitable effector groups include calicheamicins, auristatins, geldanamycins, alpha-amanitine, pyrrolobenzodiazepines and maytansines.

The term "label" or "labelling group" refers to any detectable label. In general, labels fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, β-galactosidase, luciferase, alkaline phosphatase); e) biotinylated groups; and f) predetermined polypeptide epitopes recognized by a secondary reporter (e.g., leucine zipper pair sequences, binding sites for secondary antibodies, metal binding domains, epitope tags, etc.).

In some embodiments, an effector group or a labelling group is coupled to another molecule (such as the ABP) via spacer arms of various lengths to reduce potential steric hindrance.

Preferred embodiments of the invention then pertain to ABPs which are modified to comprise an immunocytokine functional group. Preferred is the use of Interleukin 15 (IL-15) or modified IL-15. However, the use of other immunocytokines for antibody fusion constructs is well documented and includes antibody-cytokine fusion proteins containing IL-2, IL-12, IL-21, TNFα, and interferons α, β and γ.

A preferred IL15 molecule to be used as a fusion protein with an ABP of the invention is a molecule that has a reduced affinity for IL-15Rα compared to the affinity of wild-type IL-15. A modified IL-15 polypeptide to be used in the present invention preferably comprises at least one amino acid substitution at one or more positions corresponding to position(s) 92, 93, 94, 95, 96, 97, 98, 99, 100, 112, 113, 114, 115 and/or 116 of the amino acid sequence of human wild type IL-15 (which is derivable from UniProt under the accession number: P40933-1). Therefore, preferably an ABP of the invention may comprise one or two antigen binding domains as disclosed herein, and for example fused to an antibody constant region, one or more of the above mentioned modified IL-15 polypeptides. Such constructs are disclosed in EP 3 265 478, which is enclosed herein by reference in its entirety).

In **a second aspect,** the invention pertains a bispecific antigen binding protein (ABP) which comprises a first antigen binding domain capable of binding to the CD276 (B7-H3) antigen, or the variant thereof, and a second antigen binding domain capable of binding to the human cluster of differentiation 3 (CD3) antigen.

A "bispecific" or "bifunctional" ABP is an ABP that has two different epitope/antigen binding domains (or "sites"), and accordingly has binding specificities for two different target epitopes. These two epitopes may be epitopes of the same antigen or, as preferred in the present invention, of different antigens, such as the different antigens CD276 and CD3.

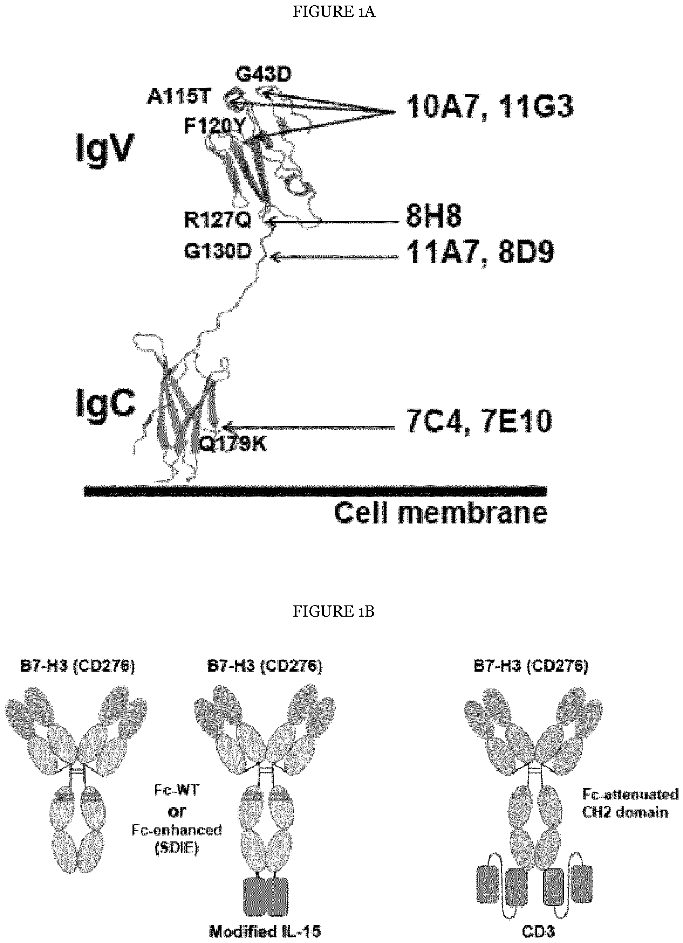

A "bispecific ABP", may be an ABP that binds one antigen or epitope with one of two or more binding arms, defined by a first pair of heavy and light chain or of main and shorter/smaller chain, and binds a different antigen or epitope on a second arm, defined by a second pair of heavy and light chain or of main and smaller chain. Such an embodiment of a bispecific ABP has two distinct antigen binding arms, in both specificity and CDR sequences. Typically, a bispecific ABP is monovalent for each antigen it binds to, that is, it binds with only one arm to the respective antigen or epitope. However, bispecific antibodies can also be dimerized or multimerized, which is preferred in context of the present invention. For example, in the dimeric IgGsc format as described herein, the antibody may have two binding sites for each antigen (figure 1B, third side construct). A bispecific antibody may be a hybrid ABP, which may have a first binding region that is defined by a first light chain variable region and a first heavy chain variable region, and a second binding region that is defined by a second light chain variable region and a second heavy chain variable region. It is envisioned by the invention that one of these binding regions may be defined by a heavy/light chain pair. In the context of the present invention the bispecific ABP may have a first binding site, defined by variable regions of a main chain and a smaller chain, and a second, different binding site defined by a variable region of a scFv fragment that is included in the main chain of the ABP.

Methods of making a bispecific ABP are known in the art, e.g. chemical conjugation of two different monoclonal antibodies or for example, also chemical conjugation of two antibody fragments, for example, of two Fab fragments. Alternatively, bispecific ABPs are made by quadroma technology, that is by fusion of the hybridomas producing the parental antibodies. Because of the random assortment of H and L chains, a potential mixture of ten different antibody structures are produced of which only one has the desired binding specificity.

The bispecific ABP of the invention can act as a monoclonal antibody (mAb) with respect to each target. In some embodiments the antibody is chimeric, humanized or fully human. A bispecific ABP may for example be a bispecific tandem single chain Fv, a bispecific Fab2, or a bispecific diabody.

On the basis of the domains included in an ABP of the invention the bispecific ABP of the invention may comprise a Fab fragment, which may generally include a hinge region, a CH2 domain and a single chain Fv fragment. Such bispecific ABPs are termed "Fabsc"-ABPs and have been described for the first time in International patent application WO 2013/092001. More specifically, a "Fabsc" format ABP as used here typically refers to a bispecific ABP of the invention having a Fab fragment, which generally includes a hinge region, which is at the C-terminus of the Fab fragment linked to the N-terminus of a CH2 domain, of which the C-terminus is in turn linked to the N-terminus of a scFv fragment. Such a "Fabsc" does not or does not essentially comprise a CH3 domain. In this context, "not comprising" or "not essentially comprising" means that the ABP does not comprise a full length CH3 domain. It preferably means that the ABP comprises 10 or less, preferably 5 or less, preferably 3 or even less amino acids of the CH3 domain.

In accordance with the publication of Coloma and Morrison (Nat Biotechnol 15:159-63, 1997), a bispecific ABP of the invention may also have a CH3 domain, generally arranged C-terminally of the CH2 domain. Such a molecule is also referred to herein as an "IgGsc" format ABP and means a bispecific ABP of the invention having a Fab fragment, which generally includes a hinge region, which is at the C-terminus of the Fab fragment typically linked to the N-terminus of a CH2 domain, of which the C-terminus is in turn typically linked to the N-terminus of a CH3 domain, of which the C-terminus is in turn typically linked to the N-terminus of a scFv fragment. An illustrative example of an IgGsc format ABP is shown in Fig. 1B. Such bispecific ABP format is preferred in context of the present invention.

In addition, or alternatively, an "Fabsc" or "IgGsc"ABP of the invention may also have an "Fc-attenuated" CH2 domain (that includes the hinge region). This "Fc-attenuation" is achieved by deleting and/ or substituting (mutating) at least one of selected amino acid residues in the CH2 domain that are able to mediate binding to an Fc-receptor. In illustrative embodiments, the at least one amino acid residue of the hinge region or the CH2 domain that is able to mediate binding to Fc receptors and that is lacking or mutated, is selected from the group consisting of sequence position 228, 230, 231, 232, 233, 234, 235, 236, 237, 238, 265, 297, 327, and 330 (numbering of sequence positions according to the EU-index). In an illustrative example, such an Fc-attenuated ABP may contain at least one mutation selected from the group consisting of a deletion of amino acid 228, a deletion of amino acid 229, a deletion of amino acid 230, a deletion of amino acid 231, a deletion of amino acid 232, a deletion of amino acid 233, a substitution Glu233→Pro, a substitution Leu234→Val, a deletion of amino acid 234, a substitution Leu235→Ala, a deletion of amino acid 235, a deletion of amino acid 236, a deletion of amino acid 237, a deletion of amino acid 238, a substitution Asp265→Gly, a substitution Asn297→Gln, a substitution Ala327→Gln, and a substitution Ala330→Ser (numbering of sequence positions according to the EU-index, see in respect, for example, also Fig. 1O and Fig. 1P of International patent application WO 2013/092001). In the case of bispecific antibodies that activate T cells, e.g. against tumor cells, Fc-attenuation may be desired to prevent binding of the antibodies to Fc-receptor carrying cells which may lead to undesirable off-target activation of T cells.

In accordance with the publication of Coloma and Morrison (Nat Biotechnol 15:159-63, 1997), a bispecific ABP which is in the IgGsc format, which is most preferred, of the invention may also have a CH3 domain, generally arranged C-terminally of the CH2 domain. Such a molecule is also referred to herein as an "IgGsc" format ABP and means a bispecific ABP of the invention having a Fab fragment, which generally includes a hinge region, which is at the C-terminus of the Fab fragment typically linked to the N-terminus of a CH2 domain, of which the C-terminus is in turn typically linked to the N-terminus of a CH3 domain, of which the C-terminus is in turn typically linked to the N-terminus of a scFv fragment. An illustrative example of an IgGsc format ABP is shown in Fig. 1B. Such bispecific ABP format is preferred in context of the present invention.

The antibody formats Fabsc and IgGsc have both in common that the N-terminal targeting part consists of "physiological" Fab- or Fab2 regions, respectively, thereby avoiding the use of single chain moieties in this part of the molecule. If these formats are to be used for target cell restricted T cell activation, attenuation of Fc receptor (FcR) binding may be employed (if wanted or required) to prevent FcR mediated activation. This can be achieved e.g. by introduction of defined and well-known mutations in the CH2 domain of the molecule as described in above and also in International patent application WO 2013/092001 and in Armour et al. Eur J Immunol 1999; 29:2613. Accordingly, also an IgGsc ABP of the invention may have a CH2 domain (including the hinge region) in which at least one amino acid residue of the hinge region or the CH2 domain that is able to mediate binding to Fc receptors is lacking or mutated. As explained above, this residue in the CH2 and hinge region, respectively, may be selected from the group consisting of sequence position 228, 230, 231, 232, 233, 234, 235, 236, 237, 238, 265, 297, 327, and 330 (numbering of sequence positions according to the EU-index). However, due to the presence of the CH3 domain in the IgGsc molecule, two individual molecules will (spontaneously) homodimerize via the CH3 domain to form a tetravalent molecule (see again Fig. 1B in this respect). Thus, it is not necessary to delete or mutate the cysteine residues at sequence position 226 and/or sequence position 229 of the hinge region. Thus, such a tetrameric IgGsc ABP of the invention may have a cysteine residue at sequence position 226 and/or at sequence position 229 of one of the respective hinge domain, in line with the Kabat numbering [EU-Index].

In line with the above disclosure of the bispecific ABPs that contain a set of CDR regions that mediate CD276 binding and/or binding to leukemic cancer cells, the ABP of the present invention may comprise a second binding site that specifically binds to a receptor on an immune cell such as a T cell or an NK cells. This receptor present on the immune cell may be a receptor that is capable of activating the immune cell or of stimulating an immune response of the immune cell. The evoked immune response may preferably be a cytotoxic immune response. Such a suitable receptor may, for example, be CD3, the antigen specific T cell receptor (TCR), CD28, CD16, NKG2D, Ox40, 4-1 BB, CD2, CD5, programmed cell death protein 1 (PD-1) and CD95. Particularly preferred is an ABP in which the second binding site binds to CD3, TCR or CD16. Most preferred is an ABP, in which the second binding site specifically binds to CD3. One preferred ABP comprises a second binding site that corresponds to the antigen binding site of the anti-CD3 antibody OKT3. The amino acid sequence of the variable domain of the heavy chain and of the variable domain of the light chain of the antibody OKT3 are, for example, also described in Arakawa et al J. Biochem. 120, 657-662 (1996) and International Patent Application WO 2015/158868 (see SEQ ID NOS: 17 and 18 in the Sequence Listings of WO 2015/158868). Another preferred ABP comprises a second binding site that corresponds to the antigen binding site of the anti-CD3 antibody UCHT1. The VH and VL sequences of a humanized UCHT1 antibody are described in International Patent Application WO 2013/092001. Other examples of CD3 binding ABPs that can be used in the present invention include the ABPs described in European Patent 2 155 783 B1 or European Patent EP 2 155 788 B1 that are capable of binding to an epitope of human and Callithrix jacchus, Saguinus oedipus or Saimiri sciureus CD3ε chain.

Accordingly, the bispecific ABP of the invention may be a bispecific ABP such as a IgGsc-molecule that comprises a Fab fragment and a scFv fragment as described herein. In this molecule the first binding site may bind to CD276 and may be comprised in a Fab (or a bivalent CD276 F(ab)2 in context of the IgGsc format) fragment as described herein and the second binding site (that may bind to an immune receptor) may be comprised in a scFv fragment, such as an scFv binding specifically to CD3. Alternatively, the first binding site that binds to CD276 is comprised in a single chain Fv fragment and the second binding site (that may bind to CD3) is comprised in a Fab fragment.

In some embodiments, the bispecific ABP of the invention does not by itself activate the immune cell, e.g. the T cell, upon binding, such as binding to CD3. Instead, only when both binding sites, e.g. the CD276-specific binding sited and the CD3 specific binding site are bound to the receptor on the T cell and to CD276 on the target cancer cell, the former may cross-link the activating receptor, triggering the effector cells to kill the specific target cell. Standard functional assays to evaluate the target cell -killing capability by lymphocytes in the presence and absence of an bispecific ABP of the invention can be set up to assess and/or screen for the ability of the ABP to activate the receptor to which it binds.

In some embodiments of the invention the bispecific ABP comprises as second antigen binding domain a scFv of and anti-CD3 antibody, such as UCHT1 or variants thereof.

In **a third aspect**, the invention pertains to an isolated nucleic acid comprising a sequence encoding for an ABP, or for an antigen binding fragment or a monomer, such as a heavy or light chain, of an ABP, of any one of the first aspect, or encoding for a bispecific ABP according to the second aspect.

For example, the component encoded by a nucleic acid of the invention may be all or part of one chain of an antibody of the invention; or the component may be a scFv of said ABP. The component encoded by such a nucleic acid may be all or part of one or other of the chains of an antibody of the invention; for example, the component encoded by such a nucleic acid may be an ABD of the invention. The nucleic acids of the invention may also encode a fragment, derivative, mutant, or variant of an ABP of the invention, and/or represent components that are polynucleotides suitable and/or sufficient for use as hybridisation probes, polymerase chain reaction (PCR) primers or sequencing primers for identifying, analyzing, mutating or amplifying a polynucleotide encoding a polypeptide, anti-sense or inhibitory nucleic acids (such as RNAi/siRNA/shRNA or gRNA molecules) for inhibiting expression of a polynucleotide, and complementary sequences of the foregoing.

In particular embodiments of the invention, a nucleic acid of the invention comprises a nucleic acid having a sequence encoding a heavy or light chain CDR, a combination of heavy and/or light chain CDR1, CDR2 and CDR3 or a heavy or light chain variable domain, in each case as displayed in Table 1, or a functional fragment thereof. In other embodiments, a nucleic acid of the invention comprises a nucleic acid sequence at least 60%, 65%, 70%, 75%, 80%, 85%, 90%; or 95% (preferably at least 75%) sequence identity to (or having no more than fifty, forty, thirty, twenty, fifteen, ten or five, preferably no more than three, two or one, base substitution(s), insertion(s) or deletion(s), to a sequence encoding any of the herein disclosed CDRs, preferably CDR3, in table 1.

The nucleic acid according to the invention may be a DNA or RNA of genomic, mRNA, cDNA, or synthetic origin or some combination thereof, optionally linked to a polynucleotide to which it is not linked in nature. In some embodiments, such nucleic acid may comprise one or more (such as 2, 3, 4, 5, 6, 7, 8, 9, 10 or more than 20, in particular between 1 and about 5, or preferably all instances of a particular nucleotide in the sequence) unnatural (e.g. synthetic) nucleotides; and/or such nucleic acid may comprise (e.g. is conjugated to) another chemical moiety, such as a labelling group or an effector group; for example, a labelling group or an effector group as described elsewhere herein.

In one embodiment, the nucleic acid of the invention may be isolated or substantially pure. In another embodiment, the nucleic acid of the invention may be recombinant, synthetic and/or modified, or in any other way non-natural. For example, a nucleic acid of the invention may contain at least one nucleic acid substitution (or deletion) modification (such as 2, 3, 4, 5, 6, 7, 8, 9, 10 or more than 10 such modifications, in particular between 1 and about 5 such modifications, preferably 2 or 3 such modifications) relative to a product of nature, such as a human nucleic acid.

The nucleic acids can be any suitable length, such as about 10, 15, 20, 25, 30, 35, 40, 45, 50, 75, 100, 125, 150, 175, 200, 250, 300, 350, 400, 450, 500, 750, 1,000, 1,500, 3,000, 5,000 or more nucleotides in length. For example: siRNA nucleic acids may, preferably, be between about 15 to about 25 base pairs in length (preferably between about 19 and about 21 base pairs in length); shRNA nucleic acids may, preferably, comprise a 20-30 base pair stem, a loop of at least 4 nucleotides, and a dinucleotide overhang at the 3' end; microRNA may, preferably, be about 22 base pairs in length; an mRNA or DNA sequence encoding an ABP or a component thereof (such as a heavy or light chain or an IgG antibody) of the invention may, preferably, be between about 500 and 1,500 nucleotides. More preferably, a nucleic acid encoding a mammalian light chain of an antibody may be between about 630 and about 650 nucleotides, and one encoding a mammalian heavy chain of an antibody may be between about 1,300 and about 1,650 nucleotides. A nucleic acid can comprise one or more additional sequences, for example, regulatory sequences, and/or be part of a larger nucleic acid. The nucleic acids can be single-stranded or double-stranded and can comprise RNA and/or DNA nucleotides, and artificial variants thereof (e.g., peptide nucleic acids).

Nucleic acids encoding antibody polypeptides (e.g., heavy or light chain, variable domain only, or full length) may be isolated from B-cells of mice, rats, llamas, alpacas, chicken or rabbits that have been immunized with an CD276 antigen or fragment thereof, such as one or more domains (or a polynucleotide encoding and capable of expressing an CD276 antigen or fragment thereof). The nucleic acid may be isolated by conventional procedures such as PCR.

Changes can be introduced by mutation into the sequence of a nucleic acid of the invention. Such changes, depending on their nature and location in a codon, can lead to changes in the amino acid sequence of a polypeptide (e.g., an antigen binding protein) that it encodes. Mutations can be introduced using any technique known in the art.

In one embodiment, one or more particular amino acid residues may be changed using, for example, a site-directed mutagenesis protocol. In another embodiment, one or more randomly selected residues may be changed using, for example, a random mutagenesis protocol. However, it is made, a mutant polypeptide can be expressed and screened for a desired property. Mutations can be introduced into a nucleic acid without significantly altering the biological activity of a polypeptide that it encodes. For example, one can make nucleotide substitutions leading to amino acid substitutions at non-essential amino acid residues.

Other changes that may be made (e.g. by mutation) to the sequence of a nucleic acid of the invention may not alter the amino acid sequence of the encoded polypeptide, but may lead to changes to its stability and/or effectiveness of expression of the encoded polypeptide. For example, by codon optimisation, the expression of a given polypeptide sequence may be improved by utilising the more common codons for a given amino acid that are found for the species in which the nucleotide is to be expressed. Methods of codon optimisation, and alternative methods (such as optimisation of CpG and G/C content), are described in, for example, Hass et al, 1996 (Current Biology 6:315); WO1996/09378; WO2006/015789 and WO 2002/098443).

In **a fourth aspect,** the invention pertains to a nucleic acid construct (NAC) comprising a nucleic acid of the third aspect and one or more additional sequence features permitting the expression of the encoded ABP or bispecific ABP, or a component of said ABP or bispecific ABP (such as an antibody heavy chain or light chain) in a cell.

Such an NAC can comprise one or more additional features permitting the expression of the encoded ABP or component of said ABP (eg the ABD) in a cell (such as in a host cell). Examples of NACs of the invention include, but are not limited to, plasmid vectors, viral vectors, mRNA, non-episomal mammalian vectors and expression vectors, for example, recombinant expression vectors. The nucleic acid constructs of the invention can comprise a nucleic acid of the invention in a form suitable for expression of the nucleic acid in a cell, such as a host cell, (see below). The nucleic acid constructs of the invention will be, typically, recombinant nucleic acids, and/or may be isolated and/or substantially pure. Recombinant nucleic acids will, typically, be non-natural; particularly if they comprise portions that are derived from different species and/or synthetic, in-vitro or mutagenic methods.

In some embodiments, an NAC of the invention comprises one or more constructs either of which includes a nucleic acid encoding either a heavy or a light antibody chain. In some embodiments, the NAC of the invention comprises two constructs, one of which includes a nucleic acid encoding the heavy antibody chain, the other of which includes a nucleic acid encoding the light antibody chain, such that expression from both constructs can generate a complete antibody molecule. In some embodiments, the NAC of the invention comprises a construct which includes nucleic acids encoding both heavy and light antibody chains, such that a complete antibody molecule can be expressed from one construct. In other embodiments, an NAC of the invention can comprise a single construct that encodes a single chain which is sufficient to form an ABP of the invention; for example, if the encoded ABP is a scFv or a single-domain antibody (such as a camelid antibody).

In some embodiments, the NAC of the invention includes sequences encoding all or part of a constant region, enabling an entire, or a part of, a heavy and/or light chain to be expressed.

An NAC according to the invention may comprise (or consist of) a mRNA molecule which includes an open reading frame encoding an ABP of the invention, and for example together with upstream and downstream elements (such as 5' and/or 3' UTRs and/or poly-A stretch) that enables expression of the ABP, and preferably enhancing stability of the mRNA and/or expression of the ABP. The use of mRNA as NACs to introduce into and express polynucleotides in cells is described, for example, in Zangi et al in Nat. Biotechnol. vol. 31, 898-907 (2013), Sahin et al (2014) Nature Reviews Drug Discovery 13:759 and by Thess et al in Mol. Ther. vol. 23 no.9, 1456-1464 (2015). Particular UTRs that may be comprised in an mRNA NAC of the invention include: 5'UTR of a TOP gene (WO2013/143699), and/or a histone stem-loop (WO 2013/120629). An mRNA NAC of the invention may further comprise one or more chemical modifications (EP 1 685 844); including a 5'-cap, such as m7G(5')ppp, (5'(A,G(5')ppp(5')A or G(5')ppp(5')G and/or at least one nucleotide that is an analogue of naturally occurring nucleotides, such as phosphorothioates, phosphoroamidates, peptide nucleotides, methylphosphonates, 7-deaza-guanosine, 5-methylcytosine or inosine.

NACs, such as DNA-, retroviral- and mRNA-based NACs of the invention may be used in genetic therapeutic methods in order to treat or prevent diseases of the immune system (see Methods of Treatment below), whereby an NAC that comprises an expressible sequence encoding an ABP of the invention is administered to the cell or organism (e.g. by transfection). In particular, the use of mRNA therapeutics for the expression of antibodies is known from WO2008/083949.

In **a fifth aspect,** the invention pertains to a recombinant host cell comprising a nucleic acid or a NAC according to the third or fourth aspect. Preferably, such cell is capable of expressing the ABP (or component thereof) encoded by said NAC(s). For example, if an ABP of the invention comprises two separate polypeptide chains (e.g. a heavy and light chain of an IgG), then the cell of the invention may comprise a first NAC that encodes (and can express) the heavy chain of such ABP as well as a second NAC that encodes (and can express) the light chain of such ABP; alternatively, the cell may comprise a single NAC that encodes both chains of such ABP. In these ways, such a cell of the invention would be capable of expressing a functional (e.g. binding and/or inhibitory) ABP of the invention. A (host) cell of invention may be one of the mammalian, prokaryotic or eukaryotic host cells as described elsewhere herein, in particularly where the cell is a Chinese hamster ovary (CHO) cell.

In certain embodiments of such aspect, the (host) cell is a human cell; in particular it may be a human cell that has been sampled from a specific individual (eg an autologous human cell). In such embodiments, such human cell can be propagated and/or manipulated in-vitro so as to introduce a NAC of the present invention. The utility of a manipulated human cell from a specific individual can be to produce an ABP of the invention, including to reintroduce a population of such manipulated human cells into a human subject, such as for use in therapy. In certain of such uses, the manipulated human cell may be introduced into the same human individual from which it was first sampled; for example, as an autologous human cell.

The human cell that is subject to such manipulation can be of any germ cell or somatic cell type in the body. For example, the donor cell can be a germ cell or a somatic cell selected from the group consisting of fibroblasts, B cells, T cells, dendritic cells, keratinocytes, adipose cells, epithelial cells, epidermal cells, chondrocytes, cumulus cells, neural cells, glial cells, astrocytes, cardiac cells, oesophageal cells, muscle cells, melanocytes, hematopoietic cells, macrophages, monocytes, and mononuclear cells. The donor cell can be obtained from any organ or tissue in the body; for example, it can be a cell from an organ selected from the group consisting of liver, stomach, intestines, lung, pancreas, cornea, skin, gallbladder, ovary, testes, kidneys, heart, bladder, and urethra.

In **a sixth aspect,** the invention pertains to a pharmaceutical composition comprising: (i) an ABP or bispecific ABP of the first or second aspect, or (ii) a nucleic acid or NAC of the third or fourth aspect, or (iii) a recombinant host cell according to the fifth aspect, and a pharmaceutically acceptable carrier, stabiliser and/or excipient.

To be used in therapy, the ABPs, nucleic acids or NACs (or the cells, such as host cells) of the invention may be formulated into a pharmaceutical composition appropriate to facilitate administration to animals or humans. The term "pharmaceutical composition" means a mixture of substances including a therapeutically active substance (such as an ABP of the invention) for pharmaceutical use.

By way of example, the pharmaceutical composition of the invention may comprise between 0.1% and 100% (w/w) active ingredient, such as about 0.5%, 1%, 2%, 3%, 4%, 5%, 6%, 8% 10%, 15%, 20%, 25%, 30%, 40%, 50%, 60%, 70%, 80%, 90%, 95%, 96%, 97%, 98% or 99%, preferably between about 1% and about 20%, between about 10% and 50% or between about 40% and 90%.

As used herein the language "pharmaceutically acceptable" excipient, stabiliser or carrier is intended to include any and all solvents, solubilisers, fillers, stabilisers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of (or for use with) the invention is, typically, formulated to be compatible with its intended route of administration. Examples of routes of administration include oral, parenteral, e.g., intrathecal, intra-arterial, intravenous, intradermal, subcutaneous, oral, transdermal (topical) and transmucosal administration.

In some embodiments, the pharmaceutical composition comprising an ABP or NAC is in unit dose form of between 10 and 1000mg. In some embodiments, the pharmaceutical composition comprising an ABP or NAC is in unit dose form of between 10 and 200mg. In some embodiments, the pharmaceutical composition comprising an ABP is in unit dose form of between 200 and 400mg. In some embodiments, the pharmaceutical composition comprising an ABP or NAC is in unit dose form of between 400 and 600mg. In some embodiments, the pharmaceutical composition comprising an ABP or NAC is in unit dose form of between 600 and 800mg. In some embodiments, the pharmaceutical composition comprising an ABP or NAC is in unit dose form of between 800 and 100 mg.

Exemplary unit dosage forms for pharmaceutical compositions comprising an ABP or NAC are tablets, capsules (eg as powder, granules, microtablets or micropellets), suspensions or as single-use pre-loaded syringes. In certain embodiments, kits are provided for producing a single-dose administration unit. The kit can contain both a first container having a dried active ingredient and a second container having an aqueous formulation. Alternatively, the kit can contain single and multi-chambered pre-loaded syringes.

Toxicity and therapeutic efficacy (eg effectiveness) of such active ingredients can be determined by standard pharmaceutical procedures in cell cultures or experimental animals, eg, for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Active agents which exhibit large therapeutic indices are preferred. While compounds that exhibit toxic side effects may be used, care should be taken to design a delivery system that targets such compounds to the site of affected tissue in order to minimise potential damage to uninfected cells and, thereby, reduce side effects.

In accordance with all aspects and embodiments of the medical uses and methods of treatment provided herein, the effective amount administered at least once to a subject in need of treatment with a ABP or NAC is, typically, between about 0.01mg/kg and about 100mg/kg per administration, such as between about 1mg/kg and about 10mg/kg per administration. In some embodiments, the effective amount administered at least once to said subject of a ABP or NAC is between about 0.01mg/kg and about 0.1mg/kg per administration, between about 0.1mg/kg and about 1mg/kg per administration, between about 1mg/kg and about 5mg/kg per administration, between about 5mg/kg and about 10mg/kg per administration, between about 10mg/kg and about 50mg/kg per administration, or between about 50mg/kg and about 100mg/kg per administration.

For the prevention or treatment of disease, the appropriate dosage of a ABP or NAC (or a pharmaceutical composition comprised thereof) will depend on the type of disease to be treated, the severity and course of the disease, whether the ABP or NAC and/or pharmaceutical composition is administered for preventive or therapeutic purposes, previous therapy, the patient's clinical history, age, size/weight and response to the ABP or NAC and/or pharmaceutical composition, and the discretion of the attending physician. The ABP or NAC and/or pharmaceutical composition is suitably administered to the patient at one time or over a series of treatments. If such ABP or NAC and/or pharmaceutical composition is administered over a series of treatments, the total number of administrations for a given course of treatment may consist of a total of about 2, 3, 4, 5, 6, 7, 8, 9, 10 or more than about 10 treatments. For example, a treatment may be given once every day (or 2, 3 or 4 times a day) for a week, a month or even several months. In certain embodiments, the course of treatment may continue indefinitely.

In a **seventh aspect,** the invention pertains to a component for use in medicine, wherein the component is selected from the list consisting of: (i) an ABP or bispecific ABP of the first or second aspect, or (ii) a nucleic acid or NAC of the third or fourth aspect, or (iii) a recombinant host cell according to the fifth aspect and (iv) a pharmaceutical composition according to the sixth aspect.

In a related aspect, the invention also relates to method of treating or preventing a disease, disorder or condition in a mammalian subject in need thereof, comprising administering to said subject at least once an effective amount of modulating compound as desired above, or, and in particular administering to said subject at least once an effective amount of the ABP, the NAC, the (host) cells, or the pharmaceutical composition as described above.

In another related aspect, the invention also relates to the use of a product of the invention as describe above, or a modulating compound as described above (in particular an ABP of the invention) for the manufacture of a medicament, in particular for the treatment of a disease, disorder or condition in a mammalian subject, in particular where the disease, disorder or condition is one as set out herein.

The term "treatment" in the present invention is meant to include therapy, e.g. therapeutic treatment, as well as prophylactic or suppressive measures for a disease (or disorder or condition). Thus, for example, successful administration of a compound according to the invention prior to onset of the disease results in treatment of the disease. "Treatment" also encompasses administration of a compound of the invention after the appearance of the disease in order to ameliorate or eradicate the disease (or symptoms thereof). Administration of CD276 binding compound of the invention after onset and after clinical symptoms, with possible abatement of clinical symptoms and perhaps amelioration of the disease, also comprises treatment of the disease. Those "in need of treatment" include subjects (such as a human subject) already having the disease, disorder or condition, as well as those prone to or suspected of having the disease, disorder or condition, including those in which the disease, disorder or condition is to be prevented.

In particular embodiments of these aspects, the modulating compound is one described above, and/or is an ABP, NAC, a (host) cell, or a pharmaceutical composition of the present invention; in particular is an ABP of the invention.

Such a compound can, for example, be a compound (such as an ABP or inhibitors nucleic acid) that enhances killing and/or lysis of cells expressing CD276, or a variant of CD276, by Natural Killer Cells (ADCC) and/or CDC.

In other aspects described elsewhere herein, are provided methods to detect and/or diagnose a disease, disorder or condition in a mammalian subject.

In one particular embodiment, the disease, disorder or condition that is characterized by a pathological immune response.

In a further particular embodiment, the disease, disorder or condition is characterized by expression of CD276, or a variant of CD276, in particular by expression of CD276, or a variant of CD276by cells associated with the disease, disorder or condition, such as cancer cells. For example, the disease, disorder or condition can be associated with the undesired presence of CD276-positive cells or cells positive for a variant of CD276. Preferably, the disease, disorder or condition is characterized by a vasculature that comprises cells expressing the CD276 or variant thereof.

A disorder, disorder or condition treatable by the subject matter of the invention is, in certain alterative embodiments, one characterized by expression of CD276; in particular, one characterized by such expression that is aberrant, for example over- (or under-) expression or representation or activity of CD276 in a given cell or tissue (such as those cells or tissues involved with the proliferative disease of the subject) compared to that in a healthy subject or a normal cell.

In yet a further particular embodiment, the disease, disorder or condition is characterized by expression and/or activity of CD276, in particular such cells express mRNA and/or protein of CD276, and/or are positive for such CD276 expression and/or activity.

In another particular embodiment, the disease, disorder or condition is a proliferative disorder (or a condition associated with such disorder or disease), in particular when the product or modulating compound (such as a ABP, nucleic acid, NAC or recombinant host cell of the invention, in particular an ABP of the invention).

A "proliferative disorder" refers to a disorder characterized by abnormal proliferation of cells. A proliferative disorder does not imply any limitation with respect to the rate of cell growth, but merely indicates loss of normal controls that affect growth and cell division. Thus, in some embodiments, cells of a proliferative disorder can have the same cell division rates as normal cells but do not respond to signals that limit such growth. Within the ambit of "proliferative disorder" is neoplasm or tumor, which is an abnormal growth of tissue or cells. Cancer is art understood, and includes any of various malignant neoplasms characterized by the proliferation of cells that have the capability to invade surrounding tissue and/or metastasise to new colonisation sites. Proliferative disorders include cancer, atherosclerosis, rheumatoid arthritis, idiopathic pulmonary fibrosis and cirrhosis of the liver. Non-cancerous proliferative disorders also include hyperproliferation of cells in the skin such as psoriasis and its varied clinical forms, Reiter's syndrome, pityriasis rubra pilaris, and hyperproliferative variants of disorders of keratinization (e.g., actinic keratosis, senile keratosis), scleroderma, and the like.

In more particular embodiments, the proliferative disorder is a cancer or tumor, in particular a solid tumor (or a condition associated with such cancer or tumor). Such proliferative disorders include, but are not limited to, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, any haemotological malignancy (e.g., chronic lymphoblastic leukemia, chronic myelomonocytic leukemia, acute lymphoblastic leukemia, acute lymphocytic leukemia, acute myelogenous leukemia, acute myeloblasts leukemia, chronic myeloblastic leukemia, Hodgkin's disease, non- Hodgkin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, marginal zone lymphoma, Burkitt Lymphoma, mycosis fungoides, seary syndrome, cutaneous T-cell lymphoma, peripheral T cell lymphoma, chronic myeloproliferative disorders, myelofibrosis, myeloid metaplasia, systemic mastocytosis), and cental nervous system tumors (e.g., brain cancer, glioblastoma, non- glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and choroid plexus papilloma), myeloproliferative disorders (e.g., polycythemia vera, thrombocythemia, idiopathic myelofibrosis), soft tissue sarcoma, thyroid cancer, endometrial cancer, carcinoid cancer, or liver cancer.

In one preferred embodiment, the various aspects of the invention relate to, for example the ABPs of the invention used to detect/diagnose, prevent and/or treat, such proliferative disorders that include but are not limited to carcinoma (including breast cancer, prostate cancer, gastric cancer, lung cancer, colorectal and/ or colon cancer, hepatocellular carcinoma, melanoma), lymphoma (including non-Hodgkin's lymphoma and mycosis fungoides), leukemia, sarcoma, mesothelioma, brain cancer (including glioma), germinoma (including testicular cancer and ovarian cancer), choriocarcinoma, renal cancer, pancreatic cancer, thyroid cancer, head and neck cancer, endometrial cancer, cervical cancer, bladder cancer, or stomach cancer.

Accordingly, in a preferred embodiment, the ABP, bispecific ABP, or NAC according to the invention, is for use in the prevention and/or treatment of a cancer, for example a cancer which is characterized by the presence of a cancer cell selected from the group consisting of a cell of an adrenal gland tumor, an AIDS-associated cancer, an alveolar soft part sarcoma, an astrocytic tumor, bladder cancer, bone cancer, a brain and spinal cord cancer, a metastatic brain tumor, a breast cancer, a carotid body tumors, a cervical cancer, a chondrosarcoma, a chordoma, a chromophobe renal cell carcinoma, a clear cell carcinoma, a colon cancer, a colorectal cancer, a cutaneous benign fibrous histiocytoma, a desmoplastic small round cell tumor, an ependymoma, a Ewing's tumor, an extraskeletal myxoid chondrosarcoma, a fibrogenesis imperfecta ossium, a fibrous dysplasia of the bone, a gallbladder or bile duct cancer, gastric cancer, a gestational trophoblastic disease, a germ cell tumor, a head and neck cancer, hepatocellular carcinoma, an islet cell tumor, a Kaposi's Sarcoma, a kidney cancer, a leukemia, a lipoma/benign lipomatous tumor, a liposarcoma/malignant lipomatous tumor, a liver cancer, a lymphoma, a lung cancer, a medulloblastoma, a melanoma, a meningioma, a multiple endocrine neoplasia, a multiple myeloma, a myelodysplastic syndrome, a neuroblastoma, a neuroendocrine tumors, an ovarian cancer, a pancreatic cancer, a papillary thyroid carcinoma, a parathyroid tumor, a pediatric cancer, a peripheral nerve sheath tumor, a phaeochromocytoma, a pituitary tumor, a prostate cancer, a posterious unveal melanoma, a rare hematologic disorder, a renal metastatic cancer, a rhabdoid tumor, a rhabdomysarcoma, a sarcoma, a skin cancer, a soft-tissue sarcoma, a squamous cell cancer, a stomach cancer, a synovial sarcoma, a testicular cancer, a thymic carcinoma, a thymoma, a thyroid metastatic cancer, and a uterine cancer.

In a particularly preferred embodiment, the disease, disorder or condition is a CD276-positive cancer or a cancer positive for the variant of CD276 and/or is a cancer characterized by the presence of CD276 positive vascular cells of the tumor environment. More preferably the cancer is a solid tumor and characterized by a dual expression of CD276, or the variant thereof, in both a cancer cell of the tumor tissue, and the expression of CD276 in a cell of the blood vessels of the tumor vascular system - dual targeting.

In other methods, the modulating (eg inhibiting) compound (eg an ABP, such as one of the present invention) may be used is in combination with a different anti-proliferative therapy, in particular a different anti-cancer therapy, in particular where the different anti-proliferative therapy is immunotherapy, in particular immunotherapy with a ligand to an immune checkpoint molecule. Accordingly, the composition can be for use in the treatment of a proliferative disorder in a subject in need thereof, where the subject is subjected to to co-treatment by immunotherapy, in particular co-therapy (eg combination treatment) with a ligand to an immune checkpoint molecule.

In such embodiments, the ligand is one that binds to an immune (inhibitory) checkpoint molecule. For example, such checkpoint molecule may be one selected from the group consisting of: A2AR, B7-H3, B7-H4, CTLA-4, IDO, KIR, LAG3, PD-1 (or one of its ligands PD-Li and PD-L2), TIM-3 (or its ligand galectin-9), TIGIT, or for example antigen binding molecules targeting FLT3, PSMA or other tumor associated targets. In particular of such embodiments, the ligand binds to a checkpoint molecule selected from: CTLA-4, PD-1 and PD-L1. In other more particular embodiments, the ligand is an antibody selected from the group consisting of: ipilimumab, nivolumab, pembrolizumab, BGB-A317, atezolizumab, avelumab and durvaluma; in particular an antibody selected from the group consisting of: ipilimumab (YERVOY), nivolumab (OPDIVO), pembrolizumab (KEYTRUDA) and atezolizumab (TECENTRIQ).

When a method or use in therapy of the present invention (eg, one involving an ABP of the invention) is used in combination treatments together with any of such other procedures (eg, another agent or a cancer immunotherapy, such as a ligand that binds to an immune (inhibitory) checkpoint molecule), then such method or use being a combination treatment regimen may comprise embodiments where such exposures/administrations are concomitant. In alternative embodiments, such administrations may be sequential; in particular those embodiments where an ABP of the invention is administered before such other procedure. For example, such compound of the invention may be sequentially administered within about 14 days of (eg before) the other procedure, such as within about 10 days, 7 days, 5 days, 2 days or 1 day of (eg before) the other procedure; and further including where the compound of the invention may be sequentially administered within about 48 hours, 24 hours, 12 hours, 8 hours, 6 hours, 4 hours, 2 hours, 1 hours, 30 mins, 15 mins or 5 mins of (eg before) the other procedure.

In **an eighth aspect,** the invention pertains to a method of enhancing a cell-mediated immune response to a human cell that expresses human CD276, comprising contacting said cell with (i) an ABP or bispecific ABP of the first or second aspect, or (ii) a nucleic acid or NAC of the third or fourth aspect, or (iii) a recombinant host cell according to the fifth aspect and (iv) a pharmaceutical composition according to the sixth aspect, in the presence of an immune cell, such as a T-cell or natural killer (NK) cell, thereby enhancing a cell-mediated immune response, preferably cytotoxicity, against said human cell.

In a **ninth aspect** the invention pertains to a method for the prevention and/or treatment of a proliferative disorder in a subject, comprising the administration of a therapeutically effective amount of a component recited in the seventh aspect to the subject; and wherein the proliferative disorder is characterized by an expression of CD276 in cells associated with the proliferative disorder.

As described above, in one aspect, herein provided is a cell, such as (recombinant) host cell or a hybridoma capable of expressing an ABP as described above. In an alternative aspect, herein provided is a cell, which comprises at least one NAC encoding an ABP or a component of an ABP as described above. Cells of the invention can be used in methods provided herein to produce the ABPs and/or NACs of the invention.

Accordingly, in another aspect the invention relates to a method of producing a recombinant cell line capable of expressing an ABP specific for CD276, or for a CD276 variant, the method comprising the steps of:
- providing a suitable host cell;
- providing at least one genetic construct comprising coding sequence(s) encoding the ABP of the present invention;
- introducing into said suitable host cell said genetic construct(s); and
- optionally, expressing said genetic construct(s) by said suitable host cell under conditions that allow for the expression of the ABP.

In yet another aspect, herein provided is a method of producing an ABP as described above, for example comprising culturing one or more cells of the invention under conditions allowing the expression of said ABP.

Accordingly, in another aspect, the invention relates to a method of producing an ABP specific for CD276, or for a CD276 variant, the method comprising the steps of:
- providing a hybridoma or (host) cell capable of expressing an ABP according to the invention, for example a recombinant cell line comprising at least one genetic construct comprising coding sequence(s) encoding said compound or ABP; and
- culturing said hybridoma or host cell under conditions that allow for the expression of the ABP.

The terms "of the [present] invention", "in accordance with the invention", "according to the invention" and the like, as used herein are intended to refer to all aspects and embodiments of the invention described and/or itemed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
**Item 1:** An isolated antigen binding protein (ABP) which specifically binds to a CD276 protein, or a variant thereof, and wherein the isolated ABP is able to induce an antibody dependent cell-mediate cytotoxicity in a cell expressing the CD276.
**Item** 2: The isolated ABP of item Item 1:, wherein the isolated ABP specifically binds to a Immunoglobulin-like V (IgV)- or C (IgC)-domain or to the FG loop region of the CD276 protein, or the variant thereof.
**Item** 3: The isolated ABP of item Item 1**:** or Item 2:, wherein the CD276 protein is human CD276 protein.
**Item 4:** The isolated ABP of any one of items Item 1**:** to Item 3:, wherein the isolated ABP specifically binds to a CD276 epitope which comprises an amino acid position of human CD276 selected from (i) Q179, (ii) G130, (iii) R127, and (iv) G43, A115, and/or F120; preferably wherein the ABP specifically binds to an (antibody) epitope which comprises Q179 of human CD276.
**Item 5**: The isolated ABP of any one of items Item 1**:** to Item 4:, comprising at least one Complementary Determining Region (CDR) 3 having an amino acid sequence with at least 80% sequence identity to, or having no more than three or two, preferably one amino acid substitution(s), deletion(s) or insertion(s) relative to, a sequence selected from SEQ ID NOs. 3, 7, 11, 15, 19, 23, 27, 31, 35, and 39.
**Item 6:** The isolated ABP of item 5, wherein said ABP further comprises at least one CDR1, and at least one CDR2.
**Item 7:** The isolated ABP of any one of items 1 to 6, wherein the ABP is an antibody or an antigen binding fragment thereof.
**Item 8:** The isolated ABP of any one of items 1 to 7, comprising an antibody heavy chain, or an antigen binding fragment thereof, and/or an antibody light chain, or an antigen binding fragment thereof.
**Item 9:** The isolated ABP of any of items 1 to 8, comprising an antibody heavy chain variable region, or an antigen binding fragment thereof, and/or an antibody light chain variable region, or an antigen binding fragment thereof.
**Item 10:** The isolated ABP of any of items 1 to 9, comprising an antibody heavy chain variable region CDR1, CDR2, and CDR3, and/or an antibody light chain variable region CDR1, CDR2, and CDR3.
**Item 11:** The isolated ABP of any of items 1 to 10, comprising an antibody heavy chain sequence and/or an antibody light chain sequence, or an antigen binding fragment thereof; wherein the antibody heavy chain sequence, or the fragment thereof, comprises a CDR3 having at least 80% sequence identity to, or having no more than three or two, preferably one amino acid substitution(s), deletion(s) or insertion(s) relative to, a sequence selected from SEQ ID NOs. 3, 11, 19, 27, and 35, and/or wherein antibody light chain sequence, or the fragment thereof, comprises a CDR3 having at least 80% sequence identity to, or having no more than three or two, preferably one amino acid substitution(s), deletion(s) or insertion(s) relative to, a sequence selected from SEQ ID NOs. 7, 15, 23, 31, and 39.
**Item 12**: The isolated ABP of any one of items 8 to 11, wherein the antibody heavy chain sequence, or the fragment thereof, further comprises a CDR1 having at least 80% sequence identity to, or having no more than three or two, preferably one amino acid substitution(s), deletion(s) or insertion(s) relative to, a sequence selected from SEQ ID NOs. 1, 9, 17, 25, and 33; and/or a CDR2 having at 80% sequence identity to, or having no more than three or two, preferably one amino acid substitution(s), deletion(s) or insertion(s) relative to, a sequence selected from SEQ ID Nos. 2, 10 ,18, 26, and 34.
**Item 13**: The isolated ABP of any one of items 8 to 12, wherein the antibody light chain sequence, or the fragment thereof, further comprises a CDR1 having at least 80% sequence identity to, or having no more than three or two, preferably one amino acid substitution(s), deletion(s) or insertion(s) relative to, a sequence selected from SEQ ID NOs. 5, 13, 21, 29, and 37; and/or a CDR2 having at least 80% sequence identity to, or having no more than three or two, preferably one amino acid substitution(s), deletion(s) or insertion(s) relative to, a sequence selected from SEQ ID NOs. 6, 14, 22, 30, and 38.
**Item 14**: The isolated ABP of any one of items 1 to 13, comprising an antibody variable chain sequence having at least 80% sequence identity to, or having no more than three or two, preferably one amino acid substitution(s), deletion(s) or insertion(s) relative to, a sequence selected from SEQ ID NOs. 4, 8, 12, 16, 20, 24, 28, 32, 36, and 40.
**Item 15**: The isolated ABP of any of items 1 to 14, comprising an antigen binding fragment of an antibody, wherein said antigen binding fragment comprises CDR1, CDR2 and CDR3, optionally selected from the CDR1, CDR2 and CDR3 sequences having the respective amino acid sequences of SEQ ID Nos. 1, 2, 3, or 5, 6, 7, or 9, 10, 11, or 13, 14, 15, or. 17, 18, 19, or 21, 22, 23, or 25, 26, 27, or. 29, 30, 31, or 33, 34, 35, or 37, 38, 39; in each case independently, optionally with not more than three or two, preferably one, amino acid substitution(s), insertion(s) or deletion(s) compared to these sequences.
**Item 16:** The isolated ABP of any of items 10 to 15, wherein said CDR1 has an amino acid sequence of SEQ ID No 1, 5, 9, 13, 17, 21, 25, 29, 33, or 37, and CDR2 has an amino acid sequence of SEQ ID No 2, 6, 10, 14, 18, 22, 26, 30, 34, or 38, and CDR3 has an amino acid sequence of SEQ ID No 3, 7, 11, 15, 19, 23, 27, 31, 35, or 39; in each case independently, optionally with not more than three or two, preferably one, amino acid substitution(s), insertion(s) or deletion(s) compared to these sequences.
**Item 17**: The isolated ABP of any of items 1 to 16, wherein the ABP is an antibody, or an antigen binding fragment thereof, composed of at least one, preferably two, antibody heavy chain sequences, and at least one, preferably two, antibody light chain sequences, wherein at least one, preferably both, of said antibody heavy chain sequences and antibody light chain sequences comprise CDR1 to CDR3 sequences in the following combination:

| **Heavy Chain CDR1 to CDR3 (SEQ ID NO)** | | | **Light Chain CDR1 to CDR3 (SEQ ID NO)** | | |
|---|---|---|---|---|---|
| 1 | 2 | 3 | 5 | 6 | 7 |
| 9 | 10 | 11 | 13 | 14 | 15 |
| 17 | 18 | 19 | 21 | 22 | 23 |
| 25 | 26 | 27 | 29 | 30 | 31 |
| 33 | 34 | 35 | 37 | 38 | 39 |

in each case independently, optionally with not more than three or two, preferably one, amino acid substitution(s), insertion(s) or deletion(s) compared to these sequences.
**Item 18:** The isolated ABP of any of items 1 to 17, wherein the ABP is an antibody, or an antigen binding fragment thereof, composed of at least one, preferably two, antibody heavy chain sequence, and at least one, preferably two, antibody light chain sequence, wherein said antibody heavy chain sequence and the antibody light chain sequence comprises each a variable region sequences of the following combination:

| **Heavy Chain Variable Domain (SEQ ID NO)** | **Light Chain Variable Domain (SEQ ID NO)** |
|---|---|
| 4 | 8 |
| 12 | 16 |
| 20 | 24 |
| 28 | 32 |
| 36 | 40 |

in each case independently, optionally with not more than three or two, preferably one, amino acid substitution(s), insertion(s) or deletion(s) compared to these sequences.
**Item 19:** The isolated ABP according to any one of items 1 to 18, which comprises an effector group and/or which is labelled.
**Item 20:** The isolated ABP according to any one of items 1 to 19, which is isolated and/or substantially pure.
**Item 21**: The isolated ABP according to any one of items 1 to 20 which is an antibody, such as a monoclonal antibody; or which is a fragment of an antibody, such as a fragment of a monoclonal antibody.
**Item 22**: The isolated ABP according to item 21, wherein said antibody is a chimeric antibody, such as a human-chimeric antibody.
**Item 23**: The isolated ABP according to item 21 or 22, wherein said antibody is an IgG, IgE, IgD, IgA, or IgM immunoglobulin; preferably an IgG immunoglobulin.
**Item 24**: The isolated ABP according to any one of items 21 to 23, which is an antibody fragment selected from the list consisting of: Fab, Fab'-SH, Fv, scFv and F(ab')2.
**Item 25:** The isolated ABP according to any one of items 1 to 24, wherein said ABP is modified or engineered to increase antibody-dependent cellular cytotoxicity (ADCC), preferably wherein said ABP is afucosylated.
**Item 26:** The isolated ABP according to any one of items 1 to 25, which comprises one or more additional antigen binding domain(s) that bind(s) to antigen(s) other than said CD276, or the variant thereof; such as antigen(s) present on a mammalian T-cell, and most preferably human CD3.
**Item 27:** The isolated ABP according to item 26, which is bispecific, and preferably which comprises two binding sites binding to CD276 and two binding sites binding to an antigen other than said CD276, such as antigen(s) present on a mammalian T-cell, and most preferably to human CD3.
**Item 28:** The isolated ABP according to item 27, wherein the two binding sites binding to an antigen other than said CD276 bind to human CD3, and preferably comprise an UCHT1 anti-CD3 scFv construct.
**Item 29**: The isolated ABP of any one of items 1 to 28, which further comprises a moiety which enhanced antibody dependent cell cytotoxicity (ADCC), preferably wherein the moiety is an immunocytokine (MIC) such as Interleukin-15 (IL-15) or modified IL-15.
**Item 30**: A bispecific antigen binding protein (ABP) which comprises a first antigen binding domain capable of binding to the CD276 antigen, or the variant thereof, and a second antigen binding domain capable of binding to the human cluster of differentiation 3 (CD3) antigen.
**Item 31:** The bispecific ABP according to items 30, which comprises not more and not less than two first antigen binding domains and two second antigen binding domains, optionally wherein the bispecific ABP is in the IgSc format.
**Item 32:** The bispecific ABP according to any one of items 30 or 31, wherein at least one amino acid residue of the CH2 domain that is able to mediate binding to Fc receptors in said antibody is lacking or mutated.
**Item 33:** The bispecific ABP according to any one of items 30 to 32, wherein the first antigen binding domain comprises the ABP, or antigen binding domain thereof, according to any one of items 1 to 29.
**Item 34:** The bispecific ABP of any one of items 30 to 34, having an activity to bind to a T-cell and to an CD276 expressing tumor cell, or a tumor cell adjacent cell expressing CD276, such as a tumor vascular cell, preferably wherein the antibody increases the recruitment of cytotoxic cells to a CD276 expressing tumor cell by binding to CD276 and CD3.
**Item 35:** The bispecific ABP according to any one of items 30 to 34, wherein the second antigen binding domain comprises the heavy chain variable region and a light chain variable region of UCHT1.
**Item 36:** An isolated nucleic acid comprising a sequence encoding for an ABP, or for an antigen binding fragment or a monomer, such as a heavy or light chain, of an ABP, of any one of items 1 to 29, or encoding for a bispecific ABP according to any one of items 30 to 35.
**Item 37:** A nucleic acid construct (NAC) comprising a nucleic acid of item 36 and one or more additional sequence features permitting the expression of the encoded ABP or bispecific ABP, or a component of said ABP or bispecific ABP (such as an antibody heavy chain or light chain) in a cell.
**Item 38:** A recombinant host cell comprising a nucleic acid of item 36 or a NAC according to item 37.
**Item 39:** A pharmaceutical composition comprising: (i) an ABP or bispecific ABP of any one of items 1 to 35, or (ii) a nucleic acid of item 36 or a NAC according to item 37, or (iii) a recombinant host cell according to item 38, and a pharmaceutically acceptable carrier, stabiliser and/or excipient.
**Item 40:** A component for use in medicine, wherein the component is selected from the list consisting of: an ABP or bispecific ABP of any one of items 1 to 35, an isolated nucleic acid of item 36 or a NAC according to item 37, a recombinant host cell according to item 38 and a pharmaceutical composition according to item 39.
**Item 41:** The component for use of item 40, wherein the component is for use in enhancing T cell-mediated killing of and/or inhibiting the proliferation of CD276 positive tumor or tumor associated cells, or tumor cells or tumor associated cells positive for the variant of CD276.
**Item 42:** The component for use according to item 40 to 41, wherein the component is for use in the diagnosis, prevention and/or treatment of a proliferative disease, wherein the proliferative disorder is associated with the expression CD276, and is preferably cancer, such as a cancer selected from (preferred CD276 positive cancers?).
**Item 43:** A method of enhancing a cell-mediated immune response to a human cell that expresses human CD276, comprising contacting said cell with an ABP of any one of items 1 to 29, or a bispecific ABP according to any one of items 30 to 35, or a nucleic acid encoding said ABP or bispecific ABP according to item 36, in the presence of an immune cell, such as a T-cell or natural killer (NK) cell, thereby enhancing a cell-mediated immune response, preferably cytotoxicity, against said human cell.
**Item 44:** A method for the prevention and/or treatment of a proliferative disorder in a subject, comprising the administration of a therapeutically effective amount of a component recited in item 40 to the subject; and wherein the proliferative disorder is characterized by an expression of CD276 in cells associated with the proliferative disorder.

### BRIEF DESCRIPTION OF THE FIGURES AND SEQUENCES

### The figures show:

**Figure 1****:** shows the antibodies and antibody constructs of the invention; (A) a summary of epitope mapping results with the newly generated B7-H3 antibodies. The scheme shows the crystal structure of B7-H3 antigen (Vigdorovich V et al., Structure 2013), arrows identify the binding sites of the various proprietary B7-H3 mAb. Note that the 7C4 antibody contained in CC-3 binds an epitope close to the cell membrane. The indicated antibodies no longer bound to CD276 mutants carrying the indicated amino acid exchanges. These changes were selected on the bases of differences between the CD276 sequence of non human primates and humans. (B) Cartoons of monospecific antibodies (left), immunocytokines (MIC proteins, middle) and bispecific IgGsc antibodies (right). Note that monospecific antibodies were used either with a wildtype human IgGi Fc part or a SDIE modified version thereof (as depicted).

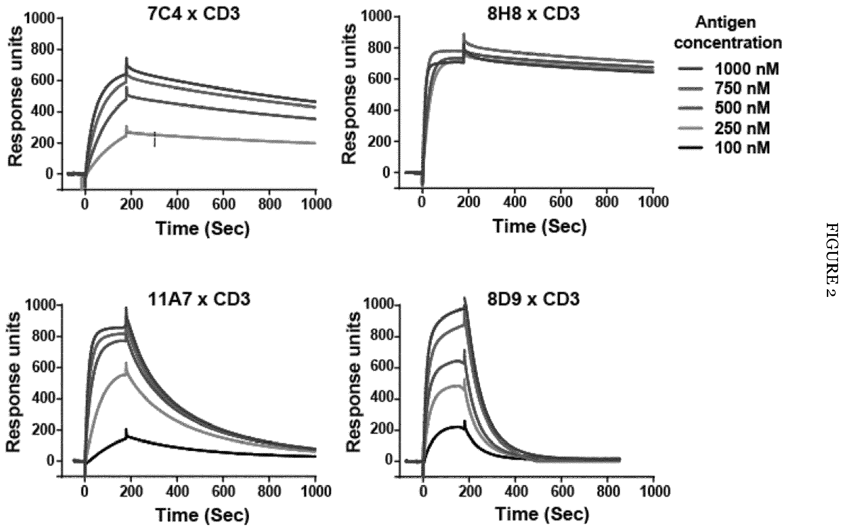

**Figure 2**: shows Biacore® sensorgrams determining the binding of B7-H3 antigen to the indicated B7-H3xCD3 bsAbs. Note that constructs containing 7C4 or 8H8 exhibit particularly long off-rates.

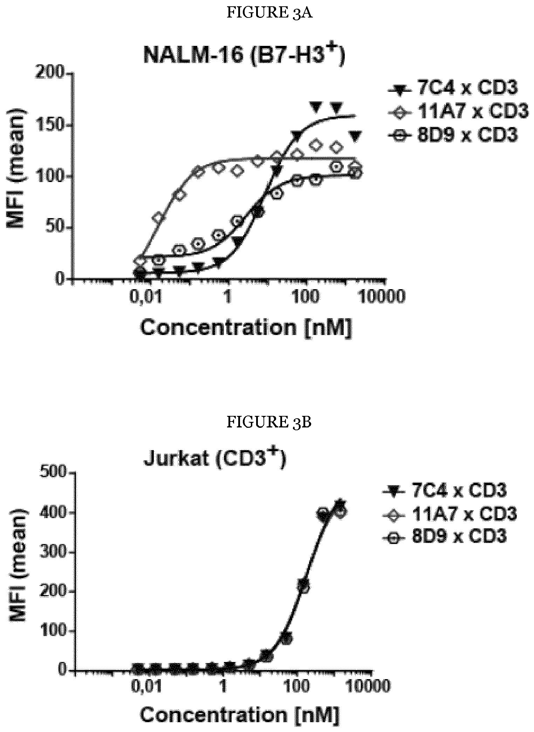

**Figure 3**: shows binding of different B7-H3xCD3 bsAbs to B7-H3 and CD3 expressed on NALM-16 (F) and Jurkat cells (G), respectively, as determined by flow cytometry. Note that in all constructs the anti CD3 antibody affinity was attenuated to reduce off target T cell activation, while binding to the target antigen B7-H3 is profoundly higher for all constructs.

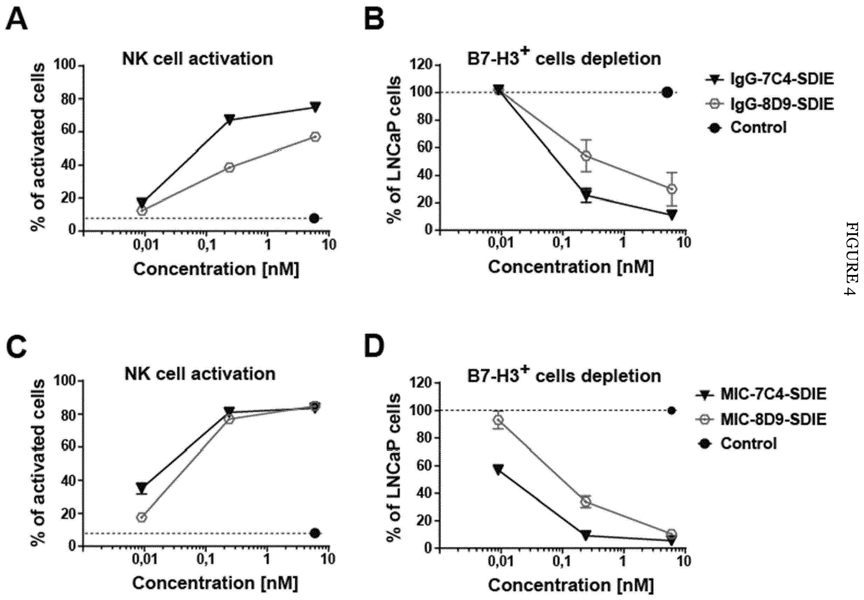

**Figure 4**: shows NK cell activation and tumor cell depletion by various monospecific CD276 antibodies (A,B) or MIC proteins containing SDIE-modified Fc-parts (C,D) Methods: Tumor cells (LNCap) were incubated with human PBMC and the indicated antibodies. After 3 days NK cell acitvation and depletion of tumor cells was determined by flow cytometry.

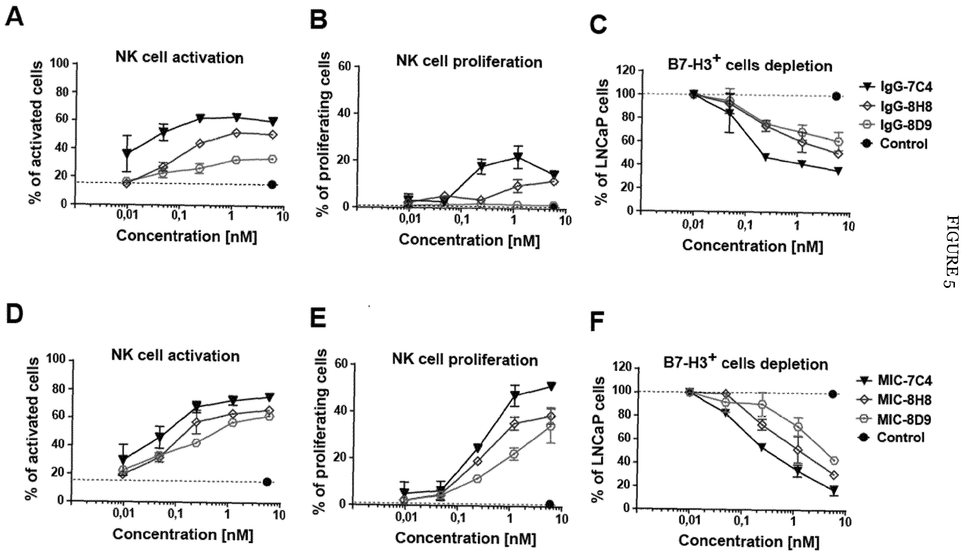

**Figure 5**: shows NK cell activation, -proliferation and tumor cell depletion by various CD276 antibodies (A-C) or MIC proteins (D-F) containing wildtype human IgGi Fc-parts Methods: Tumor cells (LNCap) were incubated with human PBMC and the indicated antibodies. After 3 days NK cell acitvation and depletion of tumor cells was determined by flow cytometry.

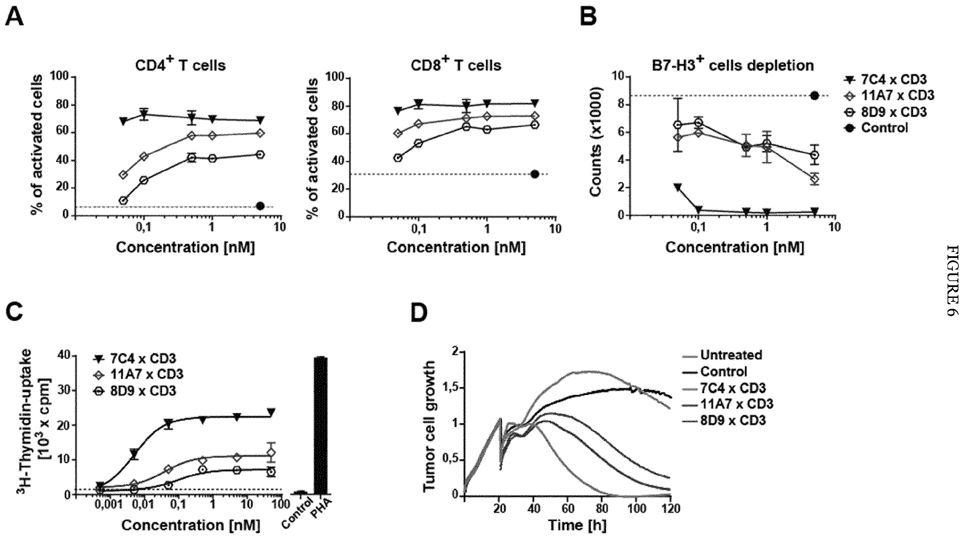

**Figure 6****:** shows T cell activation and tumor cell killing by various CD276 antibodies incorporated into bispecific antibodies in the IgGsc format (see Fig. 1B); Methods: B7-H3 expressing NALM-16 tumor cells were incubated with PBMC of healthy donors in the presence of increasing concentrations of the indicated B7-H3xCD3 constructs or a control bsAb with an unrelated specificity and activation of CD4 and CD8 T cells as well as tumor cells killing was determined by flow cytometry after 3 days (B). Induction of T cell proliferation (C) was assessed using a 3H-thymidine incorporation assay. Note that CC-3 (7C4xCD3) in all assays mediates markedly superior T cell stimulation compared to the other B7-H3xCD3 bsAbs. Long time xCELLigence tumor lysis assay (D) was performed with B7-H3 expressing LNCaP tumor cells and PBMC of healthy donors in the presence of the indicated B7-H3xCD3 bsAb. Note that CC-3 (7C4xCD3) mediates superior T cell activation and tumor cell killing compared to the other B7-H3xCD3 bsAbs

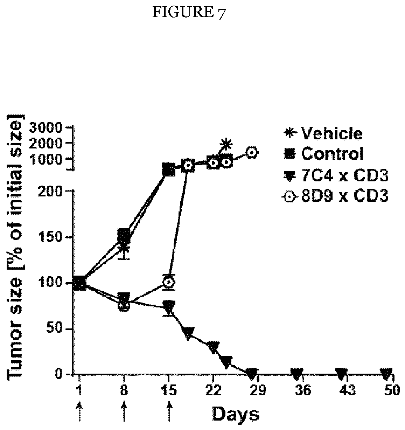

**Figure 7**: shows in *vivo* antitumor activity of different CD276 antibodies incorporated into bispecific IgGsc antibodies (Fig. 1B) Methods: LNCaP cells were injected s.c. into the right flank of female NSG mice to establish tumors with a diameter of 5mm. Then (day 1) PBMC with or without B7-H3xCD3 antibodies (2µg per dose) or an irrelevant controls bsAb were applied i.v. at days 1, 8 and 15 (indicated with arrows). Tumor growth was measured twice weekly, and mice were euthanized when tumors had reached a diameter of 15mm. Note that in this in vivo setting, CC-3 (7C4xCD3) is again markedly more effective than 8D9xCD3 confirming the in vitro data presented.

The sequences show:

**Table 1: Antibody Sequences of the Invention:**

| **SEQ ID NO:** | **Antibody Name:** | **Sequence-Domain:** | **Amino Acid Sequence:** |
|---|---|---|---|
| 1 | 7C4 | VH-CDR1 | EYTMH |
| 2 | 7C4 | VH-CDR2 | GINPNNGGTTYNQIFKN |
| 3 | 7C4 | VH-CDR3 | RGYHVSSWYFDV |
| 4 | 7C4 | VH-Variable Full Length | EVQLEQSGPELVKPGTSVKISCKTSGYTFTEY TMHWVKQSHGKSLEWIGGINPNNGGTTYN QIFKNKATLTVDKSSSTAYMELRSLTSEDSA VYYCARRGYHVSSWYFDVWGAGTTVTVSS |
| 5 | 7C4 | VL-CDR1 | SASSSVSYMH |
| 6 | 7C4 | VL-CDR2 | DTSKLAS |
| 7 | 7C4 | VL-CDR3 | LQWHSNPLT |
| 8 | 7C4 | VL-Variable Full Length | DIVLTQSTAIMSASPGEKVTMTCSASSSVSY MHWYQQKSGTSPKRWIYDTSKLASGVPARF SGSGSGTSYSLTISSMETEDSATYYCLQWHS NPLTFGAGTKLELKR |
| 9 | 11A7 | VH-CDR1 | NYWMN |
| 10 | 11A7 | VH-CDR2 | EIRLKSNNYATHYAESVKG |
| 11 | 11A7 | VH-CDR3 | HAD |
| 12 | 11A7 | VH-Variable Full Length | EVQLEESGGGLVQPGGSMKLSCVVSGFTFR NYWMNWVRQSPEKGLEWVAEIRLKSNNYA THYAESVKGRFTISRDDSKSSVYLQMNNLR AEDTGIYYCNSHADWGQGTLVSVSA |
| 13 | 11A7 | VL-CDR1 | RASENIYYTLA |
| 14 | 11A7 | VL-CDR2 | NANSLED |
| 15 | 11A7 | VL-CDR3 | KQGYDVPYT |
| 16 | 11A7 | VL-Variable Full Length | DIVLTQTTASLAASVGETVTITCRASENIYYT LAWYQQKQGKSPQLLIYNANSLEDGVPSRF SGSGSGTQYSMKINSMQPEDTATYFCKQGY DVPYTFGGGTKLEIKR |
| 17 | 8D9 | VH-CDR1 | SYAMS |
| 18 | 8D9 | VH-CDR2 | TISTGGSYTYYADSVKG |
| 19 | 8D9 | VH-CDR3 | HLYLYFDV |
| 20 | 8D9 | VH -Variable Full Length | EVKLEQSGGGLVKPGGSLKLSCAASGFTFSS YAMSWVRQKPEKRLEWVATISTGGSYTYYA DSVKGRFTISRDDARNTLNLQMSSLRSEDT AMYYCARHLYLYFDVWGAGTTVTVSS |
| 21 | 8D9 | VL-CDR1 | RASENIYSYLA |
| 22 | 8D9 | VL-CDR2 | NAKTLAE |
| 23 | 8D9 | VL-CDR3 | QHHYGTPPYT |
| 24 | 8D9 | VL-Variable Full Length | DIVMTQSTASLSASVGETVTITCRASENIYSY LAWYQQKQGKSPQLLVYNAKTLAEGVPSRF SGSGSGTQFSLKINSLQPEDFGSYYCQHHYG TPPYTFGGGTKLEIKR |
| 25 | 10A7 | VH-CDR1 | SYYIH |
| 26 | 10A7 | VH-CDR2 | WIYPGNVNTNYNERFKG |
| 27 | 10A7 | VH-CDR3 | GTYFFAY |
| 28 | 10A7 | VH-Variable Full Length | EVQLQQSGPELVKPGASVRISCKASGYTFTS YYIHWVKQRPGQGLEWIGWIYPGNVNTNY NERFKGKATLTADKSSSTAYMQLSSLTSEDS AVYFCAGGTYFFAYWGQGTLVTVSA |
| 29 | 10A7 | VL-CDR1 | RASKSISKYLA |
| 30 | 10A7 | VL-CDR2 | SGSTLQS |
| 31 | 10A7 | VL-CDR3 | QQHNEYPLT |
| 32 | 10A7 | VL-Variable Full Length | DIVLTQSPSHLAASPGETITINCRASKSISKYL AWYQEKPGKTNKLLIYSGSTLQSGVPSRFSG SGSNTDFTLTISSLEPEDFAMYYCQQHNEYP LTFGAGTELELKR |
| 33 | 8H8 | VH-CDR1 | DFDIN |
| 34 | 8H8 | VH-CDR2 | WIFPGDGSTKYDETFKD |
| 35 | 8H8 | VH-CDR3 | PRYGGSWFAY |
| 36 | 8H8 | VH-Variable Full Length | EVQLEQSGPDLVKPGASVKLSCKASGYTFTD FDINWVRQRPEQGLEWIGWIFPGDGSTKYD ETFKDKATLTTDKSSSTAYMQLSRLTSEDSA VYFCARPRYGGSWFAYWGQGTLVSVSA |
| 37 | 8H8 | VL-CDR1 | KSSQSLLYNTIQRSYLA |
| 38 | 8H8 | VL-CDR2 | WASTRES |
| 39 | 8H8 | VL-CDR3 | QQNYQYPWT |
| 40 | 8H8 | VL-Variable Full Length | DIVLTQSPSSLTVSVGEKVTMSCKSSQSLLYN TIQRSYLAWYQQKPGQSPKLLIYWASTRESG VPDRFTGSGSGTDFTLTISSVKAEDLAVYFC QQNYQYPWTFGGGTKLEIKR |

| | | | |
|---|---|---|---|
| *Abbreviations: VH: "variable heavy chain", VL: "variable light chain"; CDR: "Complementary Determining Region"* | | | |

**SEQ ID NO: 41** shows the amino acid sequence of human CD276 isoform 1: 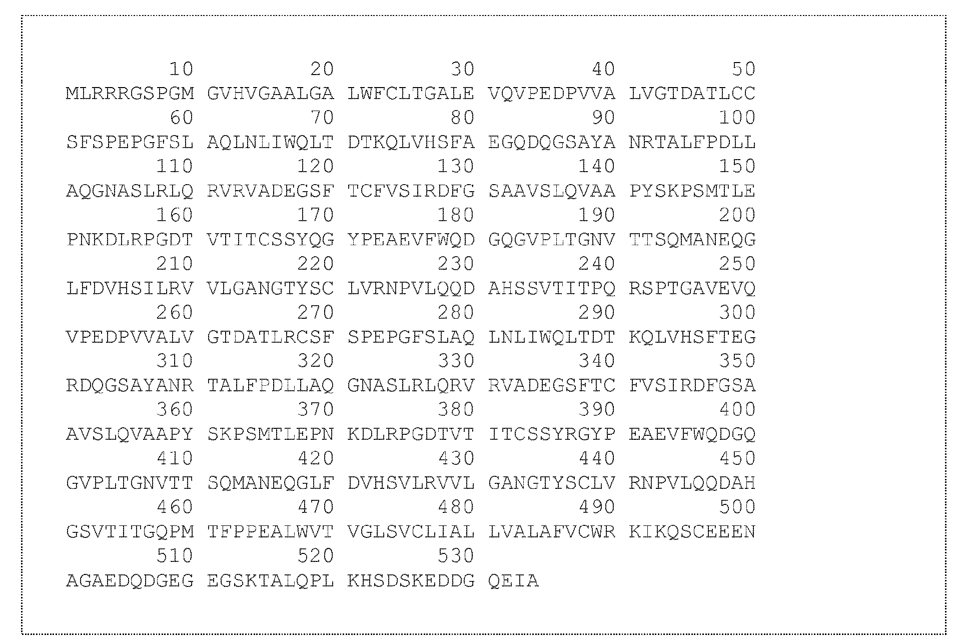
**SEQ ID NO: 42** shows the amino acid sequence of human CD276 isoform 2: 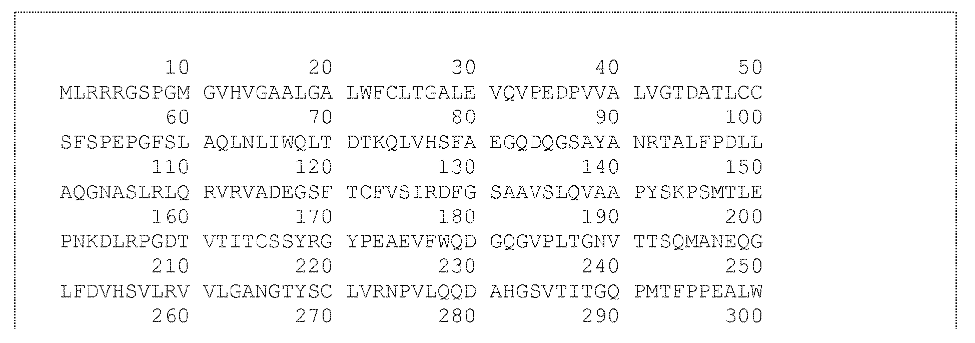 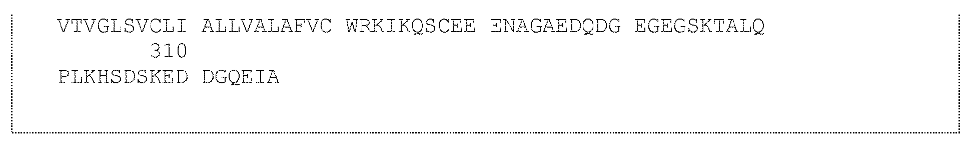
**SEQ ID NO: 43** shows the amino acid sequence of human CD276 isoform 3: 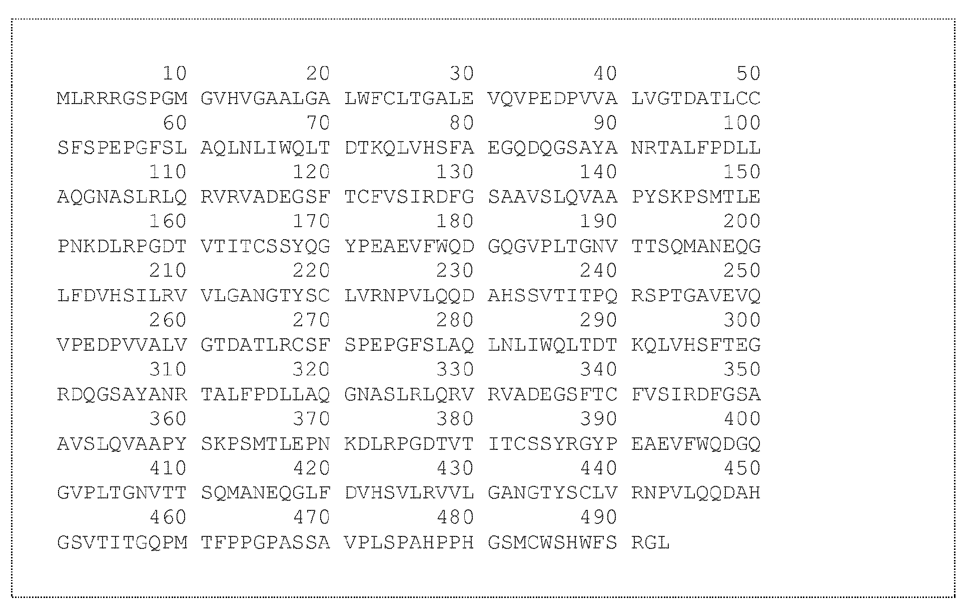
**SEQ ID NO: 44** shows the amino acid sequence of human CD276 isoform 4:

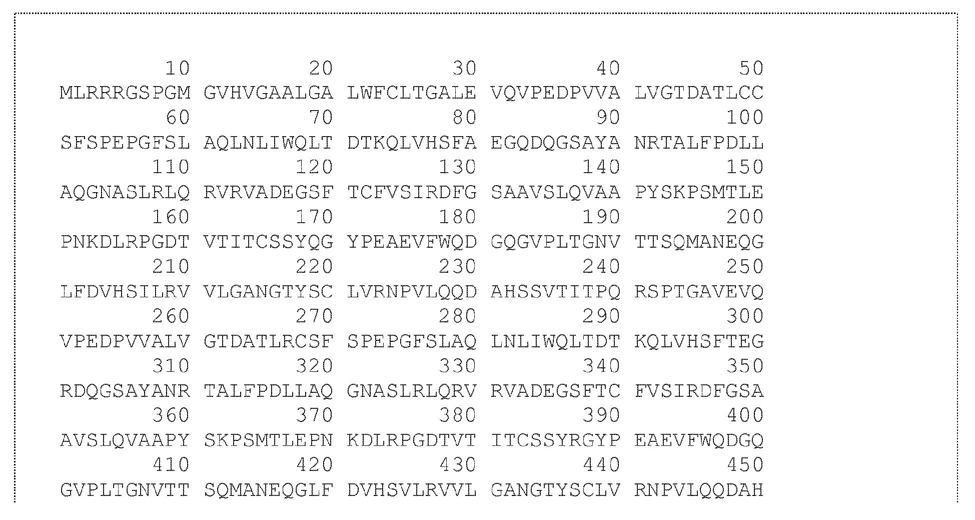

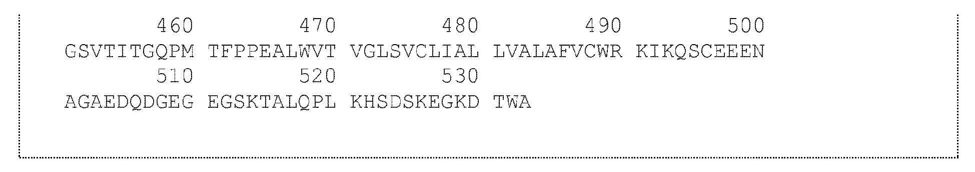

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

In course of the present invention a series of seven antibodies was generated after immunization of mice with recombinant CD276 protein using a hybridization and screening procedure. The binding epitope of the antibodies was then characterized (see Fig. 1A) and representative antibodies for each epitope were selected for construction of the following derivatives using recombinant antibody technology (see Fig.1B):
- monospecific antibodies containing wildtype or SDIE-modifiedi human IgGi Fc-parts)
- immunocytokines containing a modified IL-15 part and wild type or SDIE-modified human IgGi Fc-parts (MIC proteins)
- bispecific antibodies with CD276xCD3 specificity in the previously developed IgGsc-format

The antitumor activity of the different constructs was then tested *in vitro* and *in vivo,* and CD276 binders with optimal activity in the various formats were identified according to the following examples.

### The examples show:

### Example 1: Generation of Antibodies

Balb/c mice were immunized with recombinant CD276 protein and spleen cells were fused according to standard protocols. Female, 6 month old BALB/C mice were immunized using soluble CD276-Fc fusion protein. The protein used, consists out of the extracellular domain of human CD276 Isoform 2 (NM_001024736.1) with Met1-Thr461, fused to the N-terminus of the Fc region of human IgGi. 50 µg of the Fc fusion protein was repeatedly applied intraperitoneally in a volume of 100 µl PBS for each immunization. The mice were immunized every 10 days for a total of 3 to 4 immunizations with a final intravenous application 4 days prior of the fusion. Supernatants of fused cells were screened for production of specific antibodies by evaluating binding to CD276-transfected mouse cells by flow cytometry. A total of 7 hybridomas were then recloned and grown in advanced DMEM medium supplemented with 1.25% FCS. Antibodies were purified from culture supernatant by protein affinity chromatography and size exclusion chromatography (SEC) on Superdex S200. Purity was evaluated by by SDS page and analytical SEC. Sequences of the generated antibodies are provided in table 1.

### Example 2: Epitope Mapping of the New CD276-Antibidies

The human CD276 molecule exists as two isoforms, namely 2IgB7-H3 and 4IgB7-H3, with the latter one being the result of an exon duplication and that constitutes the predominant form in humans. The extracellular structure of the protein is characterized by IgV-IgC-like (2IgB7-H3) or IgV1-IgC1-IgV2-IgC2-like (4IgB7-H3) domains. Mice express only the 2IgB7-H3 isoform. For the epitope mapping, we first generated truncated versions of the 4IgB7-H3 molecule, IgV1-IgC1 and IgV2-IgC2, as Fc fusion proteins. We noted that all antibodies recognized both truncated versions. In addition, none of the antibodies were cross-reactive with the murine protein. Therefore, a sequence alignment between the highly conserved human and murine protein was performed. Finally, we replaced the variable amino acids in the human IgV1-IgC1-Fc protein with the respective amino acids of the murine molecule. This resulted in the epitope map and grouping of the antibodies as shown in Fig.1A. In addition, FACS based competition assays were performed, confirming that antibodies within the same group crossblock each other whereas antibodies of different groups don not.

### Example 3: Generation and Characterization of Recombinant Antibody Derivatives

The variable regions of representative antibodies of each group were sequenced, incorporated into different antibody formats and the antitumor activity of the antibodies was tested in suitable functional assays:
(1) SDIE optimized monospecific antibodies: ADCC Graph with 3 SDIE antibodies. Results are provided for the Fc enhanced version of antibodies 7C4 and 8D9 in figure 4. The antibody 7C4 outperforms the other, with significantly higher NK cell activation, and tumor cell depletion.
(2) Immunocytokine containing a modified IL-15 moiety: shown is NK cell activation and tumor cell killing of antibodies MIC 7C4, 8H8 and 8D9 in figures 4 and 5. As can be seen all antibodies perform excellent with the 7C4 being the most active antibody.
(3) bispecific antibodies with CD265xCD3-specificity Biacore, FACS binding and tumor cell killing: results are shown in figures 2, 3, and 6 to 7.

### REFERENCES

The references are:
1) Weiner GJ. Building better monoclonal antibody-based therapeutics. Nat Rev Cancer 2015;15:361-70.
2) Osenga KL, Hank JA, Albertini MR, Gan J, Sternberg AG, Eickhoff J, Seeger RC, Matthay KK, Reynolds CP, Twist C, Krailo M, Adamson PC, Reisfeld RA, Gillies SD, Sondel PM; Children's Oncology Group. A phase I clinical trial of the hu14.18-IL2 (EMD 273063) as a treatment for children with refractory or recurrent neuroblastoma and melanoma: a study of the Children's Oncology Group. Clin Cancer Res. 2006 Mar 15;12(6):1750-9.
3) Jung G, Salih HR, Lindner C, Lochmann B. Fusion proteins comprising a binding protein and an interleukin-15 polypeptide having a reduced affinity for IL15Ra and therapeutic uses thereof. EP15157911 (2015)
4) Nelson MH, Paulos CM. Novel immunotherapies for hematologic malignancies. Immunol Rev 2015;263:90-105.
5) Ribas A, Wolchok JD. Cancer immunotherapy using checkpoint blockade. Science 359, 1350-1355 (2018).
6) Gross G, Eshhar Z. Therapeutic Potential of T Cell Chimeric Antigen Receptors (CARs) in Cancer Treatment: Counteracting Off-Tumor Toxicities for Safe CAR T Cell Therapy. Annu Rev Pharmacol Toxicol 56, 59-83 (2016).
7) Porter DL, Levine BL, Kalos M, Bagg A, June CH. Chimeric antigen receptor-modified T cells in chronic lymphoid leukemia. N Engl J Med 365, 725-733 (2011).
8) Topp, M.S., et al. Targeted therapy with the T-cell-engaging antibody blinatumomab of chemotherapy-refractory minimal residual disease in B-lineage acute lymphoblastic leukemia patients results in high response rate and prolonged leukemia-free survival. J Clin Oncol 29, 2493-2498 (2011).
9) Portell, C.A., Wenzell, C.M. & Advani, A.S. Clinical and pharmacologic aspects of blinatumomab in the treatment of B-cell acute lymphoblastic leukemia. Clin Pharmacol 5, 5-11 (2013).
10) Riethmuller, G. Symmetry breaking: bispecific antibodies, the beginnings, and 50 years on. Cancer Immun 12,12 (2012).
11) Pishvaian M, Morse MA, McDevitt J, Norton JD, Ren S, Robbie GJ, Ryan PC, Soukharev S, Bao H, Denlinger CS. Phase 1 Dose Escalation Study of MEDI-565, a Bispecific T-Cell Engager that Targets Human Carcinoembryonic Antigen, in Patients With Advanced Gastrointestinal Adenocarcinomas. Clin Colorectal Cancer. 15:345-351, 2016.
12) Kebenko M, Goebeler ME, Wolf M, Hasenburg A, Seggewiss-Bernhardt R, Ritter B, Rautenberg B, Atanackovic D, Kratzer A, Rottman JB, Friedrich M, Vieser E, Elm S, Patzak I, Wessiepe D, Stienen S, Fiedler W. A multicenter phase 1 study of solitomab (MT110, AMG 110), a bispecific EpCAM/CD3 T-cell engager (BiTE®) antibody construct, in patients with refractory solid tumors. Oncoimmunology. 7: e1450710, 2018.
13) Ganss, R., Ryschich, E., Klar, E., Arnold, B. & Hammerling, G.J. Combination of T-cell therapy and trigger of inflammation induces remodeling of the vasculature and tumor eradication. Cancer Res 62, 1462-1470 (2002). 14) Sondel, P.M., Sosman, J.A., Hank, J.A., Kohler, P.C. & Storer, B. Tumor-infiltrating lymphocytes and interleukin-2 in melanomas. N Engl J Med 320, 1418-1419 (1989).

## Claims

1. **An isolated antigen binding protein (ABP)** which specifically binds to a CD276 protein, or a variant thereof, and wherein the isolated ABP is able to induce an antibody dependent cell-mediated cytotoxicity (ADCC) against a cell expressing the CD276 protein; wherein the ABP comprises at least one, preferably two, Complementary Determining Region (CDR) 3 having an amino acid sequence with at least 80% sequence identity to, or having no more than three or two, preferably one amino acid substitution(s), deletion(s) or insertion(s) relative to, a sequence selected from SEQ ID NOs. 3, 7, 11, 15, 19, 23, 27, 31, 35, and 39.

2. The isolated ABP of claim 1, wherein said ABP further comprises at least one, preferably two, CDR1, and at least one, preferably two, CDR2.

3. The isolated ABP of claim 1 or 2, comprising an antibody heavy chain sequence and/or an antibody light chain sequence, or an antigen binding fragment thereof; wherein the antibody heavy chain sequence, or the fragment thereof, comprises a CDR3 having at least 80% sequence identity to, or having no more than three or two, preferably one amino acid substitution(s), deletion(s) or insertion(s) relative to, a sequence selected from SEQ ID NOs. 3, 11, 19, 27, and 35, and/or wherein antibody light chain sequence, or the fragment thereof, comprises a CDR3 having at least 80% sequence identity to, or having no more than three or two, preferably one amino acid substitution(s), deletion(s) or insertion(s) relative to, a sequence selected from SEQ ID NOs. 7, 15, 23, 31, and 39.

4. The isolated ABP of any one of claims 1 to 3, comprising an antigen binding fragment of an antibody, wherein said antigen binding fragment comprises CDR1, CDR2 and CDR3, optionally selected from the CDR1, CDR2 and CDR3 sequences having the respective amino acid sequences of SEQ ID Nos. 1, 2, 3, or 5, 6, 7, or 9, 10, 11, or 13, 14, 15, or. 17, 18, 19, or 21, 22, 23, or 25, 26, 27, or. 29, 30, 31, or 33, 34, 35, or 37, 38, 39; in each case independently, optionally with not more than three or two, preferably one, amino acid substitution(s), insertion(s) or deletion(s) compared to these sequences.

5. The isolated ABP of any of claims 1 to 4, wherein the ABP is an antibody, or an antigen binding fragment thereof, composed of at least one, preferably two, antibody heavy chain sequences, and at least one, preferably two, antibody light chain sequences; and the ABP has at least one antigen binding domain which:
**(A)** comprises an antibody heavy chain CDR1 sequence shown in SEQ ID NO: 1, an antibody heavy chain CDR2 sequence shown in SEQ ID NO: 2, and an antibody heavy chain CDR3 sequence shown in SEQ ID NO: 3; and an antibody light chain CDR1 sequence shown in SEQ ID NO: 5, an antibody light chain CDR2 sequence shown in SEQ ID NO: 6, and an antibody light chain CDR3 sequence shown in SEQ ID NO: 7; in each case independently, optionally with no more than three or two, preferably no more than one, amino acid substitution(s), insertion(s) or deletion(s) compared to these sequences; or
**(B)** comprises an antibody heavy chain CDR1 sequence shown in SEQ ID NO: 9, an antibody heavy chain CDR2 sequence shown in SEQ ID NO: 10, and an antibody heavy chain CDR3 sequence shown in SEQ ID NO: 11; and an antibody light chain CDR1 sequence shown in SEQ ID NO: 13, an antibody light chain CDR2 sequence shown in SEQ ID NO: 14, and an antibody light chain CDR3 sequence shown in SEQ ID NO: 15; in each case independently, optionally with no more than three or two, preferably no more than one, amino acid substitution(s), insertion(s) or deletion(s) compared to these sequences; or
**(C)** comprises an antibody heavy chain CDR1 sequence shown in SEQ ID NO: 17, an antibody heavy chain CDR2 sequence shown in SEQ ID NO: 18, and an antibody heavy chain CDR3 sequence shown in SEQ ID NO: 19; and an antibody light chain CDR1 sequence shown in SEQ ID NO: 21, an antibody light chain CDR2 sequence shown in SEQ ID NO: 22, and an antibody light chain CDR3 sequence shown in SEQ ID NO: 23; in each case independently, optionally with no more than three or two, preferably no more than one, amino acid substitution(s), insertion(s) or deletion(s) compared to these sequences; or
**(D)** comprises an antibody heavy chain CDR1 sequence shown in SEQ ID NO: 25, an antibody heavy chain CDR2 sequence shown in SEQ ID NO: 26, and an antibody heavy chain CDR3 sequence shown in SEQ ID NO: 27; and an antibody light chain CDR1 sequence shown in SEQ ID NO: 29, an antibody light chain CDR2 sequence shown in SEQ ID NO: 30, and an antibody light chain CDR3 sequence shown in SEQ ID NO: 31; in each case independently, optionally with no more than three or two, preferably no more than one, amino acid substitution(s), insertion(s) or deletion(s) compared to these sequences; or
**(E)** comprises an antibody heavy chain CDR1 sequence shown in SEQ ID NO: 33, an antibody heavy chain CDR2 sequence shown in SEQ ID NO: 34, and an antibody heavy chain CDR3 sequence shown in SEQ ID NO: 35; and an antibody light chain CDR1 sequence shown in SEQ ID NO: 36, an antibody light chain CDR2 sequence shown in SEQ ID NO: 37, and an antibody light chain CDR3 sequence shown in SEQ ID NO: 38; in each case independently, optionally with no more than three or two, preferably no more than one, amino acid substitution(s), insertion(s) or deletion(s) compared to these sequences.

6. The isolated ABP according to any one of claims 1 to 5, wherein said ABP is modified or engineered to increase antibody-dependent cellular cytotoxicity (ADCC), preferably wherein said ABP comprises the SDIE mutations and/or is afucosylated.

7. The isolated ABP according to any one of claims 1 to 6, which comprises one or more additional antigen binding domain(s) that bind(s) to antigen(s) other than said CD276, or the variant thereof; such as antigen(s) present on a mammalian T-cell, and most preferably human CD3.

8. The isolated ABP of any one of claims 1 to 7, which further comprises a moiety which enhanced antibody dependent cell cytotoxicity (ADCC), preferably wherein the moiety is an immunocytokine (MIC) such as Interleukin-15 (IL-15) or modified IL-15.

9. **A bispecific antigen binding protein (ABP)** which comprises a first antigen binding domain capable of binding to the CD276 antigen, or the variant thereof, and a second antigen binding domain capable of binding to an antigen expressed on an immune cell, preferably CD3; wherein the first antigen binding domain is an antigen binding domain of the ABP according to any one of claims 1 to 8.

10. The bispecific ABP of claim 9, having an activity to bind to a T-cell expressing CD3 and to a CD276 expressing tumor cell, or a tumor cell adjacent cell expressing CD276, such as a tumor vascular cell, preferably wherein the bispecific ABP increases the recruitment of cytotoxic cells to a CD276 expressing cell by binding to CD276 and CD3.

11. **An isolated nucleic acid** comprising a sequence encoding for an ABP, or for an antigen binding fragment or a monomer, such as a heavy or light chain, of an ABP, of any one of claims 1 to 8, or encoding for a bispecific ABP according to claim 9 or 10.

12. **A nucleic acid construct (NAC)** comprising a nucleic acid of claim 11 and one or more additional sequence features permitting the expression of the encoded ABP or bispecific ABP, or a component of said ABP or bispecific ABP (such as an antibody heavy chain or light chain) in a cell.

13. **A recombinant host cell,** comprising a nucleic acid of claim 11 or a NAC according to claim 12.

14. **A pharmaceutical composition** comprising: (i) an ABP or bispecific ABP of any one of claims 1 to 10, or (ii) a nucleic acid of claim 11 or a NAC according to claim 12, or (iii) a recombinant host cell according to claim 13, and a pharmaceutically acceptable carrier, stabiliser and/or excipient.

15. **A component for use in medicine,** wherein the component is selected from the list consisting of: an ABP or bispecific ABP of any one of claims 1 to 10, an isolated nucleic acid of claim 11 or a NAC according to claim 12, a recombinant host cell according to claim 13 and a pharmaceutical composition according to claim 14.

16. The component for use of claim 15, wherein the component is for use in enhancing T cell-mediated killing of and/or inhibiting the proliferation of CD276 positive tumor or tumor associated cells, or tumor cells or tumor associated cells positive for the variant of CD276.

17. The component for use of claim 15 or 16, wherein the use is a treatment or prevention of a proliferative disorder, preferably cancer, **characterized by** the expression of CD276.


SEQUENCE LISTING

<110>  Deutsches Krebsforschungszentrum Stiftung des öffentlichen
       Rechts
       Eberhard Karls Universität Tübingen

<120>  ANTIBODIES TARGETING, AND OTHER MODULATORS OF, THE CD276 ANTIGEN,
        AND USES THEREOF

<130>  D31708EP

<160>  44

<170>  PatentIn version 3.5

<210>  1
<211>  5
<212>  PRT
<213>  ARTIFICIAL

<220>
<223>  antibody sequence

<400>  1

Glu Tyr Thr Met His
1               5

<210>  2
<211>  17
<212>  PRT
<213>  ARTIFICIAL

<220>
<223>  antibody sequence

<400>  2

Gly Ile Asn Pro Asn Asn Gly Gly Thr Thr Tyr Asn Gln Ile Phe Lys
1               5                   10                  15

Asn

<210>  3
<211>  12
<212>  PRT
<213>  ARTIFICIAL

<220>
<223>  antibody sequence

<400>  3

Arg Gly Tyr His Val Ser Ser Trp Tyr Phe Asp Val
1               5                   10

<210>  4
<211>  121

<212>  PRT
<213>  ARTIFICIAL

<220>
<223>  antibody sequence

<400>  4

Glu Val Gln Leu Glu Gln Ser Gly Pro Glu Leu Val Lys Pro Gly Thr
1               5                   10                  15

Ser Val Lys Ile Ser Cys Lys Thr Ser Gly Tyr Thr Phe Thr Glu Tyr
            20                  25                  30

Thr Met His Trp Val Lys Gln Ser His Gly Lys Ser Leu Glu Trp Ile
        35                  40                  45

Gly Gly Ile Asn Pro Asn Asn Gly Gly Thr Thr Tyr Asn Gln Ile Phe
    50                  55                  60

Lys Asn Lys Ala Thr Leu Thr Val Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Ala Arg Arg Gly Tyr His Val Ser Ser Trp Tyr Phe Asp Val Trp Gly
            100                 105                 110

Ala Gly Thr Thr Val Thr Val Ser Ser
        115                 120


<210>  5
<211>  10
<212>  PRT
<213>  ARTIFICIAL

<220>
<223>  antibody sequence

<400>  5

Ser Ala Ser Ser Ser Val Ser Tyr Met His
1               5                   10


<210>  6
<211>  7
<212>  PRT
<213>  ARTIFICIAL

<220>
<223>  antibody sequence

<400>  6

Asp Thr Ser Lys Leu Ala Ser
1               5

<210>  7
<211>  9
<212>  PRT
<213>  ARTIFICIAL

<220>
<223>  antibody sequence

<400>  7

Leu Gln Trp His Ser Asn Pro Leu Thr
1               5

<210>  8
<211>  107
<212>  PRT
<213>  ARTIFICIAL

<220>
<223>  antibody sequence

<400>  8

Asp Ile Val Leu Thr Gln Ser Thr Ala Ile Met Ser Ala Ser Pro Gly
1               5                   10                  15

Glu Lys Val Thr Met Thr Cys Ser Ala Ser Ser Ser Val Ser Tyr Met
            20                  25                  30

His Trp Tyr Gln Gln Lys Ser Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Thr Glu
65                  70                  75                  80

Asp Ser Ala Thr Tyr Tyr Cys Leu Gln Trp His Ser Asn Pro Leu Thr
                85                  90                  95

Phe Gly Ala Gly Thr Lys Leu Glu Leu Lys Arg
            100                 105

<210>  9
<211>  5
<212>  PRT
<213>  ARTIFICIAL

<220>

<223>  antibody sequence

<400>  9

Asn Tyr Trp Met Asn
1               5

<210>  10
<211>  19
<212>  PRT
<213>  ARTIFICIAL

<220>
<223>  antibody sequence

<400>  10

Glu Ile Arg Leu Lys Ser Asn Asn Tyr Ala Thr His Tyr Ala Glu Ser
1               5                   10                  15

Val Lys Gly

<210>  11
<211>  3
<212>  PRT
<213>  ARTIFICIAL

<220>
<223>  antibody sequence

<400>  11

His Ala Asp
1

<210>  12
<211>  114
<212>  PRT
<213>  ARTIFICIAL

<220>
<223>  antibody sequence

<400>  12

Glu Val Gln Leu Glu Glu Ser Gly Gly Gly Leu Val Gln Pro Gly Gly
1               5                   10                  15

Ser Met Lys Leu Ser Cys Val Val Ser Gly Phe Thr Phe Arg Asn Tyr
            20                  25                  30

Trp Met Asn Trp Val Arg Gln Ser Pro Glu Lys Gly Leu Glu Trp Val
        35                  40                  45

Ala Glu Ile Arg Leu Lys Ser Asn Asn Tyr Ala Thr His Tyr Ala Glu

50                  55                  60

Ser Val Lys Gly Arg Phe Thr Ile Ser Arg Asp Asp Ser Lys Ser Ser
65                  70                  75                  80

Val Tyr Leu Gln Met Asn Asn Leu Arg Ala Glu Asp Thr Gly Ile Tyr
                85                  90                  95

Tyr Cys Asn Ser His Ala Asp Trp Gly Gln Gly Thr Leu Val Ser Val
            100                 105                 110

Ser Ala

<210>  13
<211>  11
<212>  PRT
<213>  ARTIFICIAL

<220>
<223>  antibody sequence

<400>  13

Arg Ala Ser Glu Asn Ile Tyr Tyr Thr Leu Ala
1               5                   10

<210>  14
<211>  7
<212>  PRT
<213>  ARTIFICIAL

<220>
<223>  antibody sequence

<400>  14

Asn Ala Asn Ser Leu Glu Asp
1               5

<210>  15
<211>  9
<212>  PRT
<213>  ARTIFICIAL

<220>
<223>  antibody sequence

<400>  15

Lys Gln Gly Tyr Asp Val Pro Tyr Thr
1               5

<210>  16
<211>  108

<212>  PRT
<213>  ARTIFICIAL

<220>
<223>  antibody sequence

<400>  16

Asp Ile Val Leu Thr Gln Thr Thr Ala Ser Leu Ala Ala Ser Val Gly
1               5                   10                  15

Glu Thr Val Thr Ile Thr Cys Arg Ala Ser Glu Asn Ile Tyr Tyr Thr
            20                  25                  30

Leu Ala Trp Tyr Gln Gln Lys Gln Gly Lys Ser Pro Gln Leu Leu Ile
        35                  40                  45

Tyr Asn Ala Asn Ser Leu Glu Asp Gly Val Pro Ser Arg Phe Ser Gly
    50                  55                  60

Ser Gly Ser Gly Thr Gln Tyr Ser Met Lys Ile Asn Ser Met Gln Pro
65                  70                  75                  80

Glu Asp Thr Ala Thr Tyr Phe Cys Lys Gln Gly Tyr Asp Val Pro Tyr
                85                  90                  95

Thr Phe Gly Gly Gly Thr Lys Leu Glu Ile Lys Arg
            100                 105


<210>  17
<211>  5
<212>  PRT
<213>  ARTIFICIAL

<220>
<223>  antibody sequence

<400>  17

Ser Tyr Ala Met Ser
1               5


<210>  18
<211>  17
<212>  PRT
<213>  ARTIFICIAL

<220>
<223>  antibody sequence

<400>  18

Thr Ile Ser Thr Gly Gly Ser Tyr Thr Tyr Tyr Ala Asp Ser Val Lys
1               5                   10                  15

Gly

<210> 19
<211> 8
<212> PRT
<213> ARTIFICIAL

<220>
<223> antibody sequence

<400> 19

His Leu Tyr Leu Tyr Phe Asp Val
1               5

<210> 20
<211> 117
<212> PRT
<213> ARTIFICIAL

<220>
<223> antibody sequence

<400> 20

Glu Val Lys Leu Glu Gln Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Ala Met Ser Trp Val Arg Gln Lys Pro Glu Lys Arg Leu Glu Trp Val
        35                  40                  45

Ala Thr Ile Ser Thr Gly Gly Ser Tyr Thr Tyr Tyr Ala Asp Ser Val
    50                  55                  60

Lys Gly Arg Phe Thr Ile Ser Arg Asp Asp Ala Arg Asn Thr Leu Asn
65                  70                  75                  80

Leu Gln Met Ser Ser Leu Arg Ser Glu Asp Thr Ala Met Tyr Tyr Cys
                85                  90                  95

Ala Arg His Leu Tyr Leu Tyr Phe Asp Val Trp Gly Ala Gly Thr Thr
            100                 105                 110

Val Thr Val Ser Ser
        115

<210> 21
<211> 11

<212>  PRT
<213>  ARTIFICIAL

<220>
<223>  antibody sequence

<400>  21

Arg Ala Ser Glu Asn Ile Tyr Ser Tyr Leu Ala
1               5                   10


<210>  22
<211>  7
<212>  PRT
<213>  ARTIFICIAL

<220>
<223>  antibody sequence

<400>  22

Asn Ala Lys Thr Leu Ala Glu
1               5


<210>  23
<211>  10
<212>  PRT
<213>  ARTIFICIAL

<220>
<223>  antibody sequence

<400>  23

Gln His His Tyr Gly Thr Pro Pro Tyr Thr
1               5                   10


<210>  24
<211>  109
<212>  PRT
<213>  ARTIFICIAL

<220>
<223>  antibody sequence

<400>  24

Asp Ile Val Met Thr Gln Ser Thr Ala Ser Leu Ser Ala Ser Val Gly
1               5                   10                  15


Glu Thr Val Thr Ile Thr Cys Arg Ala Ser Glu Asn Ile Tyr Ser Tyr
            20                  25                  30


Leu Ala Trp Tyr Gln Gln Lys Gln Gly Lys Ser Pro Gln Leu Leu Val
        35                  40                  45


Tyr Asn Ala Lys Thr Leu Ala Glu Gly Val Pro Ser Arg Phe Ser Gly

50                  55                  60

Ser Gly Ser Gly Thr Gln Phe Ser Leu Lys Ile Asn Ser Leu Gln Pro
65                  70                  75                  80

Glu Asp Phe Gly Ser Tyr Tyr Cys Gln His His Tyr Gly Thr Pro Pro
                85                  90                  95

Tyr Thr Phe Gly Gly Gly Thr Lys Leu Glu Ile Lys Arg
            100                 105

<210>  25
<211>  5
<212>  PRT
<213>  ARTIFICIAL

<220>
<223>  antibody sequence

<400>  25

Ser Tyr Tyr Ile His
1               5

<210>  26
<211>  17
<212>  PRT
<213>  ARTIFICIAL

<220>
<223>  antibody sequence

<400>  26

Trp Ile Tyr Pro Gly Asn Val Asn Thr Asn Tyr Asn Glu Arg Phe Lys
1               5                   10                  15

Gly

<210>  27
<211>  7
<212>  PRT
<213>  ARTIFICIAL

<220>
<223>  antibody sequence

<400>  27

Gly Thr Tyr Phe Phe Ala Tyr
1               5

<210>  28
<211>  116

<212>  PRT
<213>  ARTIFICIAL

<220>
<223>  antibody sequence

<400>  28

Glu Val Gln Leu Gln Gln Ser Gly Pro Glu Leu Val Lys Pro Gly Ala
1               5                   10                  15

Ser Val Arg Ile Ser Cys Lys Ala Ser Gly Tyr Thr Phe Thr Ser Tyr
            20                  25                  30

Tyr Ile His Trp Val Lys Gln Arg Pro Gly Gln Gly Leu Glu Trp Ile
        35                  40                  45

Gly Trp Ile Tyr Pro Gly Asn Val Asn Thr Asn Tyr Asn Glu Arg Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Gly Gly Thr Tyr Phe Phe Ala Tyr Trp Gly Gln Gly Thr Leu Val
            100                 105                 110

Thr Val Ser Ala
        115


<210>  29
<211>  11
<212>  PRT
<213>  ARTIFICIAL

<220>
<223>  antibody sequence

<400>  29

Arg Ala Ser Lys Ser Ile Ser Lys Tyr Leu Ala
1               5                   10


<210>  30
<211>  7
<212>  PRT
<213>  ARTIFICIAL

<220>
<223>  antibody sequence

<400>  30

Ser Gly Ser Thr Leu Gln Ser
1               5

<210>  31
<211>  9
<212>  PRT
<213>  ARTIFICIAL

<220>
<223>  antibody sequence

<400>  31

Gln Gln His Asn Glu Tyr Pro Leu Thr
1               5

<210>  32
<211>  108
<212>  PRT
<213>  ARTIFICIAL

<220>
<223>  antibody sequence

<400>  32

Asp Ile Val Leu Thr Gln Ser Pro Ser His Leu Ala Ala Ser Pro Gly
1               5                   10                  15

Glu Thr Ile Thr Ile Asn Cys Arg Ala Ser Lys Ser Ile Ser Lys Tyr
            20                  25                  30

Leu Ala Trp Tyr Gln Glu Lys Pro Gly Lys Thr Asn Lys Leu Leu Ile
        35                  40                  45

Tyr Ser Gly Ser Thr Leu Gln Ser Gly Val Pro Ser Arg Phe Ser Gly
    50                  55                  60

Ser Gly Ser Asn Thr Asp Phe Thr Leu Thr Ile Ser Ser Leu Glu Pro
65                  70                  75                  80

Glu Asp Phe Ala Met Tyr Tyr Cys Gln Gln His Asn Glu Tyr Pro Leu
                85                  90                  95

Thr Phe Gly Ala Gly Thr Glu Leu Glu Leu Lys Arg
            100                 105

<210>  33
<211>  5
<212>  PRT
<213>  ARTIFICIAL

<220>

<223>  antibody sequence

<400>  33

Asp Phe Asp Ile Asn
1               5

<210>  34
<211>  17
<212>  PRT
<213>  ARTIFICIAL

<220>
<223>  antibody sequence

<400>  34

Trp Ile Phe Pro Gly Asp Gly Ser Thr Lys Tyr Asp Glu Thr Phe Lys
1               5                   10                  15

Asp

<210>  35
<211>  10
<212>  PRT
<213>  ARTIFICIAL

<220>
<223>  antibody sequence

<400>  35

Pro Arg Tyr Gly Gly Ser Trp Phe Ala Tyr
1               5                   10

<210>  36
<211>  119
<212>  PRT
<213>  ARTIFICIAL

<220>
<223>  antibody sequence

<400>  36

Glu Val Gln Leu Glu Gln Ser Gly Pro Asp Leu Val Lys Pro Gly Ala
1               5                   10                  15

Ser Val Lys Leu Ser Cys Lys Ala Ser Gly Tyr Thr Phe Thr Asp Phe
            20                  25                  30

Asp Ile Asn Trp Val Arg Gln Arg Pro Glu Gln Gly Leu Glu Trp Ile
        35                  40                  45

Gly Trp Ile Phe Pro Gly Asp Gly Ser Thr Lys Tyr Asp Glu Thr Phe

50                  55                  60

Lys Asp Lys Ala Thr Leu Thr Thr Asp Lys Ser Ser Ser Thr Ala Tyr
65                  70                  75                  80

Met Gln Leu Ser Arg Leu Thr Ser Glu Asp Ser Ala Val Tyr Phe Cys
                85                  90                  95

Ala Arg Pro Arg Tyr Gly Gly Ser Trp Phe Ala Tyr Trp Gly Gln Gly
            100                 105                 110

Thr Leu Val Ser Val Ser Ala
        115

<210>  37
<211>  17
<212>  PRT
<213>  ARTIFICIAL

<220>
<223>  antibody sequence

<400>  37

Lys Ser Ser Gln Ser Leu Leu Tyr Asn Thr Ile Gln Arg Ser Tyr Leu
1               5                   10                  15

Ala

<210>  38
<211>  7
<212>  PRT
<213>  ARTIFICIAL

<220>
<223>  antibody sequence

<400>  38

Trp Ala Ser Thr Arg Glu Ser
1               5

<210>  39
<211>  9
<212>  PRT
<213>  ARTIFICIAL

<220>
<223>  antibody sequence

<400>  39

Gln Gln Asn Tyr Gln Tyr Pro Trp Thr
1               5

<210>  40
<211>  114
<212>  PRT
<213>  ARTIFICIAL

<220>
<223>  antibody sequence

<400>  40

Asp Ile Val Leu Thr Gln Ser Pro Ser Ser Leu Thr Val Ser Val Gly
1               5                   10                  15

Glu Lys Val Thr Met Ser Cys Lys Ser Ser Gln Ser Leu Leu Tyr Asn
            20                  25                  30

Thr Ile Gln Arg Ser Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Phe Cys Gln Gln
                85                  90                  95

Asn Tyr Gln Tyr Pro Trp Thr Phe Gly Gly Gly Thr Lys Leu Glu Ile
            100                 105                 110

Lys Arg

<210>  41
<211>  534
<212>  PRT
<213>  Homo sapiens

<400>  41

Met Leu Arg Arg Arg Gly Ser Pro Gly Met Gly Val His Val Gly Ala
1               5                   10                  15

Ala Leu Gly Ala Leu Trp Phe Cys Leu Thr Gly Ala Leu Glu Val Gln
            20                  25                  30

Val Pro Glu Asp Pro Val Val Ala Leu Val Gly Thr Asp Ala Thr Leu
        35                  40                  45

Cys Cys Ser Phe Ser Pro Glu Pro Gly Phe Ser Leu Ala Gln Leu Asn
    50                  55                  60

Leu Ile Trp Gln Leu Thr Asp Thr Lys Gln Leu Val His Ser Phe Ala
65                  70                  75                  80

Glu Gly Gln Asp Gln Gly Ser Ala Tyr Ala Asn Arg Thr Ala Leu Phe
                85                  90                  95

Pro Asp Leu Leu Ala Gln Gly Asn Ala Ser Leu Arg Leu Gln Arg Val
            100                 105                 110

Arg Val Ala Asp Glu Gly Ser Phe Thr Cys Phe Val Ser Ile Arg Asp
        115                 120                 125

Phe Gly Ser Ala Ala Val Ser Leu Gln Val Ala Ala Pro Tyr Ser Lys
    130                 135                 140

Pro Ser Met Thr Leu Glu Pro Asn Lys Asp Leu Arg Pro Gly Asp Thr
145                 150                 155                 160

Val Thr Ile Thr Cys Ser Ser Tyr Gln Gly Tyr Pro Glu Ala Glu Val
                165                 170                 175

Phe Trp Gln Asp Gly Gln Gly Val Pro Leu Thr Gly Asn Val Thr Thr
            180                 185                 190

Ser Gln Met Ala Asn Glu Gln Gly Leu Phe Asp Val His Ser Ile Leu
        195                 200                 205

Arg Val Val Leu Gly Ala Asn Gly Thr Tyr Ser Cys Leu Val Arg Asn
    210                 215                 220

Pro Val Leu Gln Gln Asp Ala His Ser Ser Val Thr Ile Thr Pro Gln
225                 230                 235                 240

Arg Ser Pro Thr Gly Ala Val Glu Val Gln Val Pro Glu Asp Pro Val
                245                 250                 255

Val Ala Leu Val Gly Thr Asp Ala Thr Leu Arg Cys Ser Phe Ser Pro
            260                 265                 270

Glu Pro Gly Phe Ser Leu Ala Gln Leu Asn Leu Ile Trp Gln Leu Thr
        275                 280                 285

Asp Thr Lys Gln Leu Val His Ser Phe Thr Glu Gly Arg Asp Gln Gly
    290                 295                 300

Ser Ala Tyr Ala Asn Arg Thr Ala Leu Phe Pro Asp Leu Leu Ala Gln
305                 310                 315                 320

Gly Asn Ala Ser Leu Arg Leu Gln Arg Val Arg Val Ala Asp Glu Gly
                325                 330                 335

Ser Phe Thr Cys Phe Val Ser Ile Arg Asp Phe Gly Ser Ala Ala Val
            340                 345                 350

Ser Leu Gln Val Ala Ala Pro Tyr Ser Lys Pro Ser Met Thr Leu Glu
        355                 360                 365

Pro Asn Lys Asp Leu Arg Pro Gly Asp Thr Val Thr Ile Thr Cys Ser
    370                 375                 380

Ser Tyr Arg Gly Tyr Pro Glu Ala Glu Val Phe Trp Gln Asp Gly Gln
385                 390                 395                 400

Gly Val Pro Leu Thr Gly Asn Val Thr Thr Ser Gln Met Ala Asn Glu
                405                 410                 415

Gln Gly Leu Phe Asp Val His Ser Val Leu Arg Val Val Leu Gly Ala
            420                 425                 430

Asn Gly Thr Tyr Ser Cys Leu Val Arg Asn Pro Val Leu Gln Gln Asp
        435                 440                 445

Ala His Gly Ser Val Thr Ile Thr Gly Gln Pro Met Thr Phe Pro Pro
    450                 455                 460

Glu Ala Leu Trp Val Thr Val Gly Leu Ser Val Cys Leu Ile Ala Leu
465                 470                 475                 480

Leu Val Ala Leu Ala Phe Val Cys Trp Arg Lys Ile Lys Gln Ser Cys
                485                 490                 495

Glu Glu Glu Asn Ala Gly Ala Glu Asp Gln Asp Gly Glu Gly Glu Gly
            500                 505                 510

Ser Lys Thr Ala Leu Gln Pro Leu Lys His Ser Asp Ser Lys Glu Asp
        515                 520                 525

Asp Gly Gln Glu Ile Ala
    530

<210>  42
<211>  316

<212>  PRT
<213>  Homo sapiens

<400>  42

Met Leu Arg Arg Arg Gly Ser Pro Gly Met Gly Val His Val Gly Ala
1               5                   10                  15

Ala Leu Gly Ala Leu Trp Phe Cys Leu Thr Gly Ala Leu Glu Val Gln
            20                  25                  30

Val Pro Glu Asp Pro Val Val Ala Leu Val Gly Thr Asp Ala Thr Leu
        35                  40                  45

Cys Cys Ser Phe Ser Pro Glu Pro Gly Phe Ser Leu Ala Gln Leu Asn
    50                  55                  60

Leu Ile Trp Gln Leu Thr Asp Thr Lys Gln Leu Val His Ser Phe Ala
65                  70                  75                  80

Glu Gly Gln Asp Gln Gly Ser Ala Tyr Ala Asn Arg Thr Ala Leu Phe
                85                  90                  95

Pro Asp Leu Leu Ala Gln Gly Asn Ala Ser Leu Arg Leu Gln Arg Val
            100                 105                 110

Arg Val Ala Asp Glu Gly Ser Phe Thr Cys Phe Val Ser Ile Arg Asp
        115                 120                 125

Phe Gly Ser Ala Ala Val Ser Leu Gln Val Ala Ala Pro Tyr Ser Lys
    130                 135                 140

Pro Ser Met Thr Leu Glu Pro Asn Lys Asp Leu Arg Pro Gly Asp Thr
145                 150                 155                 160

Val Thr Ile Thr Cys Ser Ser Tyr Arg Gly Tyr Pro Glu Ala Glu Val
                165                 170                 175

Phe Trp Gln Asp Gly Gln Gly Val Pro Leu Thr Gly Asn Val Thr Thr
            180                 185                 190

Ser Gln Met Ala Asn Glu Gln Gly Leu Phe Asp Val His Ser Val Leu
        195                 200                 205

Arg Val Val Leu Gly Ala Asn Gly Thr Tyr Ser Cys Leu Val Arg Asn
    210                 215                 220

Pro Val Leu Gln Gln Asp Ala His Gly Ser Val Thr Ile Thr Gly Gln
225                 230                 235                 240

Pro Met Thr Phe Pro Pro Glu Ala Leu Trp Val Thr Val Gly Leu Ser
                245                 250                 255

Val Cys Leu Ile Ala Leu Leu Val Ala Leu Ala Phe Val Cys Trp Arg
            260                 265                 270

Lys Ile Lys Gln Ser Cys Glu Glu Glu Asn Ala Gly Ala Glu Asp Gln
        275                 280                 285

Asp Gly Glu Gly Glu Gly Ser Lys Thr Ala Leu Gln Pro Leu Lys His
    290                 295                 300

Ser Asp Ser Lys Glu Asp Asp Gly Gln Glu Ile Ala
305                 310                 315

<210>  43
<211>  493
<212>  PRT
<213>  Homo sapiens

<400>  43

Met Leu Arg Arg Arg Gly Ser Pro Gly Met Gly Val His Val Gly Ala
1               5                   10                  15

Ala Leu Gly Ala Leu Trp Phe Cys Leu Thr Gly Ala Leu Glu Val Gln
            20                  25                  30

Val Pro Glu Asp Pro Val Val Ala Leu Val Gly Thr Asp Ala Thr Leu
        35                  40                  45

Cys Cys Ser Phe Ser Pro Glu Pro Gly Phe Ser Leu Ala Gln Leu Asn
    50                  55                  60

Leu Ile Trp Gln Leu Thr Asp Thr Lys Gln Leu Val His Ser Phe Ala
65                  70                  75                  80

Glu Gly Gln Asp Gln Gly Ser Ala Tyr Ala Asn Arg Thr Ala Leu Phe
                85                  90                  95

Pro Asp Leu Leu Ala Gln Gly Asn Ala Ser Leu Arg Leu Gln Arg Val
            100                 105                 110

Arg Val Ala Asp Glu Gly Ser Phe Thr Cys Phe Val Ser Ile Arg Asp
        115                 120                 125

Phe Gly Ser Ala Ala Val Ser Leu Gln Val Ala Ala Pro Tyr Ser Lys
    130                 135                 140

Pro Ser Met Thr Leu Glu Pro Asn Lys Asp Leu Arg Pro Gly Asp Thr
145                 150                 155                 160

Val Thr Ile Thr Cys Ser Ser Tyr Gln Gly Tyr Pro Glu Ala Glu Val
                165                 170                 175

Phe Trp Gln Asp Gly Gln Gly Val Pro Leu Thr Gly Asn Val Thr Thr
            180                 185                 190

Ser Gln Met Ala Asn Glu Gln Gly Leu Phe Asp Val His Ser Ile Leu
        195                 200                 205

Arg Val Val Leu Gly Ala Asn Gly Thr Tyr Ser Cys Leu Val Arg Asn
    210                 215                 220

Pro Val Leu Gln Gln Asp Ala His Ser Ser Val Thr Ile Thr Pro Gln
225                 230                 235                 240

Arg Ser Pro Thr Gly Ala Val Glu Val Gln Val Pro Glu Asp Pro Val
                245                 250                 255

Val Ala Leu Val Gly Thr Asp Ala Thr Leu Arg Cys Ser Phe Ser Pro
            260                 265                 270

Glu Pro Gly Phe Ser Leu Ala Gln Leu Asn Leu Ile Trp Gln Leu Thr
        275                 280                 285

Asp Thr Lys Gln Leu Val His Ser Phe Thr Glu Gly Arg Asp Gln Gly
    290                 295                 300

Ser Ala Tyr Ala Asn Arg Thr Ala Leu Phe Pro Asp Leu Leu Ala Gln
305                 310                 315                 320

Gly Asn Ala Ser Leu Arg Leu Gln Arg Val Arg Val Ala Asp Glu Gly
                325                 330                 335

Ser Phe Thr Cys Phe Val Ser Ile Arg Asp Phe Gly Ser Ala Ala Val
            340                 345                 350

Ser Leu Gln Val Ala Ala Pro Tyr Ser Lys Pro Ser Met Thr Leu Glu
        355                 360                 365

Pro Asn Lys Asp Leu Arg Pro Gly Asp Thr Val Thr Ile Thr Cys Ser
    370                 375                 380

Ser Tyr Arg Gly Tyr Pro Glu Ala Glu Val Phe Trp Gln Asp Gly Gln

385                 390                 395                 400

Gly Val Pro Leu Thr Gly Asn Val Thr Thr Ser Gln Met Ala Asn Glu
                405                 410                 415

Gln Gly Leu Phe Asp Val His Ser Val Leu Arg Val Val Leu Gly Ala
            420                 425                 430

Asn Gly Thr Tyr Ser Cys Leu Val Arg Asn Pro Val Leu Gln Gln Asp
        435                 440                 445

Ala His Gly Ser Val Thr Ile Thr Gly Gln Pro Met Thr Phe Pro Pro
    450                 455                 460

Gly Pro Ala Ser Ser Ala Val Pro Leu Ser Pro Ala His Pro Pro His
465                 470                 475                 480

Gly Ser Met Cys Trp Ser His Trp Phe Ser Arg Gly Leu
                485                 490

<210>  44
<211>  533
<212>  PRT
<213>  Homo sapiens

<400>  44

Met Leu Arg Arg Arg Gly Ser Pro Gly Met Gly Val His Val Gly Ala
1               5                   10                  15

Ala Leu Gly Ala Leu Trp Phe Cys Leu Thr Gly Ala Leu Glu Val Gln
            20                  25                  30

Val Pro Glu Asp Pro Val Val Ala Leu Val Gly Thr Asp Ala Thr Leu
        35                  40                  45

Cys Cys Ser Phe Ser Pro Glu Pro Gly Phe Ser Leu Ala Gln Leu Asn
    50                  55                  60

Leu Ile Trp Gln Leu Thr Asp Thr Lys Gln Leu Val His Ser Phe Ala
65                  70                  75                  80

Glu Gly Gln Asp Gln Gly Ser Ala Tyr Ala Asn Arg Thr Ala Leu Phe
                85                  90                  95

Pro Asp Leu Leu Ala Gln Gly Asn Ala Ser Leu Arg Leu Gln Arg Val
            100                 105                 110

Arg Val Ala Asp Glu Gly Ser Phe Thr Cys Phe Val Ser Ile Arg Asp

115                 120                 125

Phe Gly Ser Ala Ala Val Ser Leu Gln Val Ala Ala Pro Tyr Ser Lys
    130                 135                 140

Pro Ser Met Thr Leu Glu Pro Asn Lys Asp Leu Arg Pro Gly Asp Thr
145                 150                 155                 160

Val Thr Ile Thr Cys Ser Ser Tyr Gln Gly Tyr Pro Glu Ala Glu Val
                165                 170                 175

Phe Trp Gln Asp Gly Gln Gly Val Pro Leu Thr Gly Asn Val Thr Thr
            180                 185                 190

Ser Gln Met Ala Asn Glu Gln Gly Leu Phe Asp Val His Ser Ile Leu
        195                 200                 205

Arg Val Val Leu Gly Ala Asn Gly Thr Tyr Ser Cys Leu Val Arg Asn
    210                 215                 220

Pro Val Leu Gln Gln Asp Ala His Ser Ser Val Thr Ile Thr Pro Gln
225                 230                 235                 240

Arg Ser Pro Thr Gly Ala Val Glu Val Gln Val Pro Glu Asp Pro Val
                245                 250                 255

Val Ala Leu Val Gly Thr Asp Ala Thr Leu Arg Cys Ser Phe Ser Pro
            260                 265                 270

Glu Pro Gly Phe Ser Leu Ala Gln Leu Asn Leu Ile Trp Gln Leu Thr
        275                 280                 285

Asp Thr Lys Gln Leu Val His Ser Phe Thr Glu Gly Arg Asp Gln Gly
    290                 295                 300

Ser Ala Tyr Ala Asn Arg Thr Ala Leu Phe Pro Asp Leu Leu Ala Gln
305                 310                 315                 320

Gly Asn Ala Ser Leu Arg Leu Gln Arg Val Arg Val Ala Asp Glu Gly
                325                 330                 335

Ser Phe Thr Cys Phe Val Ser Ile Arg Asp Phe Gly Ser Ala Ala Val
            340                 345                 350

Ser Leu Gln Val Ala Ala Pro Tyr Ser Lys Pro Ser Met Thr Leu Glu
        355                 360                 365

Pro Asn Lys Asp Leu Arg Pro Gly Asp Thr Val Thr Ile Thr Cys Ser
    370                 375                 380

Ser Tyr Arg Gly Tyr Pro Glu Ala Glu Val Phe Trp Gln Asp Gly Gln
385                 390                 395                 400

Gly Val Pro Leu Thr Gly Asn Val Thr Thr Ser Gln Met Ala Asn Glu
                405                 410                 415

Gln Gly Leu Phe Asp Val His Ser Val Leu Arg Val Val Leu Gly Ala
            420                 425                 430

Asn Gly Thr Tyr Ser Cys Leu Val Arg Asn Pro Val Leu Gln Gln Asp
        435                 440                 445

Ala His Gly Ser Val Thr Ile Thr Gly Gln Pro Met Thr Phe Pro Pro
    450                 455                 460

Glu Ala Leu Trp Val Thr Val Gly Leu Ser Val Cys Leu Ile Ala Leu
465                 470                 475                 480

Leu Val Ala Leu Ala Phe Val Cys Trp Arg Lys Ile Lys Gln Ser Cys
                485                 490                 495

Glu Glu Glu Asn Ala Gly Ala Glu Asp Gln Asp Gly Glu Gly Glu Gly
            500                 505                 510

Ser Lys Thr Ala Leu Gln Pro Leu Lys His Ser Asp Ser Lys Glu Gly
        515                 520                 525

Lys Asp Thr Trp Ala
    530